# EUROPEAN PATENT SPECIFICATION

(11) **EP 4 301 874 B1**
(45) Date of publication and mention of the grant of the patent: **23.04.2025**
(21) Application number: 22709783.9
(22) Date of filing: 03.03.2022
(51) Int. Cl.: C12Q 1/6883, C12Q 1/6886

(54) **CHROMOSOME INTERACTION MARKERS**
CHROMOSOMINTERAKTIONSMARKER
MARQUEURS D'INTERACTION CHROMOSOMIQUE

(30) Priority: 04.03.2021 US 202163156659 P; 23.11.2021 US 202163282284 P
(43) Date of publication of application: 10.01.2024
(73) Proprietor: Oxford BioDynamics PLC, Oxford, OX4 2WB (GB)
(72) Inventor: RAMADASS, Aroul Selvam, Oxford Oxfordshire OX4 2WB (GB); HUNTER, Ewan, Oxford Oxfordshire OX4 2WB (GB); AKOULITCHEV, Alexandre, Oxford Oxfordshire OX4 2WB (GB)
(74) Representative: Avidity IP
(86) International application number: PCT/GB2022/050561
(87) International publication number: WO 2022/185062

(56) References cited:
- WO-A1-2020/198676
- WO-A1-2020/225551
- WO-A1-2021/228888
- WO-A2-2019/086898
- SHEN PAN ET AL: "Hyperprogressive Disease in Cancers Treated With Immune Checkpoint Inhibitors", FRONTIERS IN PHARMACOLOGY, vol. 12, 1 July 2021 (2021-07-01), XP055916632, Retrieved from the Internet <URL:https://www.ncbi.nlm.nih.gov/pmc/articles/PMC8287409/pdf/fphar-12-678409.pdf> DOI: 10.3389/fphar.2021.678409
- OXFORD BIODYNAMICS: "EpiSwitch Methodology A robust and reliable methodology for personalised medicine", 1 November 2018 (2018-11-01), XP055712853, Retrieved from the Internet <URL:https://www.oxfordbiodynamics.com/wp-content/uploads/2018/11/EpiSwitch_Methodology_v2_US.pdf> [retrieved on 20200708]
- REFAE SADAL ET AL: "Hyperprogression under Immune Checkpoint Inhibitor: a potential role for germinal immunogenetics", SCIENTIFIC REPORTS, vol. 10, no. 1, 1 December 2020 (2020-12-01), pages 3565, XP055916627, Retrieved from the Internet <URL:https://www.nature.com/articles/s41598-020-60437-0.pdf> DOI: 10.1038/s41598-020-60437-0
- RAMADASS AROUL ET AL: "EpiSwitch Methodology", HTTPS://WWW.OXFORDBIODYNAMICS.COM/WP-CONTENT/UPLOADS/2020/06/EPISWITCH_METHODOLOGY-JUN-2020-US.PDF, June 2020 (2020-06-01), XP055916230, Retrieved from the Internet <URL:https://www.oxfordbiodynamics.com/wp-content/uploads/2020/06/EpiSwitch_Methodology-Jun-2020-US.pdf>
- HUNTER EWAN ET AL: "Development and validation of blood-based predictive biomarkers for response to PD-(L)-1 checkpoint inhibitors: evidence of a universal systemic core of 3D immunogenetic profiling across multiple oncological indications", MEDRXIV, 27 December 2021 (2021-12-27), pages 21, XP055916413, Retrieved from the Internet <URL:https://www.medrxiv.org/content/10.1101/2021.12.21.21268094v1.full.pdf> [retrieved on 20220429], DOI: 10.1101/2021.12.21.21268094
- XUEPING WANG ET AL: "The biomarkers of hyperprogressive disease in PD-1/PD-L1 blockage therapy", MOLECULAR CANCER, vol. 19, no. 1, 2 May 2020 (2020-05-02), XP055740103, DOI: 10.1186/s12943-020-01200-x
- DENIS MORGANE ET AL: "How Can Immune Checkpoint Inhibitors Cause Hyperprogression in Solid Tumors?", FRONTIERS IN IMMUNOLOGY, vol. 11, 20 January 2020 (2020-01-20), Lausanne, CH, XP055916618, ISSN: 1664-3224, DOI: 10.3389/fimmu.2020.00492
- KATO SHUMEI ET AL: "Hyperprogressors after immunotherapy: Analysis of genomic alterations associated with accelerated growth rate", CLINICAL CANCER RESEARCH, 1 August 2017 (2017-08-01), United States, pages 4242 - 4250, XP055916622, Retrieved from the Internet <URL:https://watermark.silverchair.com/4242.pdf?token=AQECAHi208BE49Ooan9kkhW_Ercy7Dm3ZL_9Cf3qfKAc485ysgAAAuUwggLhBgkqhkiG9w0BBwagggLSMIICzgIBADCCAscGCSqGSIb3DQEHATAeBglghkgBZQMEAS4wEQQM5ProPgyrpJ9o18mCAgEQgIICmFD_DF54iDi3J-kuhtxvbI_BZyCGO4hP_9H-PRZkN8m3-TsFzmRWnPRySy64IBexvH-TJ8ePdIegkf9KIt_-4XZyI3YY6Sb> [retrieved on 20220429], DOI: 10.1158/1078-0432.CCR-16-3133
- DONGHAI XIONG ET AL: "Immunogenomic Landscape Contributes to Hyperprogressive Disease after Anti-PD-1 Immunotherapy for Cancer", ISCIENCE, vol. 9, 1 November 2018 (2018-11-01), US, pages 258 - 277, XP055740818, ISSN: 2589-0042, DOI: 10.1016/j.isci.2018.10.021

## Description

### Field of the Invention

The invention relates to immunotherapy.

### Background of the Invention

Cancer is a major burden of disease worldwide. Each year, tens of millions of people are diagnosed with cancer around the world, and more than half of the patients eventually die from it. In many countries, cancer ranks the second most common cause of death following cardiovascular diseases. With significant improvement in treatment and prevention of cardiovascular diseases, cancer has or will soon become the number one killer in many parts of the world. As elderly people are most susceptible to cancer and population aging continues in many countries, cancer will remain a major health problem around the globe.

Whilst the primary purpose of the immune system is to fight infections caused by external foreign agents such as pathogens, it also has the important function of attacking and eliminating cancer cells. Immunotherapy of cancer usually works by assisting the immune system in some way to fight cancer cells.

WO2019/086898 discloses a process for analysing chromosome regions and interactions relating to immunoresponsiveness.

Wang et al., 2020, vol 19, no. 1, 2 May 2020 'The biomarkers of hyperprogressive disease in PD-1/PD-L1 blockage therapy' discloses immune checkpoint inhibitors such as PD-1/PD-L1 antibodies and anti-cytotoxic T lymphocyte antigen 4 antibodies are effective for patients with various cancers.

### Summary of the Invention

The inventors have identified chromosome conformation signatures that define states of the immune system that are relevant to therapy of cancer. This elucidates the role of this modality in regulation of the immune system and allows a 'readout' of in respect of how a patient's immune system will respond to immunotherapy. It has also allowed identification of certain types of responder population for which immunotherapy is not appropriate, and in fact be very harmful. This analysis at the level of the 3D architecture of the genome defined by chromosome interactions offers very early readouts of patient response to immunotherapy allowing decisions to be made at early disease stages as to the most appropriate therapies. Detection of the relevant chromosome interactions has according to the invention has been found to be robust, working across different immunotherapies and cancers.

The identified markers are consistent with deregulations in T cells, NK (natural killer) cells, macrophages, B cells and dendritic cells (DC) showing the role played by the specific set up at cellular level of the adaptive and innate immune system in individual patients as part of the cancer-host interaction which defines disease progression (hyper-progressors) and responsiveness to immunotherapy.

Accordingly, the invention provides a method of determining how an individual responds to immunotherapy for cancer comprising detecting the presence or absence in the individual of:
- all of the chromosome interactions represented by the probe sequences shown in Table 8c to thereby determine whether the individual will be responsive to immunotherapy; and/or
- all of the chromosome interactions represented by the probe sequence shown in Table 2.a3 to thereby determine whether the individual is a hyper-progressor in whom immunotherapy will accelerate disease.

### Brief Description of the Drawings

Figure 1 shows a preferred method for typing chromosome interactions, essentially based on the EpiSwitch method.

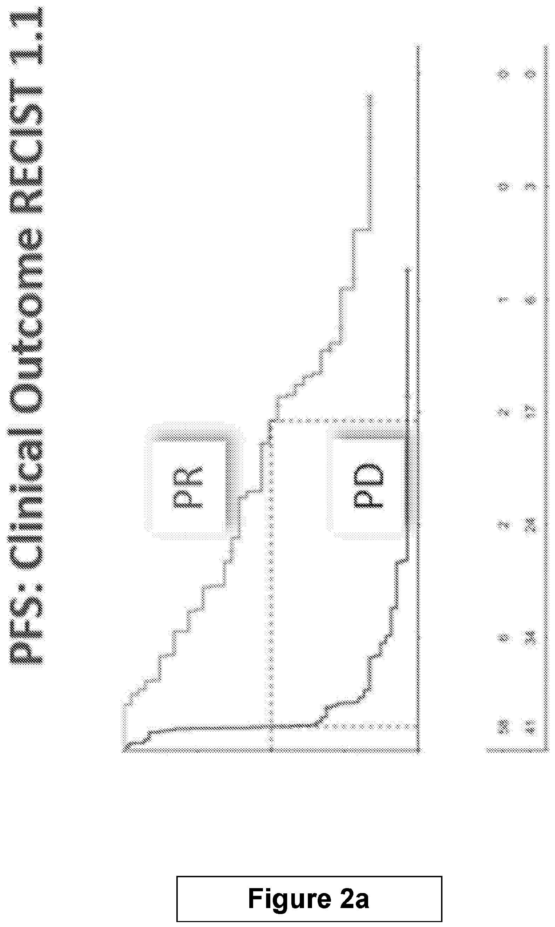

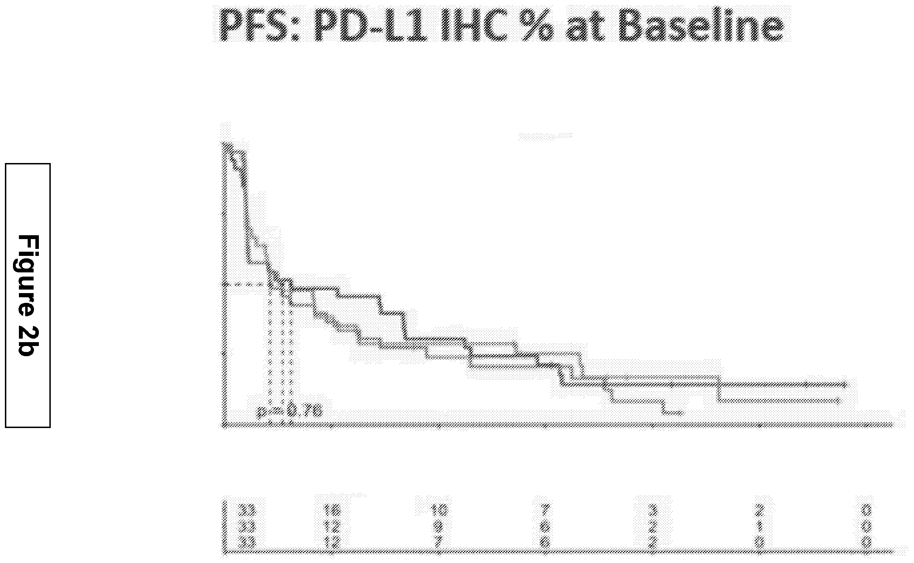

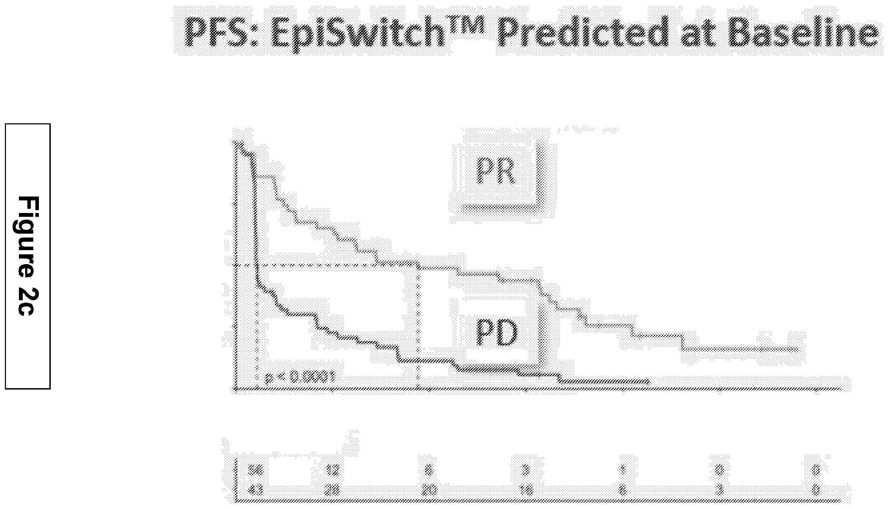

Figure 2 shows predictive and prognostic base-line patient profiling and shows data for response to PD-L1 (Avelumab in second-line (2L) non-small cell lung cancer (NSCLC). For the top graph in each of figures 2a, 2b and 2c the vertical axis shows survival probability from 0.00 to 1.00 and the horizontal axis shows time from 0 to 1200, with the p values for the dotted lines being <0.0001, 0.76 and <0.0001 on figures 2a, 2b and 2c respectively. The bottom table on each figure shows the corresponding number at risk for each line of the graphs across time from 0 to 1200.
Figure 3 relates to validation of EpiSwitch anti-PD-L1 response markers in an independent cohort for 21 patients across cancer types and checkpoint inhibitor therapies.
Figure 4 shows the high concordance between baseline EpiSwitch calls, PD-L1 expression and observed clinical response.
Figure 5 shows data for a training set for an 11 marker model based on 80 NSCLC patients who are a mixture of 1L, 2L Avelumab (54) and 2L Pembrolizumab (36).
Figure 6 shows data for a test set of the 11 marker model based on 38 NSCLC patients who are a mixture of 1L, 2L Avelumab (27) and 2L Pembrolizumab (11).
Figures 7 and 8 show data for a second test set for the Malaysian Observational Study looking a mixture of checkpoint inhibitors and tumours.
Figure 9 shows longitudinal calls in blind Asian samples.
Figure 10 shows the actual EpiSwitch calls for the patients sampled over multiple time points.

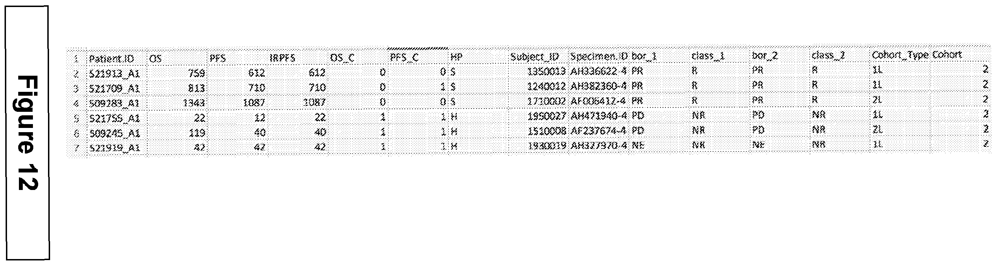

Figures 11 and 12 shows sample selection and patient details for the work relating to hyper-progressors.
Figure 13 shows EpiSwitch chromosome conformation marker selection with associated genetic locations.

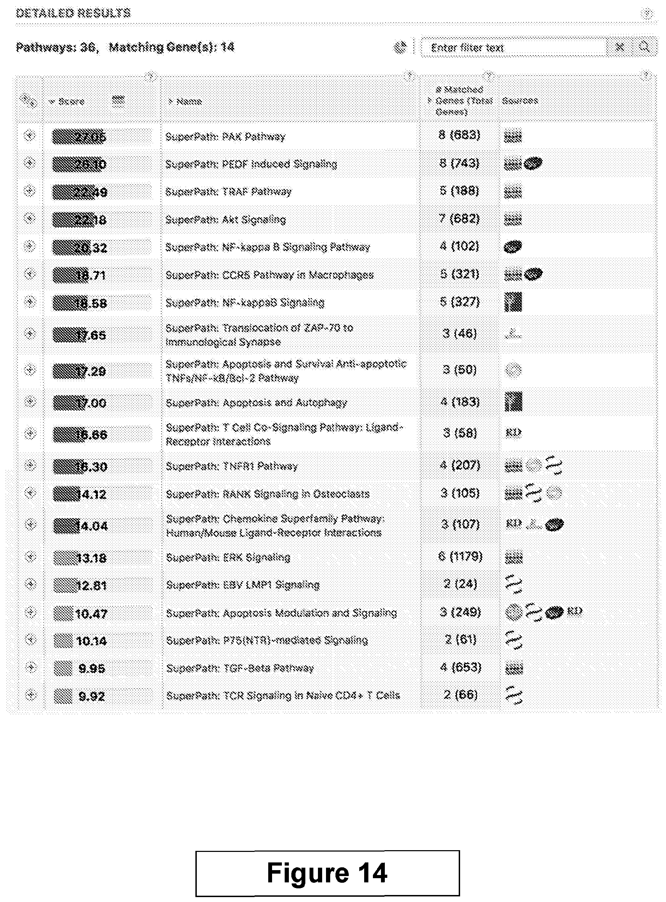

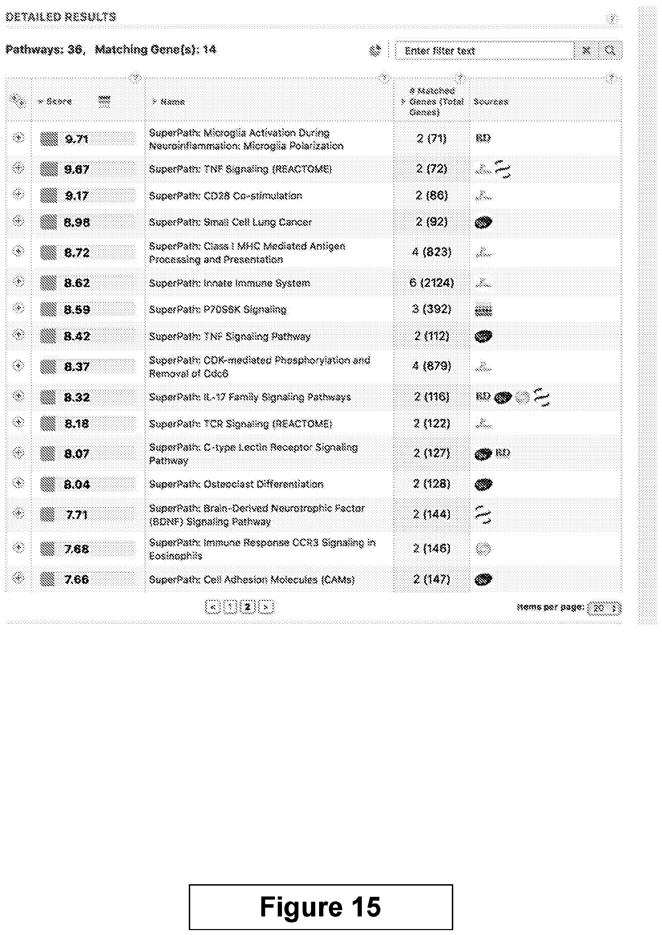

Figures 14 and 15 shows the pathway analysis of the genetic locations.
Figure 16 shows the training set for hyper-progressors.
Figure 17 shows the test set for hyper-progressors.
Figure 18 shows the logistic PCA (principle component analysis) of the training set for hyper-progressors. The squares show H and the circles show S.
Figure 19 shows the logistic PCA of the training set with predicted test samples for hyper-progressors. The squares show H and the dark circles show S.
Figure 20 shows the logistic PCA of the training set with PFS as label for hyper-progressors. The squares show H and the circles show S.
Figure 21 shows the logistic PCA of the training set with OS as label for hyper-progressors. The squares show H and the circles show S.
Figure 22 shows a confusion matrix and statistics. The training model is 78 patients. 30 were NR (non-responders). 39 were R (responders). 9 were SD (stable disease).
Figure 23 shows a confusion matrix and statistics. Test set is 24 patients. 8 were NR. 12 were R. 4 were SD.
Figure 24 shows a confusion matrix and statistics. Test set is 128 patients.
Figure 25 shows the global variable importance for different markers. From the top the bottom the results are shows for (i) obd189_q65-q67_p65, (ii) obd189_q53_q55_p53, (iii) obd189_q81_q83_p81, (iv) obd148_q893_q895_p893, (v) obd189_q49_q51_p49, (vi) obd189_q29_q31_p31, (vii) obd189_q57_q59_p57, (viii) obd189_q05_q07_p05. The horizontal axis show top model features. The vertical axis shows value going from 0 to 20.
Figure 26 shows the genetic locations of markers.
Figure 27 shows pathways associated with the genes of Figure 26.

### Description of the Tables

Table 1 shows the universal marker set and how each marker relates to responsiveness (R) or non-responsiveness (NR) to immunotherapy.
Table 2 shows the marker set for detection of hyper-progressors and how each marker relates to hyper-progression (HS) or being stable (S).
Table 3 shows immune checkpoint molecules that can be targeted and/or modulated by the immunotherapy.
Table 4 to 6 provides examples of cancer immunotherapies for which responder status can be determined and are also the therapies that can be given to the individual based on the outcome of determination of the responder status according to the invention. These tables also show preferred cancers.
Table 7 shows markers relevant to the screens carried out in Example 2 to develop the set of markers shown in Table 8.
Table 8 shows a further universal marker set and how each marker relates to responsiveness (R) or non-responsiveness (NR) to immunotherapy.
Table 9 gives patient data for Example 2. The patients shown with an asterisk (*) were studied in the second screen described in Example 2.

### Detailed Description of the Invention

### Terms Used Herein

The method of the invention may be referred to as the 'process' of the invention herein.

The chromosome interactions which are typed may be referred to as 'markers', 'CCS', 'chromosome conformation signature', 'epigenetic interaction' or 'EpiSwitch markers' herein.

The word 'type' will be interpreted as per the context, but will usually refer to detection of whether a specific chromosome interaction is present or absent. The typing will generally be by physical determination of whether the chromosome interaction is present.

The word 'responder' is used to refer to refer to response to immunotherapy, and covers both aspects relating to responsiveness to immunotherapy (the universal marker set) and detection of hyper-progressors. The term 'responder group' covers all four of the different groups discussed herein:
- responder to immunotherapy
- non-responder to immunotherapy
- hyper-progressor when given immunotherapy
- stable disease (non-hyper-progressor) when given immunotherapy.

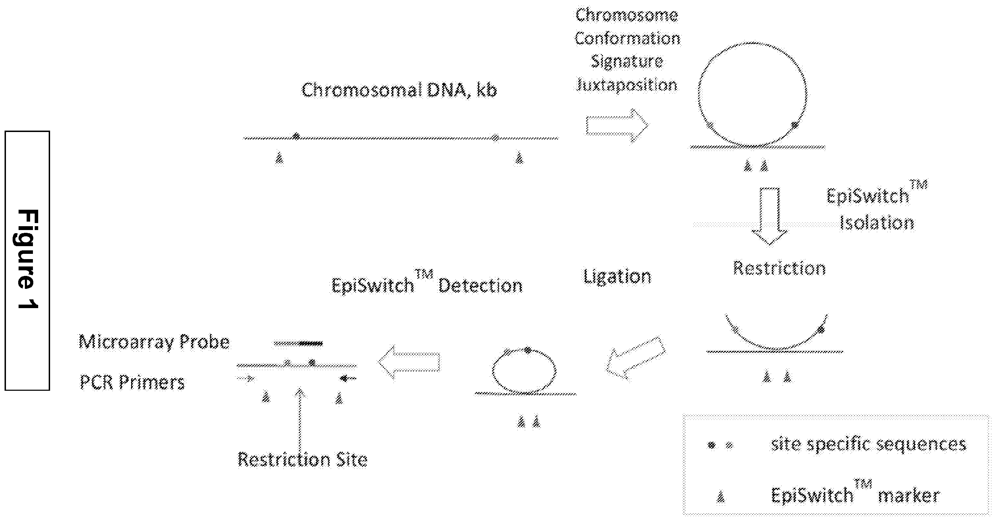

The chromosome interactions which are typed in the method of the invention are defined in Tables 2 and 8. The chromosome interactions which are typed in the method of the invention are further defined in Table 1. They are defined by means of the probe sequences which detect the ligated product made by an EpiSwitch method (see Figure 1). They are also defined by the position numbers of the interaction which are within the probe name and they are also defined by the primer sequences which allow detection of the ligated sequence.

### The Epigenetic Interactions Relevant to the Invention

The chromosome interactions which are typed in the invention are typically interactions between distal regions of a chromosome, said interactions being dynamic and altering, forming or breaking depending upon the state of the region of the chromosome. That state will reflect how the immune system interacts with immunotherapy which is given which responder group the individual falls into.

The chromosome interaction may, for example, reflect if it is being transcribed or repressed. Chromosome interactions which are specific to responder 'groups' as defined herein have been found to be stable, thus providing a reliable means of measuring the differences between groups (for example reflecting different responses to immunotherapy).

Chromosome interactions specific to responder groups will normally be present before or in the early stages of a disease process, for example compared to other epigenetic markers such as methylation or changes to binding of histone proteins. Thus the process of the invention is able to provide valuable information about the way the immune system will react at an early stage. This allows early intervention (for example treatment) which as a consequence will be more effective and also allows early choices to be made of the type of treatment which is appropriate for the patient, and which treatments should not be used. Chromosome interactions also reflect the current state of the individual and therefore can be used to assess changes to disease status. Furthermore there is little variation in the relevant chromosome interactions between individuals within the same group.

The chromosome interactions which are detected in the invention could be impacted by changes to the underlying DNA sequence, by environmental factors, DNA methylation, non-coding antisense RNA transcripts, non-mutagenic carcinogens, histone modifications, chromatin remodelling and specific local DNA interactions. However it must be borne in mind that chromosome interactions as defined herein are a regulatory modality in their own right and do not have a one to one correspondence with any genetic marker (DNA sequence change) or any other epigenetic marker.

The changes which lead to the chromosome interactions may be impacted by changes to the underlying nucleic acid sequence which themselves do not directly affect a gene product or the mode of gene expression. Such changes may be for example, SNPs within and/or outside of the genes, gene fusions and/or deletions of intergenic DNA, microRNA, and non-coding RNA. For example, it is known that roughly 20% of SNPs are in non-coding regions, and therefore the process as described is also informative in non-coding situation. Typically regions of the chromosome which come together to form the interaction are less than 5 kb, 3 kb, 1 kb, 500 base pairs or 200 base pairs apart on the same chromosome.

### The Process of the Invention

The process of the invention comprises a typing system for detecting chromosome interactions relating to responder status. Any suitable typing method can be used, for example a method in which the proximity of the chromosomes in the interaction is detected and/or in which a marker that reflects chromosome interaction status is detected. The typing method may be performed using the *EpiSwitch*^{™} system mentioned herein, which for example may be carried out by a method comprising the following steps (for example on DNA and/or a sample from the subject):
(i) cross-linking regions of chromosome which have come together in a chromosome interaction;
(ii) optionally isolating the cross-linked DNA from said chromosomal locus;
(iii) subjecting the cross-linked DNA to cleavage; and
(iv) ligating the nucleic acids present in the cross-linked entity to derive ligated nucleic acids with sequence from both the regions which formed a chromosomal interaction.

Detection of this ligated nucleic acid allows determination of the presence or absence of a particular chromosome interaction. The ligated nucleic acid therefore acts as a marker for the presence of the chromosome interaction. Preferably the ligated nucleic acid is detected by PCR or a probe based method, including a qPCR method.

In the method the chromosomes can be cross-linked by any suitable means, for example by a cross-linking agent, which is typically a chemical compound. In a preferred aspect, the interactions are cross-linked using formaldehyde, but may also be cross-linked by any aldehyde, or D-Biotinoyl-e-aminocaproic acid-N-hydroxysuccinimide ester or Digoxigenin-3-O-methylcarbonyl-e-aminocaproic acid-N-hydroxysuccinimide ester. Para-formaldehyde can cross link DNA chains which are 4 Angstroms apart. Preferably the chromosome interactions are on the same chromosome. Typically the chromosome interactions are 2 to 10 Angstroms apart.

The cross-linking is preferably *in vitro.* The cleaving is preferably by restriction digestion with an enzyme, such as Taql. The ligating may form DNA loops.

Where PCR (polymerase chain reaction) is used to detect or identify the ligated nucleic acid, the size of the PCR product produced may be indicative of the specific chromosome interaction which is present, and may therefore be used to identify the status of the locus. In preferred aspects the primers shown in any table herein are used, for example the primer pairs shown in Table 2 or 8 are used (corresponding to the chromosome interaction which is being detected). The primers shown in Table 1 may be used. Homologues of such primers or primer pairs may also be used, which can have at least 70% identity to the original sequence.

Where a probe is used to detect or identify the ligated nucleic acid, this is generally by Watson-Crick based base-pairing between the probe and ligated nucleic acid. Probe sequences as shown in any table herein may be used, for example the probe sequences shown in Table 2 or 8 (corresponding to the chromosome interaction which is being detected). Probe sequences as shown in Table 1 may be used. Homologues of such probe sequences may also be used, which can have at least 70% identity to the original sequence.

Typing according to the process of the invention may be carried out at multiple time points, for example to monitor the progression of the disease. This may be at one or more defined time points, for example at at least 1, 2, 5, 8 or 10 different time points. The durations between at least 1, 2, 5 or 8 of the time points may be at least 5, 10, 20, 50, 80 or 100 days. Typically there are 3 time points at least 50 days apart.

### The Individual to Tested and/or Treated

The individual who is tested in the method of the invention is preferably a eukaryote, animal, bird or mammal. Most preferably the individual is a human. The individual may be male or female. In the case of a human individual they are typically aged 65 or above.

The invention includes detecting and treating particular groups in a population, typically differing in their responder status, for example their response to immunotherapy. The inventors have discovered that chromosome interactions differ between these groups, and identifying these differences will allow physicians to categorize their patients as a part of a particular group of the population. The invention therefore provides physicians with a process of personalizing medicine for an individual based on their epigenetic chromosome interactions. Such testing may be used to select how to subsequently treat the patient, for example the type of drug that will be administered. The process of the invention may be carried out to select treatment for an individual, for example whether or not to give any specific treatment mentioned herein is administered to the individual.

The individual that is tested in the process of the invention may have been selected in some way, for example based on a risk factor, symptom or physical characteristic. The individual may have been selected based on having a symptom of cancer and/or or being in the early stages of cancer.

The individual may be susceptible to any cancer mentioned herein and/or may be in need of any therapy mentioned in. The individual may be receiving any therapy mentioned herein. In particular, the individual may have, or be suspected of having, cancer, for example any specific cancer mentioned herein.

### Types of Cancer

The cancer which is relevant to the invention can include any cancer mentioned herein, and preferably is melanoma, lung cancer, non-small cell lung carcinoma (NSCLC), diffuse large B-cell lymphoma, liver cancer, hepatocellular carcinoma, prostate cancer, breast cancer, leukaemia, acute myeloid leukaemia, pancreatic cancer, thyroid cancer, nasal cancer, brain cancer, bladder cancer, cervical cancer, non-Hodgkin lymphoma, ovarian cancer, colorectal cancer or kidney cancer. The cancer may be one which can be treated by immunotherapy, for example any specific immunotherapy mentioned herein.

### Types of Immunotherapy

The invention relates to determining response to immunotherapy, and in particular whether the individual is responsive to immunotherapy and/or whether they are hyper-progressors in whom immunotherapy will cause acceleration of disease.

Preferably the response which is determined is to a therapy which comprises a molecule or cell that is relevant to the immune system, such as a composition that comprises an antibody or immune cell (for example a T cell or dendritic cell) or any therapeutic substance mentioned herein. It may be response to a substance that modulates or stimulates the immune system, such as a vaccine therapy. The immunotherapy may modulate, block or stimulate an immune checkpoint, and thus may target or modulate PD-L1, PD-L2 or CTLA4 or any other immune checkpoint molecule disclosed herein, and thus is preferably immunocheckpoint therapy. Preferably the response is responsiveness to an antibody therapy, or to any specific therapy disclosed herein. The therapy may be a combination therapy, for example any specific combination therapy disclosed herein.

In one embodiment the response is to a PD-1 inhibitor or PD-L1 inhibitor, including an antibody specific for PD-1 or PD-L1. PD-1 is 'programmed cell death protein' and PD-L1 is 'programmed death-ligand 1'.

The term 'antibody' includes all fragments and derivatives of an antibody that retain the ability to bind the antigen target, for example single chain scFV's or Fab's.

The therapy may be mono or combination therapy, for example with immunocheckpoint modulators (preferably inhibitors) for PD-1 and or its ligand, PD-L1. The therapy could comprise administering at least one immunocheckpoint modulator, for example as disclosed herein, such as in any table, figure or example. The therapy could be a combination of an anti-PD-1 or anti-PD-L1 combined with another drug that targets a checkpoint like CTLA4 (Ipilimumab/Yervoy) or small molecules. The PD-1 inhibitors could be pembrolizumab (Keytruda) or nivolumab (Opdivo). The modulator of PD-L1 or therapeutic agent could be Atezolizumab (Tecentriq), Avelumab (Bavencio), Durvalumab (Imfinzi), CA-170, Ipilimumab, Tremelimumab, Nivolumab, Pembrolizumab, Pidilizumab, BMS935559, GVAXMPDL3280A, MEDI4736, MSB0010718C, MDX-1105/BMS-936559, AMP-224, MEDI0680.

The therapy may comprise administering agents that target and/or modulate interferon gamma or the JAK-START pathway.

The therapeutic agent may be any such agent disclosed in any table herein or may target any 'target' disclosed herein, including any protein disclosed herein. It is understood that any agent that is disclosed in a combination should be seen as also disclosed for administration individually.

### Hyper-Progressors

Hyper-progression in cancer can be recognised in a straightforward manner by the skilled person, and it is preferably an increase in disease progression in cancer upon administration of immunotherapy and/or an adverse response to immunotherapy in an individual with cancer. It be measured using any suitable parameter for disease, such as a 2-fold increase in tumour size. It is typically more than a 50% increase in tumour burden within 60 days of administration of immunotherapy. In one aspect hyper-progressors can be defined as having less than 60 days progression-free after administration of immunotherapy and/or overall survival of less than 150 days after immunotherapy.

### Choice of Treatment

Based on the results of testing by the method of the invention decisions can be made as to what treatments will be administered or not administered to the individual.

If a person is found to be responsive to immunotherapy they could be given any immunotherapy mentioned herein. In one aspect if an individual is found to be a non-responsiveness then can be given a combination therapy, such as any combination therapy listed herein. Typically a combination therapy comprises an antibody and a small molecule.

### The Data in the Tables Provided Herein

Tables 1, 2 and 8 show specific markers which can be used to detect responder status. Their presence or absence can be used in such a detection (i.e. they are 'disseminating' markers). Tables 1 and 8 show markers which detect responsiveness to immunotherapy, and the table shows which are linked to responsiveness and which are linked to non-responsiveness. Table 2 shows markers which detect hyper-progressors, and the tables shows which are linked to being a hyper-progressor and which are linked to stable disease.

The markers are defined using probe sequences (which detect a ligated product as defined herein). The first two sets of Start-End positions show probe positions, and the second two sets of Start-End positions show the relevant 4kb region.

The following information is provided in the probe data table:
RP - Rsum the Rank Product statistics evaluated per each chromosome interaction.
FC - Interaction frequency (positive or negative).
Pfp - estimated percentage of false positive predictions (pfp), both considering positive and negative chromosome interactions.
Pval - estimated pvalues per each CCSs being positive and negative.
Adj.P.value (FDR) - False discovery rate adjusted p.value.
Type - which state the loop is found in.

Simple permutation-based estimation is used to determine how likely a given RP value or better is observed in a random experiment. This has the following steps:
1. Generate p permutations of k rank lists of length n.
2. Calculate the rank products of the n CCS in the p permutations.
3. Count (c) how many times the rank products of the CCS in the permutations are smaller or equal to the observed rank product. Set c to this value.
4. Calculate the average expected value for the rank product by: Erp(g)=c/p.
5. Calculate the percentage of false positives as: pfp (g)=Erp(g)/rank (g) where rank(g) is the rank of CCS g in a list of all n CCSs sorted by increasing RP.

The rank product statistic ranks chromosome interactions according to intensities within each microarray and calculates the product of these ranks across multiple microarrays. This technique can identify chromosome interactions that are consistently detected among the most differential chromosome interactions in a number of replicated microarrays. Where the p-value is 0 this indicates that there is very little variation in the Rank Product of the CCS across the samples, this is a good example of the signal to noise and effect size of CCS. Where p value is 0 and pfp is 0 this means that permutated Rank Product doesn't differ from the actual observed Rank Product. These methods are described Breitling R and Herzyk P (2005) Rank-based methods as a non-parametric alternative of the t-test for the analysis of biological microarray data. J Bioinf Comp Biol 3, 1171-1189.

The FC indicates prevalence of marker in each comparison, 2 means twice over average test, 1.5 means 1.5 over the average test, etc., and so FC indicates the weight of a marker to phenotype/group. The FC value can be used to give an indication of how many markers are needed for a highly effective test.

The probes are designed to be 30bp away from the Taq1 site. In case of PCR, PCR primers are typically designed to detect ligated product but their locations from the Taq1 site vary. Probe locations:
Start 1 - 30 bases upstream of Taql site on fragment 1
End 1 - Taql restriction site on fragment 1
Start 2 - Taql restriction site on fragment 2
End 2 - 30 bases downstream of Taql site on fragment 2
4kb Sequence Location:
   Start 1 - 4000 bases upstream of Taql site on fragment 1
   End 1 - Taql restriction site on fragment 1
   Start 2 - Taql restriction site on fragment 2
   End 2 - 4000 bases downstream of Taql site on fragment 2

### Types of Detection

When detection is performed using a probe, typically sequence from both regions of the probe (i.e. from both sites of the chromosome interaction) could be detected. In preferred aspects probes are used in the process which comprise or consist of the same or complementary sequence to a probe shown in any table. In some aspects probes are used which comprise sequence which is homologous to any of the probe sequences shown in the tables.

### The Approach Taken to Identify Markers and Panels of Markers

The invention described herein relates to chromosome conformation profile and 3D architecture as a regulatory modality in its own right, closely linked to the phenotype. The discovery of biomarkers was based on annotations through pattern recognition and screening on representative cohorts of clinical samples representing the differences in phenotypes. We annotated and screened significant parts of the genome, across coding and non-coding parts and over large sways of non-coding 5' and 3' of known genes for identification of statistically disseminating consistent conditional disseminating chromosome conformations, which for example anchor in the non-coding sites within (intronic) or outside of open reading frames.

In selection of the best markers we are driven by statistical data and p values for the marker leads. Selected and validated chromosome conformations within the signature are disseminating stratifying entities in their own right, irrespective of the expression profiles of the genes used in the reference.

Further work may be done on relevant regulatory modalities, such as SNPs at the anchoring sites, changes in gene transcription profiles, changes at the level of H3K27ac.

We are taking the question of clinical phenotype differences and their stratification from the basis of fundamental biology and epigenetic controls over phenotype - including for example from the framework of network of regulation. As such, to assist stratification, one can capture changes in the network and it is preferably done through signatures of several biomarkers, for example through following a machine learning algorithm for marker reduction which includes evaluating the optimal number of markers to stratify the testing cohort with minimal noise. This may end with 3-20 markers.

Selection of markers for panels may be done by cross-validation statistical performance (and not for example by the functional relevance of the neighbouring genes, used for the reference name).

A panel of markers (with names of adjacent genes) is a product of clustered selection from the screening across significant parts of the genome, in non-biased way analysing statistical disseminating powers over 14,000-60,000 annotated EpiSwitch sites across significant parts of the genome. It should not be perceived as a tailored capture of a chromosome conformation on the gene of know functional value for the question of stratification. The total number of sites for chromosome interaction are 1.2 million, and so the potential number of combinations is 1.2 million to the power 1.2 million. The approach that we have followed nevertheless allows the identifying of the relevant chromosome interactions.

The specific markers that are provided by this application have passed selection, being statistically (significantly) associated with the condition or subgroup. This is what the data in the relevant table demonstrates. Each marker can be seen as representing an event of biological epigenetic as part of network deregulation that is manifested in the relevant condition. In practical terms it means that these markers are prevalent across groups of patients when compared to controls. On average, as an example, an individual marker may typically be present in 80% of the relevant responder group and in 10% of controls, and therefore the results of the testing by the method of the invention is straightforward to interpret and essentially amounts to a 'binary readout'.

Simple addition of all markers would not directly represent the network interrelationships between some of the deregulations. This is where the standard multivariate biomarker analysis GLMNET (R package) can be brought in. GLMNET package helps to identify interdependence between some of the markers, that reflect their joint role in achieving deregulations leading to disease phenotype. Modelling and then testing markers with highest GLMNET scores offers not only identify the minimal number of markers that accurately identifies the patient cohort, but also the minimal number that offers the least false positive results in the control group of patients, due to background statistical noise of low prevalence in the control group. Typically a group (combination) of selected markers (such as 3 to 11) offers the best balance between both sensitivity and specificity of detection, emerging in the context of multivariate analysis from individual properties of all the selected statistical significant markers for the condition.

The tables herein show the reference names for the array probes (60-mer) for array analysis that overlaps the juncture between the long range interaction sites, the chromosome number and the start and end of two chromosomal fragments that come into juxtaposition.

In a preferred aspect all 11 of the markers of Table 1 are typed. In another preferred aspect all 11 of the markers of Table 2 are typed. In another preferred aspect all 8 of the markers of Table 8 are typed.

### Samples and Sample Treatment

The process of the invention will normally be carried out on a sample. The sample may be obtained at a defined time point, for example at any time point defined herein. The sample will normally contain DNA from the individual. It will normally contain cells. In one aspect a sample is obtained by minimally invasive means, and may for example be a blood sample. DNA may be extracted and cut up with a standard restriction enzyme. This can pre-determine which chromosome conformations are retained and will be detected with the EpiSwitch^{™} platforms. Due to the synchronisation of chromosome interactions between tissues and blood, including horizontal transfer, a blood sample can be used to detect the chromosome interactions in tissues, such as tissues relevant to disease.

### Preferred Aspects for Sample Preparation and Chromosome Interaction Detection

Methods of preparing samples and detecting chromosome conformations are described herein. Optimised (non-conventional) versions of these processes can be used, for example as described in this section.

Typically the sample will contain at least 2 ×10⁵ cells. The sample may contain up to 5 ×10⁵ cells. In one aspect, the sample will contain 2 ×10⁵ to 5.5 ×10⁵ cells.

Crosslinking of epigenetic chromosomal interactions present at the chromosomal locus is described herein. This may be performed before cell lysis takes place. Cell lysis may be performed for 3 to 7 minutes, such as 4 to 6 or about 5 minutes. In some aspects, cell lysis is performed for at least 5 minutes and for less than 10 minutes.

Digesting DNA with a restriction enzyme is described herein. Typically, DNA restriction is performed at about 55°C to about 70°C, such as for about 65°C, for a period of about 10 to 30 minutes, such as about 20 minutes.

Preferably a frequent cutter restriction enzyme is used which results in fragments of ligated DNA with an average fragment size up to 4000 base pair. Optionally the restriction enzyme results in fragments of ligated DNA have an average fragment size of about 200 to 300 base pairs, such as about 256 base pairs. In one aspect, the typical fragment size is from 200 base pairs to 4,000 base pairs, such as 400 to 2,000 or 500 to 1,000 base pairs.

In one aspect of the EpiSwitch process a DNA precipitation step is not performed between the DNA restriction digest step and the DNA ligation step.

DNA ligation is described herein. Typically the DNA ligation is performed for 5 to 30 minutes, such as about 10 minutes.

The protein in the sample may be digested enzymatically, for example using a proteinase, optionally Proteinase K. The protein may be enzymatically digested for a period of about 30 minutes to 1 hour, for example for about 45 minutes. In one aspect after digestion of the protein, for example Proteinase K digestion, there is no cross-link reversal or phenol DNA extraction step.

In one aspect PCR detection is capable of detecting a single copy of the ligated nucleic acid, preferably with a binary read-out for presence/absence of the ligated nucleic acid.

Figure 1 shows a preferred process of detecting chromosome interactions.

### Processes and Uses of the Invention

The process of the invention can be described in different ways. It can be described as a process of making one or more ligated nucleic acids comprising (i) *in vitro* cross-linking of chromosome regions which have come together in a chromosome interaction; (ii) subjecting said cross-linked DNA to cutting or restriction digestion cleavage; and (iii) ligating said cross-linked cleaved DNA ends to form one or more ligated nucleic acids, wherein optionally detection of the ligated nucleic acid may be used to determine the chromosome state at a locus, and wherein preferably the chromosomal interactions may be 1, 3, 5, 8 or all the chromosome interactions of Table 1 or 2. In this process the chromosomal interactions may be 1, 3, 5 or 8 of the chromosome interactions of Table 8.

### Homologues

Homologues of polynucleotide / nucleic acid (e.g. DNA) sequences are referred to herein. Such homologues typically have at least 70% homology, preferably at least 80%, at least 85%, at least 90%, at least 95%, at least 97%, at least 98% or at least 99% homology, for example over a region of at least 10, 15, 20, 30, 100 or more contiguous nucleotides, or across the portion of the nucleic acid which is from the region of the chromosome involved in the chromosome interaction. The homology may be calculated on the basis of nucleotide identity (sometimes referred to as "hard homology").

Therefore, in a particular aspect, homologues of polynucleotide / nucleic acid (e.g. DNA) sequences are referred to herein by reference to percentage sequence identity. Typically such homologues have at least 70% sequence identity, preferably at least 80%, at least 85%, at least 90%, at least 95%, at least 97%, at least 98% or at least 99% sequence identity, for example over a region of at least 10, 15, 20, 30, 100 or more contiguous nucleotides, or across the portion of the nucleic acid which is from the region of the chromosome involved in the chromosome interaction. The homologues may have at least 70% sequence identity, preferably at least 80%, at least 85%, at least 90%, at least 95%, at least 97%, at least 98% or at least 99% sequence identity across the entire probe, primer or primer pair.

For example the UWGCG Package provides the BESTFIT program which can be used to calculate homology and/or % sequence identity (for example used on its default settings) (Devereux et al (1984) Nucleic Acids Research 12, p387-395). The PILEUP and BLAST algorithms can be used to calculate homology and/or % sequence identity and/or line up sequences (such as identifying equivalent or corresponding sequences (typically on their default settings)), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information. This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W5 T and X determine the sensitivity and speed of the alignment. The BLAST program uses as defaults a word length (W) of 11 , the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologous sequence typically differs by 1, 2, 3, 4 or more bases, such as less than 10, 15 or 20 bases (which may be substitutions, deletions or insertions of nucleotides). These changes may be measured across any of the regions mentioned above in relation to calculating homology and/or % percentage sequence identity.

Homology of a 'pair of primers' can be calculated, for example, by considering the two sequences as a single sequence (as if the two sequences are joined together) for the purpose of then comparing against the another primer pair which again is considered as a single sequence.

### The Threshold of Detection

The markers which are disclosed herein have been found to be 'disseminating markers' capable of determining responder status and tables 1 and 2 show which responder group each marker is present in (responder/non-responder to immunotherapy, or hyper-progressor/stable disease).

In practical terms it means that these markers are prevalent across the relevant responder group when compared to controls (as is shown by the FC value, for example). On average, as an example, an individual marker may typically be present in 80% of the relevant responder group and in 10% of controls. When testing an individual the result will be a combination of 'present' and 'absent' chromosome interactions for each of the markers shown in Table 1 or 2 allowing determination of the responder group for the individual. Typically presence/absence of at least 8 markers out of 11 compared to the 'ideal' result shown in the table can be used to assign the individual to a responder group.

### Therapeutic Agents and Treatments

This section is relevant both to immunotherapies which define the responder group of the individual and also to therapy which may be given to individuals based on the results of the testing method of the invention.

We disclose therapeutic agents for use in preventing or treating any condition mentioned herein. This may comprise administering to an individual in need a therapeutically effective amount of the agent. We disclose use of the agent in the manufacture of a medicament to prevent or treat the condition, for example in individuals tested by the method of the invention.

The formulation of the agent will depend upon the nature of the agent. The agent will be provided in the form of a pharmaceutical composition containing the agent and a pharmaceutically acceptable carrier or diluent. Suitable carriers and diluents include isotonic saline solutions, for example phosphate-buffered saline. Typical oral dosage compositions include tablets, capsules, liquid solutions and liquid suspensions. The agent may be formulated for parenteral, intravenous, intramuscular, subcutaneous, transdermal or oral administration.

The dose of an agent may be determined according to various parameters, especially according to the substance used; the age, weight and condition of the individual to be treated; the route of administration; and the required regimen. A physician will be able to determine the required route of administration and dosage for any particular agent. A suitable dose may however be from 0.1 to 100 mg/kg body weight such as 1 to 40 mg/kg body weight, for example, to be taken from 1 to 3 times daily.

We disclose an immunotherapeutic agent, preferably selected from any of tables 4 to 6, for use in a method of treating an individual identified as being responsive to immunotherapy, optionally said method comprising:
- identifying whether an individual is responsive to immunotherapy by the method of the invention, and
- administering to any individual identified as responsive to immunotherapy said agent.

We disclose (i) a combination immunotherapy or (ii) a therapeutic agent which is not an immunotherapy for use in a method of treating an individual identified as being non-responsive to immunotherapy, optionally said method comprising:
- identifying whether an individual is responsive to immunotherapy by the method of the invention, and
- administering to any individual identified as non-responsive (i) and/or (ii), wherein optionally the combination therapy of (i) is any combination therapy shown in table 4 or which comprises at least one agent chosen from tables 4 and 5.

### Screening for Therapeutic Agents

We disclose a screening method to identify therapeutic agents for cancer comprising determining whether a candidate agent is able to cause a change to all of the chromosome interactions shown in Table 1 and/or Table 2. This screening method may comprise determining whether a candidate agent is able to cause a change to all of the chromosome interactions shown in Table 8.

### Nucleic Acids

We disclose certain nucleic acids, including probes and primers. Preferably the nucleic acids are DNA. It is understood that where a specific sequence is provided the invention may use the complementary sequence as required in the particular aspect.

The primers or probes shown in Table 1 or 2 may be used in the invention. In one aspect probes or primers are used which comprise any of: the sequences shown in Table 1 or 2; or fragments and/or homologues of any sequence shown in Table 1 or 2. The primers or probes shown in Table 8 may be used in the invention. In one aspect probes or primers are used which comprise any of: the sequences shown in Table 8; or fragments and/or homologues of any sequence shown in Table 8.

### Labelled Nucleic Acids and Pattern of Hybridisation

The nucleic acids mentioned herein may be labelled, preferably using an independent label such as a fluorophore (fluorescent molecule) or radioactive label which assists detection of successful hybridisation. Certain labels can be detected under UV light.

### Forms of the Substance Mentioned Herein

Any of the substances, such as nucleic acids or therapeutic agents, mentioned herein may be in purified or isolated form. They may be in a form which is different from that found in nature, for example they may be present in combination with other substance with which they do not occur in nature. The nucleic acids (including portions of sequences defined herein) may have sequences which are different to those found in nature, for example having at least 1, 2, 3, 4 or more nucleotide changes in the sequence as described in the section on homology. The nucleic acids may have heterologous sequence at the 5' or 3' end. The nucleic acids may be chemically different from those found in nature, for example they may be modified in some way, but preferably are still capable of Watson-Crick base pairing. Where appropriate the nucleic acids will be provided in double stranded or single stranded form. The invention provides all of the specific nucleic acid sequences mentioned herein in single or double stranded form, and thus includes the complementary strand to any sequence which is disclosed.

We disclose a kit for carrying out any process of the invention, including detection of a chromosomal interaction relating to prognosis. Such a kit can include a specific binding agent capable of detecting the relevant chromosomal interaction, such as agents capable of detecting a ligated nucleic acid generated by processes of the invention. Preferred agents present in the kit include probes capable of hybridising to the ligated nucleic acid or primer pairs, for example as described herein, capable of amplifying the ligated nucleic acid in a PCR reaction. Preferred agents include any of the specific primers and probes disclosed herein and/or homologues of such primers and probes.

We disclose a device that is capable of detecting the relevant chromosome interactions. The device preferably comprises any specific binding agents, probe or primer pair capable of detecting the chromosome interaction, such as any such agent, probe or primer pair described herein.

### Detection Process

In one aspect quantitative detection of the ligated sequence which is relevant to a chromosome interaction is carried out using a probe which is detectable upon activation during a PCR reaction, wherein said ligated sequence comprises sequences from two chromosome regions that come together in an epigenetic chromosome interaction, wherein said process comprises contacting the ligated sequence with the probe during a PCR reaction, and detecting the extent of activation of the probe, and wherein said probe binds the ligation site. The process typically allows particular interactions to be detected in a MIQE compliant manner using a dual labelled fluorescent hydrolysis probe.

The probe is generally labelled with a detectable label which has an inactive and active state, so that it is only detected when activated. The extent of activation will be related to the extent of template (ligation product) present in the PCR reaction. Detection may be carried out during all or some of the PCR, for example for at least 50% or 80% of the cycles of the PCR.

The probe can comprise a fluorophore covalently attached to one end of the oligonucleotide, and a quencher attached to the other end of the nucleotide, so that the fluorescence of the fluorophore is quenched by the quencher. In one aspect the fluorophore is attached to the 5'end of the oligonucleotide, and the quencher is covalently attached to the 3' end of the oligonucleotide. Fluorophores that can be used in the process of the invention include FAM, TET, JOE, Yakima Yellow, HEX, Cyanine3, ATTO 550, TAMRA, ROX, Texas Red, Cyanine 3.5, LC610, LC 640, ATTO 647N, Cyanine 5, Cyanine 5.5 and ATTO 680. Quenchers that can be used with the appropriate fluorophore include TAM, BHQ1, DAB, Eclip, BHQ2 and BBQ650, optionally wherein said fluorophore is selected from HEX, Texas Red and FAM. Preferred combinations of fluorophore and quencher include FAM with BHQ1 and Texas Red with BHQ2.

### Use of the Probe in a qPCR Assay

Hydrolysis probes of the invention are typically temperature gradient optimised with concentration matched negative controls. Preferably single-step PCR reactions are optimized. More preferably a standard curve is calculated. An advantage of using a specific probe that binds across the junction of the ligated sequence is that specificity for the ligated sequence can be achieved without using a nested PCR approach. The processes described herein allow accurate and precise quantification of low copy number targets. The target ligated sequence can be purified, for example gel-purified, prior to temperature gradient optimization. The target ligated sequence can be sequenced. Preferably PCR reactions are performed using about 10ng, or 5 to 15 ng, or 10 to 20ng, or 10 to 50ng, or 10 to 200ng template DNA. Forward and reverse primers are designed such that one primer binds to the sequence of one of the chromosome regions represented in the ligated DNA sequence, and the other primer binds to other chromosome region represented in the ligated DNA sequence, for example, by being complementary to the sequence.

### Choice of Ligated DNA Target

The invention includes selecting primers and a probe for use in a PCR process as defined herein comprising selecting primers based on their ability to bind and amplify the ligated sequence and selecting the probe sequence based properties of the target sequence to which it will bind, in particular the curvature of the target sequence.

Probes are typically designed/chosen to bind to ligated sequences which are juxtaposed restriction fragments spanning the restriction site. In one aspect of the invention, the predicted curvature of possible ligated sequences relevant to a particular chromosome interaction is calculated, for example using a specific algorithm referenced herein. The curvature can be expressed as degrees per helical turn, e.g. 10.5° per helical turn. Ligated sequences are selected for targeting where the ligated sequence has a curvature propensity peak score of at least 5° per helical turn, typically at least 10°, 15° or 20° per helical turn, for example 5° to 20° per helical turn. Preferably the curvature propensity score per helical turn is calculated for at least 20, 50, 100, 200 or 400 bases, such as for 20 to 400 bases upstream and/or downstream of the ligation site. Thus in one aspect the target sequence in the ligated product has any of these levels of curvature. Target sequences can also be chosen based on lowest thermodynamic structure free energy.

### Particular Aspects

In particular aspects certain chromosome interactions are not typed, for example any specific interaction mentioned not mentioned herein. In some aspects only the markers of Table 1 or Table 2 are typed and no other markers are typed. In some aspects only the markers of Table 2 or Table 8 are typed and no other markers are typed. In some aspect only the markers of Table 1 and Table 2 are typed and no other markers are typed. In some aspect only the markers of Table 2 and Table 8 are typed and no other markers are typed.

### Techniques Used to Identify the Specific Relevant Chromosome Interactions

The EpiSwitch^{™} platform technology detects epigenetic regulatory signatures of regulatory changes between normal and abnormal conditions at loci. The EpiSwitch^{™} platform identifies and monitors the fundamental epigenetic level of gene regulation associated with regulatory high order structures of human chromosomes also known as chromosome conformation signatures. Chromosome signatures are a distinct primary step in a cascade of gene deregulation. They are high order biomarkers with a unique set of advantages against biomarker platforms that utilize late epigenetic and gene expression biomarkers, such as DNA methylation and RNA profiling.

### EpiSwitch^{™} Array Assay

The custom EpiSwitch^{™} array-screening platforms come in 4 densities of, 15K, 45K, 100K, and 250K unique chromosome conformations, each chimeric fragment is repeated on the arrays 4 times, making the effective densities 60K, 180K, 400K and 1 million respectively.

### Custom Designed EpiSwitch^{™} Arrays

The 15K EpiSwitch^{™} array can screen the whole genome including around 300 loci interrogated with the EpiSwitch^{™} Biomarker discovery technology. The EpiSwitch^{™} array is built on the Agilent SurePrint G3 Custom CGH microarray platform; this technology offers 4 densities, 60K, 180K, 400K and 1 million probes. The density per array is reduced to 15K, 45K, 100K and 250K as each EpiSwitch^{™} probe is presented as a quadruplicate, thus allowing for statistical evaluation of the reproducibility. The average number of potential EpiSwitch^{™} markers interrogated per genetic loci is 50, as such the numbers of loci that can be investigated are 300, 900, 2000, and 5000.

### EpiSwitch^{™} Custom Array Pipeline

The EpiSwitch^{™} array is a dual colour system with one set of samples, after EpiSwitch^{™} library generation, labelled in Cy5 and the other of sample (controls) to be compared/ analyzed labelled in Cy3. The arrays are scanned using the Agilent SureScan Scanner and the resultant features extracted using the Agilent Feature Extraction software. The data is then processed using the EpiSwitch^{™} array processing scripts in R. The arrays are processed using standard dual colour packages in Bioconductor in R: Limma*. The normalisation of the arrays is done using the normalisedWithinArrays function in Limma* and this is done to the on chip Agilent positive controls and EpiSwitch^{™} positive controls. The data is filtered based on the Agilent Flag calls, the Agilent control probes are removed and the technical replicate probes are averaged, in order for them to be analysed using Limma*. The probes are modelled based on their difference between the 2 scenarios being compared and then corrected by using False Discovery Rate. Probes with Coefficient of Variation (CV) <=30% that are <=-1.1 or =>1.1 and pass the p<=0.1 FDR p-value are used for further screening. To reduce the probe set further Multiple Factor Analysis is performed using the FactorMineR package in R.
* Note: LIMMA is Linear Models and Empirical Bayes Processes for Assessing Differential Expression in Microarray Experiments. Limma is an R package for the analysis of gene expression data arising from microarray or RNA-Seq.

The pool of probes is initially selected based on adjusted p-value, FC and CV <30% (arbitrary cut off point) parameters for final picking. Further analyses and the final list are drawn based only on the first two parameters (adj. p-value; FC).

### Statistical Pipeline

EpiSwitch^{™} screening arrays are processed using the EpiSwitch^{™} Analytical Package in R in order to select high value EpiSwitch^{™} markers for translation on to the EpiSwitch^{™} PCR platform.

### Step 1

Probes are selected based on their corrected p-value (False Discovery Rate, FDR), which is the product of a modified linear regression model. Probes below p-value <= 0.1 are selected and then further reduced by their Epigenetic ratio (ER), probes ER have to be <=-1.1 or =>1.1 in order to be selected for further analysis. The last filter is a coefficient of variation (CV), probes have to be below <=0.3.

### Step 2

The top 40 markers from the statistical lists are selected based on their ER for selection as markers for PCR translation. The top 20 markers with the highest negative ER load and the top 20 markers with the highest positive ER load form the list.

### Step 3

The resultant markers from step 1, the statistically significant probes form the bases of enrichment analysis using hypergeometric enrichment (HE). This analysis enables marker reduction from the significant probe list, and along with the markers from step 2 forms the list of probes translated on to the EpiSwitch^{™} PCR platform.

The statistical probes are processed by HE to determine which genetic locations have an enrichment of statistically significant probes, indicating which genetic locations are hubs of epigenetic difference.

The most significant enriched loci based on a corrected p-value are selected for probe list generation. Genetic locations below p-value of 0.3 or 0.2 are selected. The statistical probes mapping to these genetic locations, with the markers from step 2, form the high value markers for EpiSwitch^{™} PCR translation.

### Array design and processing

### Array Design

Genetic loci are processed using the SII software (currently v3.2) to:
- Pull out the sequence of the genome at these specific genetic loci (gene sequence with 50kb upstream and 20kb downstream)
- Define the probability that a sequence within this region is involved in CCs
- Cut the sequence using a specific RE
- Determine which restriction fragments are likely to interact in a certain orientation
- Rank the likelihood of different CCs interacting together.
- Determine array size and therefore number of probe positions available (x)
- Pull out x/4 interactions.
- For each interaction define sequence of 30bp to restriction site from part 1 and 30bp to restriction site of part 2. Check those regions are not repeats, if so exclude and take next interaction down on the list. Join both 30bp to define probe.
- Create list of x/4 probes plus defined control probes and replicate 4 times to create list to be created on array
- Upload list of probes onto Agilent Sure design website for custom CGH array.
- Use probe group to design Agilent custom CGH array.

### Array Processing

- Process samples using EpiSwitch^{™} Standard Operating Procedure (SOP) for template production.
- Clean up with ethanol precipitation by array processing laboratory.
- Process samples as per Agilent SureTag complete DNA labelling kit - Agilent Oligonucleotide Array-based CGH for Genomic DNA Analysis Enzymatic labelling for Blood, Cells or Tissues
- Scan using Agilent C Scanner using Agilent feature extraction software.

*EpiSwitch^{™}* biomarker signatures demonstrate high robustness, sensitivity and specificity in the stratification of complex disease phenotypes. This technology takes advantage of the latest breakthroughs in the science of epigenetics, monitoring and evaluation of chromosome conformation signatures as a highly informative class of epigenetic biomarkers. Current research methods deployed in academic environment require from 3 to 7 days for biochemical processing of cellular material in order to detect CCSs. Those procedures have limited sensitivity, and reproducibility; and furthermore, do not have the benefit of the targeted insight provided by the *EpiSwitch*^{™} *Analytical Package* at the design stage.

### EpiSwitch^{™} Array in silico marker identification

CCS sites across the genome are directly evaluated by the EpiSwitch^{™} Array on clinical samples from testing cohorts for identification of all relevant stratifying lead biomarkers. The EpiSwitch^{™} Array platform is used for marker identification due to its high-throughput capacity, and its ability to screen large numbers of loci rapidly. The array used was the Agilent custom-CGH array, which allows markers identified through the *in silico* software to be interrogated.

### EpiSwitch^{™} PCR

Potential markers identified by *EpiSwitch*^{™} *Array* are then validated either by *EpiSwitch*^{™} *PCR* or DNA sequencers (i.e. Roche 454, Nanopore MiniON, etc.). The top PCR markers which are statistically significant and display the best reproducibility are selected for further reduction into the final *EpiSwitch^{™}* Signature Set, and validated on an independent cohort of samples. *EpiSwitch*^{™} *PCR* can be performed by a trained technician following a standardised operating procedure protocol established. All protocols and manufacture of reagents are performed under ISO 13485 and 9001 accreditation to ensure the quality of the work and the ability to transfer the protocols. *EpiSwitch*^{™} *PCR* and *EpiSwitch*^{™} *Array* biomarker platforms are compatible with analysis of both whole blood and cell lines. The tests are sensitive enough to detect abnormalities in very low copy numbers using small volumes of blood.

### Use of a Classifier

The method of the invention may include analysis of the chromosome interactions identified in the individual, for example using a classifier, which may increase performance, such as sensitivity or specificity. The classifier is typically one that has been 'trained' on samples from the population and such training may assist the classifier to detect any responder group mentioned herein.

The invention is illustrated by the following:

### Examples

### Example 1. Development of a Universal Marker Set and a Marker Set for Detecting Hyper-progressors

In working on populations of patients undergoing immunotherapy for cancer two distinct marker sets were developed: one is a universal marker set that allows responsiveness to therapy to be detected across a range of cancers and specific therapies, and the second is a marker set that detects hyper-progressors who should never be treated with particular types of immunotherapy.

We have now defined a specialised distinct and optimised panel of 11 biomarkers (the universal set), from a few hundreds identified in an original array screen and later tested on specific cohorts of patients. The unique feature of each of these 11 markers is that each of them is statistically significant (as part of the discovered core) across all PD-1/PD-L1 cases in all tested oncological indications, defining a universal core of response/non-response to treatment by PD-1/PD-L1. A classifier using these 11 markers works very robustly as a distinct performance entity across all tested patient cohorts.

As background to the present work, Figure 1 shows performance of baseline prediction of response /non-response for avelumab (PD-L1) in NSCLC for based on a large set of markers tested.

In contrast, for the universal 11 marker set the list of all treatments by various therapeutic assets PD-1/PD-L1 and various oncological indications we have worked with is shown in List A below: - PD-1 and PD-L1 assets, such as pembrolizumab, durvalumab, avelumab, atezolizumab, in melanoma, NSCLC, Lung, HCC, Bladder, Prostate, NPC, Parotid Gland, Alveolar Soft Part Sarcoma. The universal 11 marker classifier works well across all those cohorts and identifies universal profile that delivers robust baseline classification for response/non-response, irrespective of which exactly PD-1 or PD-L1 treatment and what exactly type of cancer was tested. We capture with 11 markers a very specific conducive/non-conducive epigenetic systemic network set up of features which define outcomes in immune-checkpoint therapies.

Turning to the second set of markers, a very serious issue in cancer immunotherapy is the consistent presence of a subgroup of patients who should never be treated with PD-1/PD-L1 therapies. They are called hyper-progressors (or super-progressors), where progressor means progression into disease. These patients upon treatment react very differently - their rate of tumour growth shoots up and they die very quickly, essentially in a matter of weeks.

Hyper-progressors can be defined as patients who respond adversely to immuno-checkpoint immuno-oncology treatment by demonstrating significant reduction in either progression-free survival (as a measure of survival in response to drug treatment) (PFS<60 days) or overall survival (OS<150 days).

Average trials demonstrate between 8-15% of their patients as showing a super-progressor profile. Currently, there are no means to identify and exclude these patients to prevent serious adverse effect of the immunotherapy. In most studies hyper-progressors are categorised with the bigger group of non-responders, also termed as progressors/progressive disease (PD). Most of non-responders are patients who do not benefit from immunotherapy. Today, the use of checkpoint inhibitors is justified by the overall benefits among the percentage of patients who respond to immunotherapy (10-70%).

Here we utilized patients from immunotherapy cohorts, focusing on those with a PFS/OS within the range of hyper-progressors and those beyond those survival time limits (marked S as "Standard" in the slides and tables). The hyper-progressor markers identify patient profile that is predicted to demonstrate short PFS/OS upon the treatment. On a group of 32 patients (equal arms of H and S) tested before treatment, when compared to PFS and OS after the treatment 11 super-progressor markers predicted correctly 15 out of 17 H (sensitivity 0.88), 14/15 (specificity 0.93), 15/16 (PPV 0.94), 14/16 (0.875).

These markers are specifically selected to identify and exclude patients prior to treatment on the basis of a predicted severe reduction in their survival as a consequence of immunotherapy. This could be seen as a subgroup of the bigger cohort of non-responders that could be identified and predicted by the universal 11 marker set.

Whilst the present work has been carried out on patients with melanoma, lung cancer, hepatocellular carcinoma (liver cancer), bladder , prostate, nasal cancer, parotid gland (salivary gland cancer), alveolar soft part sarcoma (soft tissue cancer), it also applies to other cancers where immune-checkpoint inhibitors PD-1/PD-L1 are used for therapy, such as breast cancer, cervical cancer, colon cancer, head and neck cancer, Hodgkin lymphoma, kidney cancer, stomach cancer, rectal cancer, and any solid tumour.

### Mechanism of Detection

The marker sets that have been identified capture a network of deregulations at the level of cellular 3D genomics, which reflects a network of deregulated cell types acting in conjunction to sustain and advance the pathological or physiological phenotype of cancer. So far, observing statistically significant chromosome conformations as evidence of deregulation in conjunction with cell sub-typing CD loci, we can state that observed universal signatures contain and represent deregulations in T cells, NK (natural killer) cells, macrophages, B cells and dendritic cells (DC). This emphasizes the role played by the specific set up at cellular level of the adaptive and innate immune system in individual patients as part of the cancer-host interaction, which defines disease progression (hyper-progressors) and responsiveness to immuno-checkpoint inhibitors PD-1/PD-L1.

### Methods

Initial studies of chromosome interactions were carried out on the following populations (work described in Figure 1):

### List A - Initial work

- 16 anti-PD-1 (Pembrolizumab) Melanoma cohort
- 16 anti-PD-L1 NSCLC cohort
- 99 anti-PD-L1 NSCLC cohort
- 49 anti-PD-1 NSCLC cohort
- 50 anti-PD-1 and combined therapy NSCLC cohort
- 48 anti-PD-1 (pembrolizumab) Melanoma cohort, including hyper-progressors
- 550 anti-PD-L1 Urethral Cancer

### Observational Longitudinal:

- anti-PD-L1 (Durvalumab, Atezolizumab) and anti-PD1(Pembrolizumab)
- Lung, HCC, Bladder, Prostate, NPC, Parotid Gland, Alveolar Soft Part Sarcoma.

The marker sets were developed using the following patients:

### Training 80 patients all NSCLC

Mixture of 1L, 2L Avelumab (54) and 2L Pembrolizumab (36)

### Test: 38 Patients all NSCLC

Mixture of 1L, 2L Avelumab (27) and 2L Pembrolizumab (11)

### Test: 20 Samples Mixture

Mixture 2L patients with different Check point inhibitors and different solid tumours
Atezolizumab (7), Durvalumab (3), and Pembrolizumab (10)
Lung (13), NPC (2), HCC (2), Bladder (1), Alveolar Sarcoma (1), and Parotid Gland (1)
Total 138 samples.

For testing the universal markers a blind cohort was collected to test the feature of Non-Response. This cohort was collected in Malaysia and consisted of 21 patients who all provided blood samples at baseline prior to immunotherapy. All patients had previous lines of therapy. 3 check-points were used: Atezolizumab (anti-PD-L1), Durvalumab (anti-PD-L1) and Pembrolizumab (anti-PD-1). There were 7 disease indications: Lung, HCC, Bladder, Prostate, NPC, Parotid Gland and Alveolar soft part sarcoma. 11 of the patients had multiple collections: 2-4. 3 patients had up to 4 collections. The ethnicity of the Patients was either Han Chinese or Indonesian.

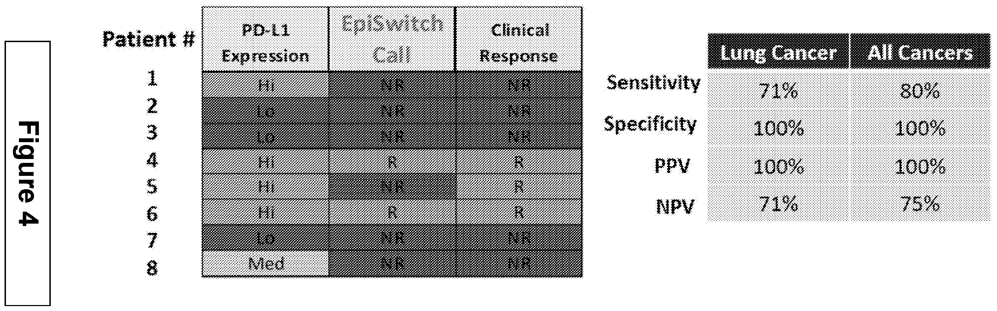

Figure 4 shows the high concordance between baseline EpiSwitch calls, PD-L1 expression and observed clinical response.

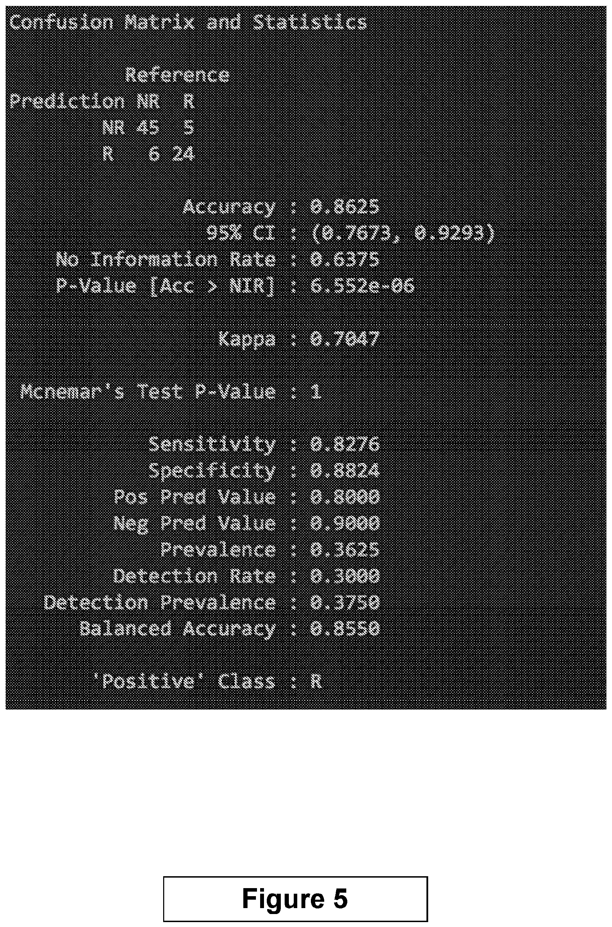

Figure 5 shows data for a training set for an 11 marker model based on 80 NSCLC patients who are a mixture of 1L, 2L Avelumab (54) and 2L Pembrolizumab (36).

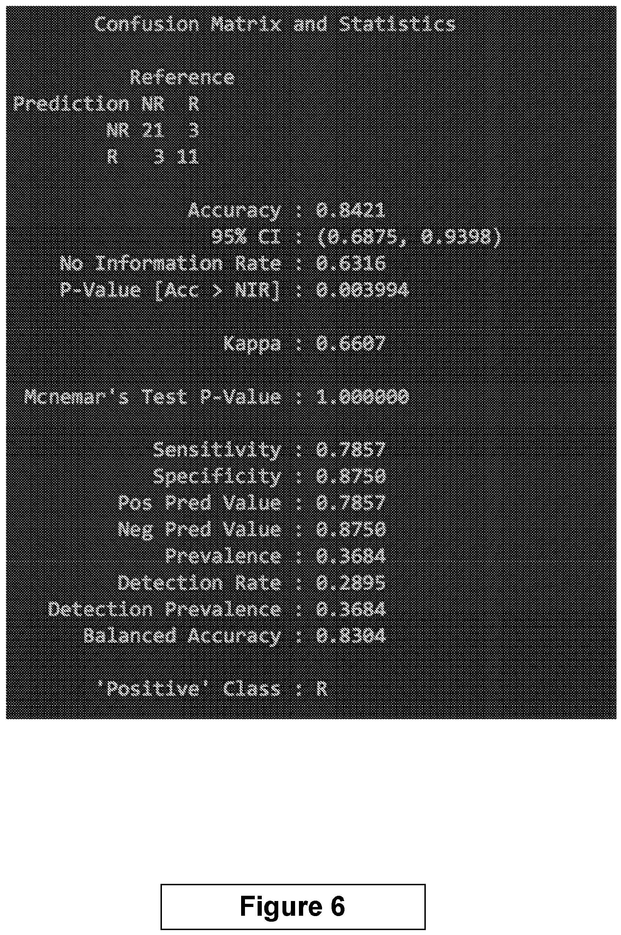

Figure 6 shows data for a test set of the 11 marker model based on 38 NSCLC patients who are a mixture of 1L, 2L Avelumab (27) and 2L Pembrolizumab (11).

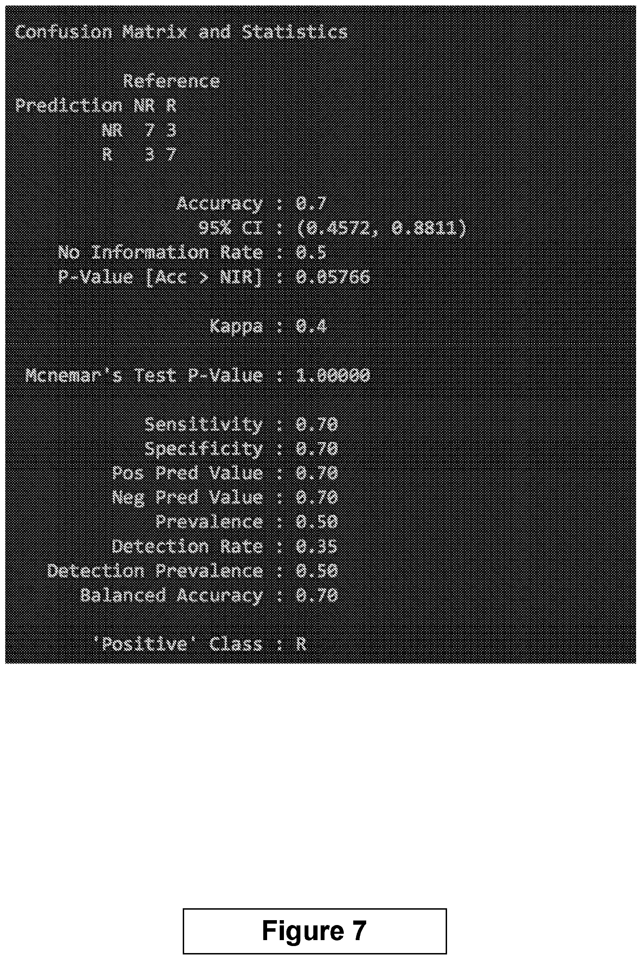

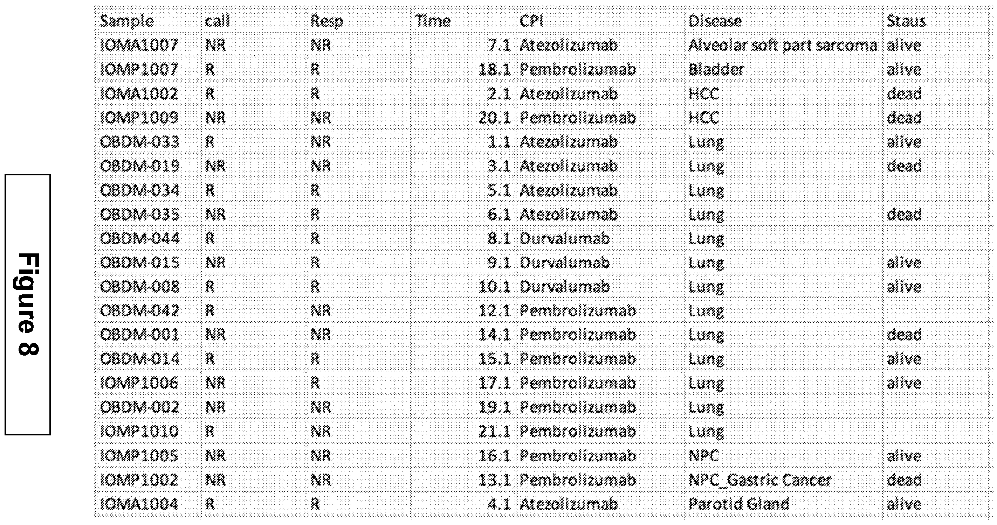

Figures 7 and 8 show data for a second test set for the Malaysian Observational Study looking a mixture of CPI and tumours.

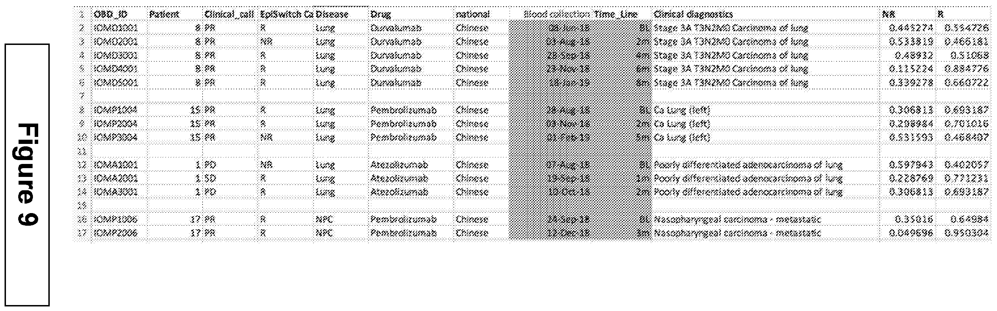

Figure 9 shows the calls for the patients who had multiple collections. The calls over the time points are largely consistent and concordant:
Patient 12 on Durvalumab shows the profile of Responder over 4 collections, and a score over the second collection entering into grey zone R/NR. Overall profile of probabilities actually get stronger over time for Responder.

Patient 1 on Atezolizumab, shows an interesting initial non-response, but becomes a late responder.

Patient 17 on Pembrolizumab is an NPC patient, and shows Response profile over both samplings.

This emphasizes the ability of EpiSwitch^{™} Markers to find Non-Responders and Responders to capture common features of host response profile for multi check-point inhibitors under diverse oncological conditions.

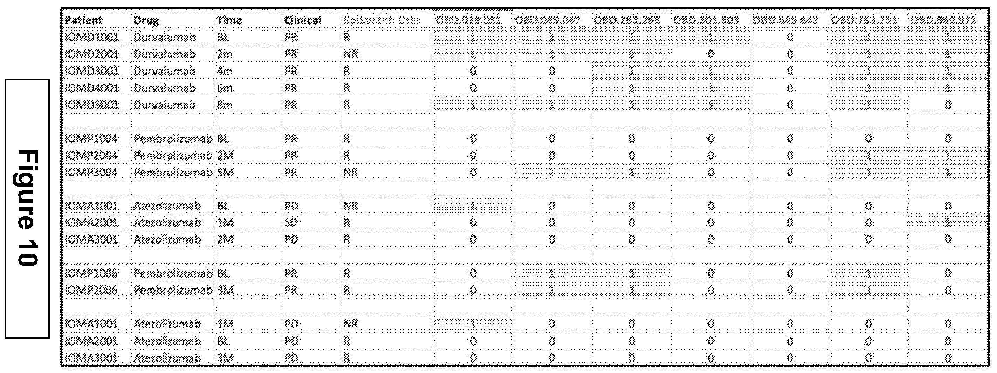

Figure 10 shows EpiSwitch calls for patients sampled over multiple time points, with OBD.261.263 and OBD.301.303 representing universal response markers and OBD.029.031, OBD.045.047, OBD.645.647, OBD.753.755 and OBD.8.69.871 representing universal non-response markers.

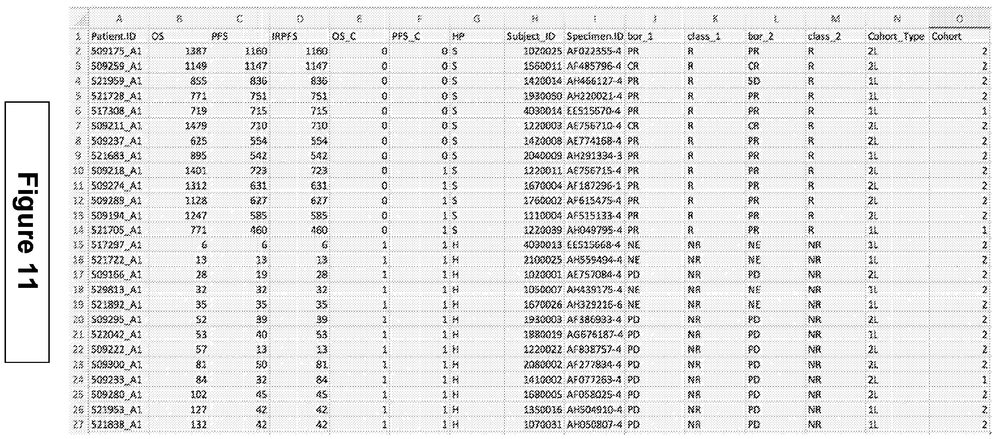

For the work relating to hyper-progressors, Figure 11 shows sample selection with hyper-progressors selected based on having a PFS<=60 days (2months) and OS <= 150 days (4.5months), the survival group (S) were selected as they mostly all had over 1 year PFS.

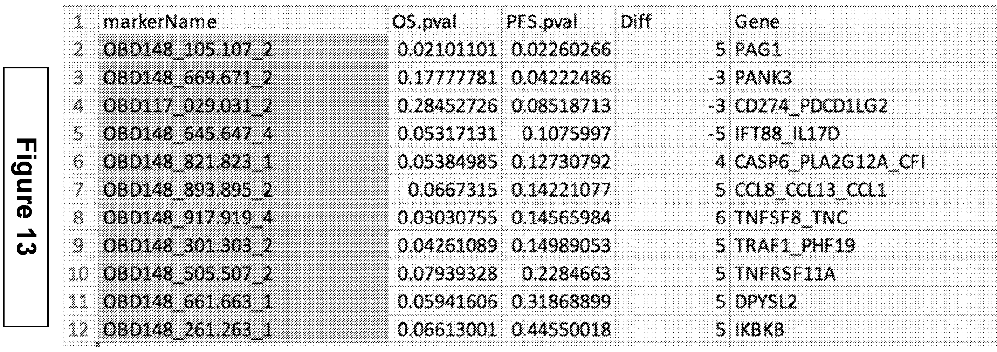

Figure 13 shows 11 EpiSwitch CCSs selected from a total of 60, markers selected based on binary difference, OS and PFS rank log analysis. The associated genetic locations are also shown. Figures 10 and 11 show a pathway analysis of the genetic locations.

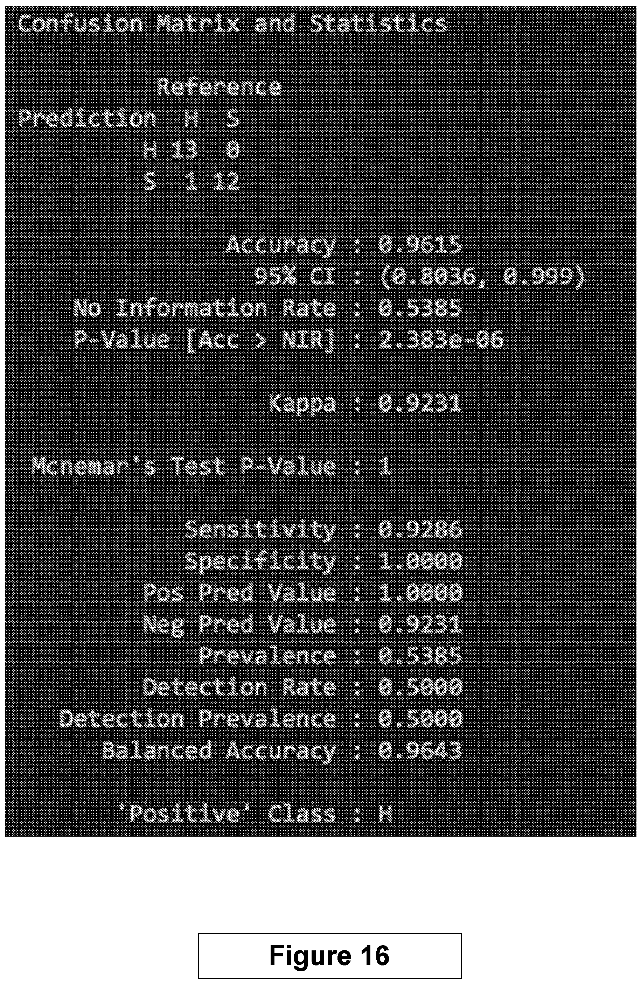

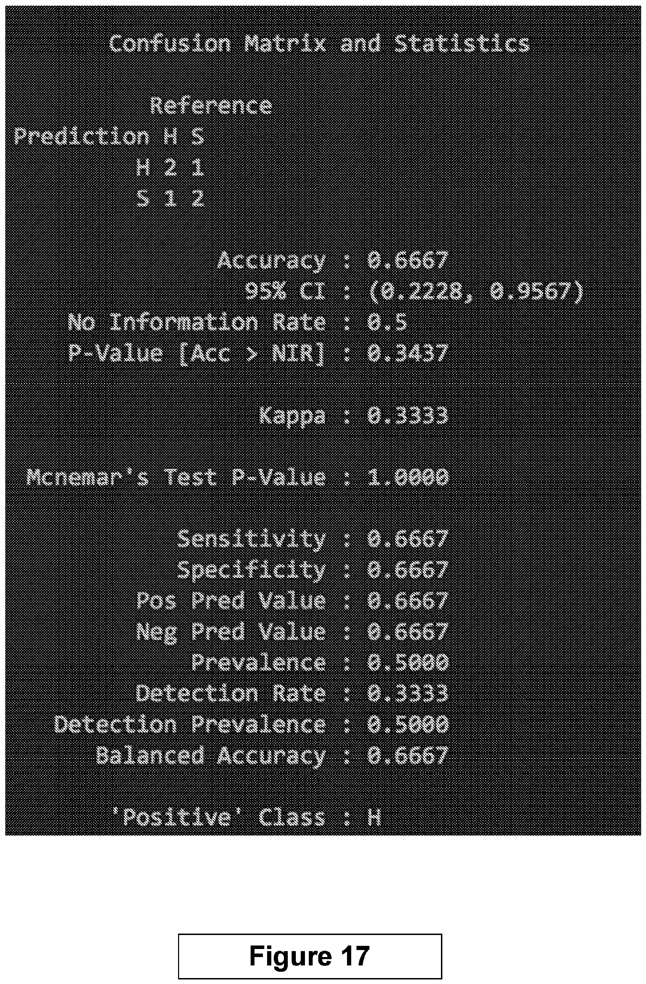

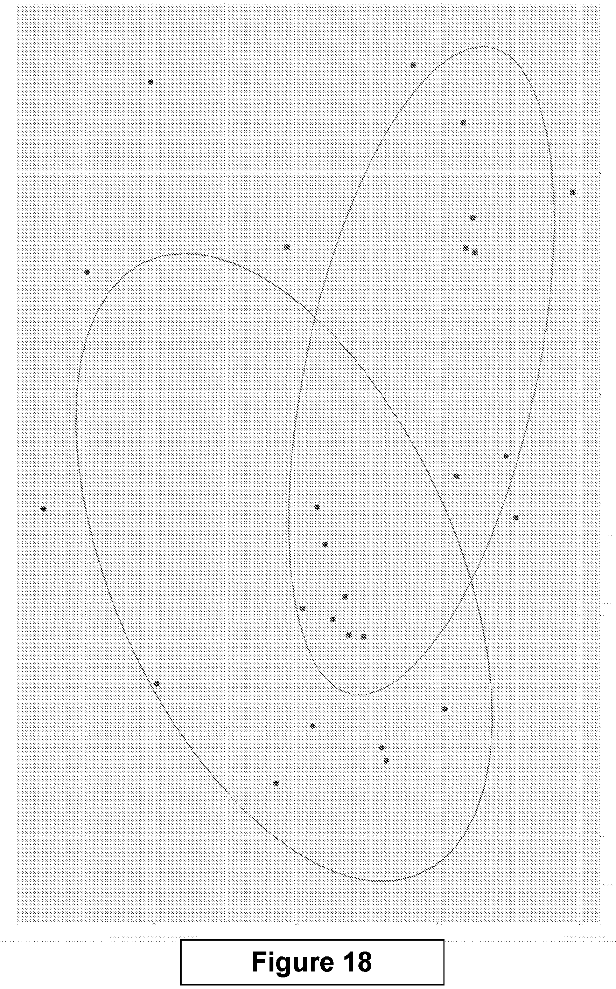

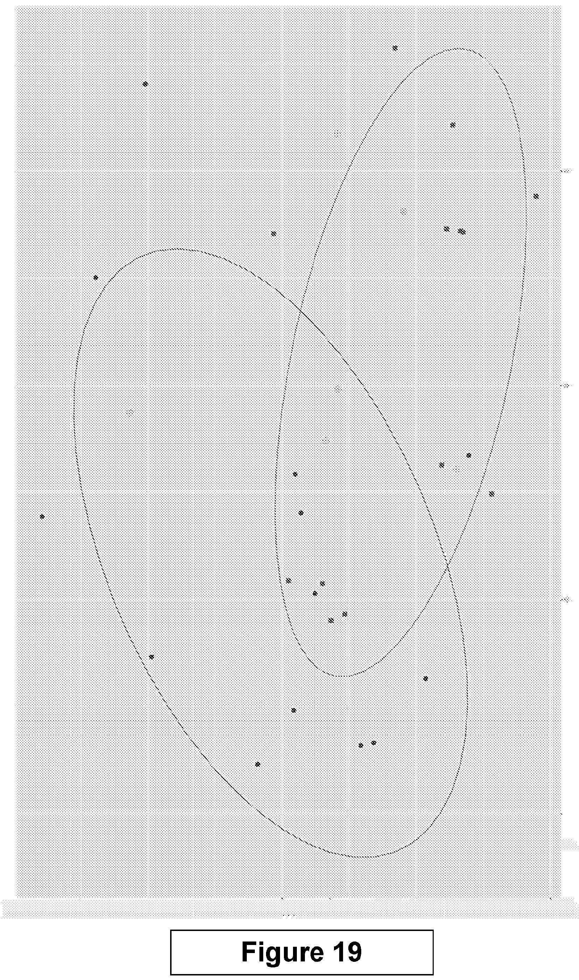

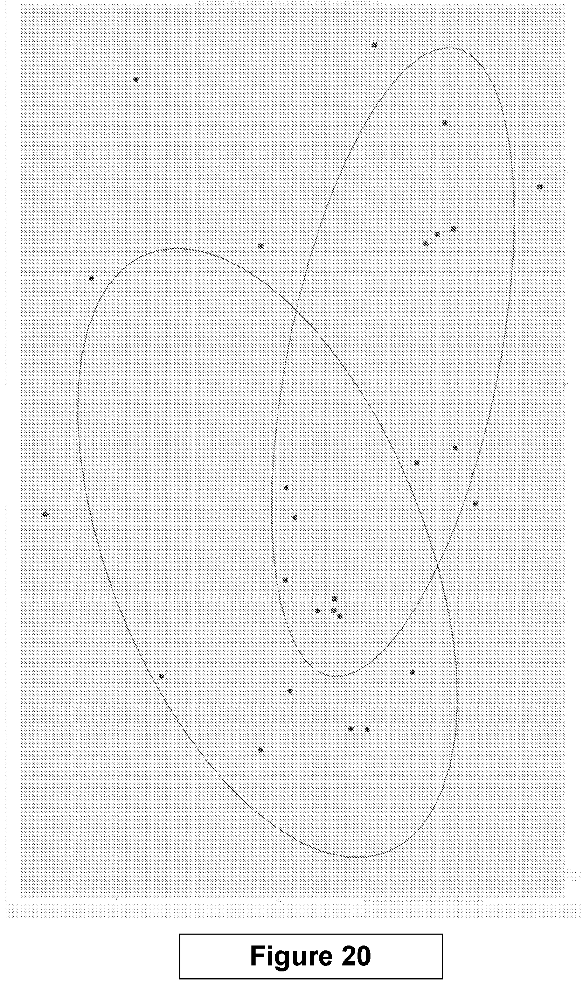

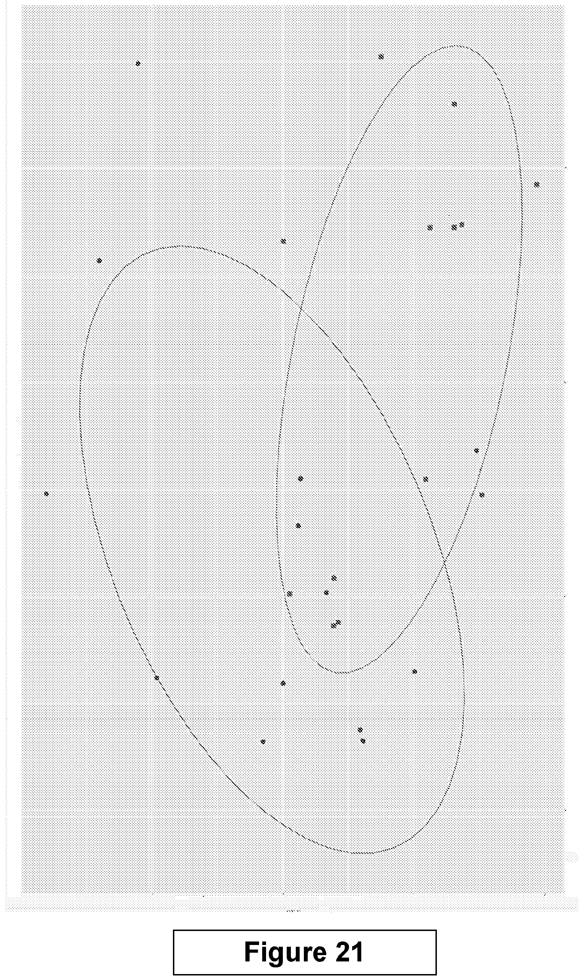

Figure 16 shows data for a training set for hyper-progressors: an XGBoost 11 marker model. Figure 17 shows data for a test test for hyper-progressors with 6 samples excluded from marker selection. Figure 18 shows a logistic principal component analysis (PCA) of a training set. Figure 19 shows a logistic PCA of the training set with predicted test samples. This is a second classification approach and is in concordance with XGBoost. Figure 20 shows the logistic PCA of the training set with PFS as label. Figure 21 shows the logistic PCA of the training set with OS as label.

### Approach to Analysing the Patients

A specific approach was taken to the way the patient population was analysed. The relevant patient cohorts had either been given prior therapy (one round of cisplatin-based chemotherapy) or had been treated with check-point inhibitors only. We also only looked at patients with a defined response, so either complete response, partial response or no response. We removed from the analysis patients who experienced stable disease.

The EpiSwitch^{™} nested PCR platform data output was analysed with multiple statistical techniques, including, but not limited to, established univariate (Fishers Exact test) and multivariant (permutated GLMNET, Random Forest with SHapley Additive exPlanations values (SHAP)), procedures.

For development of the diagnostic and prognostic EpiSwitch^{™} classifiers, the following statistical analysis were used: (i) XGBoost: A gradient boosted decision tree algorithm. An ensemble of weak decision tree models is generated and combined to produce one strong classification model (level wise tree growth); (ii) Logistic Principal Component Analysis (PCA): Principal component Analysis optimized to use binary data; and (iii) GLMNET: Generalized linear model fitted via penalized maximum likelihood technique (iv) LightGBM: gradient boosting framework that uses tree based learning algorithms (vertical leaf tree growth).

A SHAP analysis is shown below for universal marker set.

| Marker | Shap1 | Rank_1 | Shap2 | Rank_2 | Shap3 | Rank_3 | Average |
|---|---|---|---|---|---|---|---|
| OBD117_029.031_2 | 0.939792 | 3 | 0.898573 | 5 | 1.210319 | 2 | 3 |
| OBD148_045.047_1 | 0.516578 | 6 | 1.335644 | 1 | 1.457146 | 1 | 3 |
| OBD148_869.871_2 | 0.989211 | 1 | 1.034225 | 3 | 0.942668 | 4 | 3 |
| OBD148_929.931_2 | 0.971228 | 2 | 0.998071 | 4 | 1.128059 | 3 | 3 |
| OBD148_261.263_1 | 0.400625 | 8 | 1.172577 | 2 | 0.681067 | 5 | 5 |
| OBD148_753.755_2 | 0.798995 | 4 | 0.549536 | 6 | 0.679744 | 6 | 5 |
| OBD148_397.399_1 | 0.428492 | 7 | 0.254456 | 11 | 0.567525 | 7 | 8 |
| OBD148_645.647_4 | 0.675223 | 5 | 0.407179 | 9 | 0.48619 | 9 | 8 |
| OBD148_301.303_2 | 0.329259 | 10 | 0.541 | 7 | 0.263039 | 11 | 9 |
| OBD148_777.779_2 | 0.396967 | 9 | 0.311894 | 10 | 0.520779 | 8 | 9 |
| OBD148_821.823_2 | 0.180874 | 11 | 0.461653 | 8 | 0.335826 | 10 | 10 |

Markers ranked by their SHAP scores, with the best marker being OBD117_029_0.31. The SHAP (SHapley Additive exPlanations) value is a united approach to explaining the output of any machine learning model. There are three important benefits.

The first one is global interpretability - the collective SHAP values can show how much each predictor contributes, either positively or negatively, to the target variable. This is like the variable importance plot but it is able to show the positive or negative relationship for each variable with the target.

The second benefit is local interpretability - each observation gets its own set of SHAP values This greatly increases its transparency. We can explain why a case receives its prediction and the contributions of the predictors. Traditional variable importance algorithms only show the results across the entire population but not on each individual case. The local interpretability enables us to pinpoint and contrast the impacts of the factors.

Third, the SHAP values can be calculated for any tree-based model (our model is an XGboost, boosted tree based model), while other methods use linear regression or logistic regression models as the surrogate models.

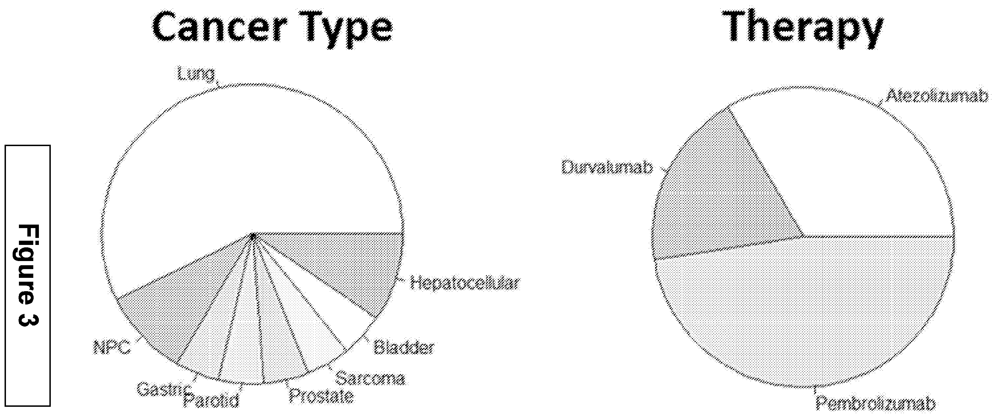

This represents the analytical pipeline for marker selection. The high performance of the two markers sets is shown in the figures and tables. In particular for the universal marker set all 7 types of cancer shown in Figure 3 were represented in 21 patients observed longitudinally, where the performance was 100% on specificity and positive predictive value across the cohort.

### Example 2. Further Work Leading to Development of the Set of Markers Shown in Table 8

Immune checkpoint inhibitors are a class of drugs targeting a narrow set of proteins in a specific regulatory network present in immune cells, like T cells, and some cancer cells of the patients. The checkpoint protein targets and the network they control help keep immune responses from being too strong and provide additional protection from the autoimmune conditions, but in the case of cancer can keep T cells from killing cancer cells. Use of immune checkpoint inhibitors helps to reactivate the immune response in cancer patients, with efficacious outcome and improved survival in patients.

Immune checkpoint inhibitors act by resetting and activating immune response by targeting either: 1) PD-1 (Nivolumab, Pembrolizumab, Cemiplimab, Camrelizumab, Tislelizumab, Sasanlimab); or 2) PD-1 ligand, called PD-L1 (Avelumab, Atezolizumab and Durvalumab).

The present work has asked several questions, including if, taking into account the role played by immune system of the patient in successful response to immune checkpoint inhibitors and the limited number of targets for the therapy, particularly PD-1 receptor and its ligand PD-L1, one can discover and validate EpiSwitch biomarkers in a qPCR format of detection for baseline patients that would universally predict response/non-response to treatment in advance, irrespective of the type of the checkpoint inhibitor used and across the spectrum on oncological conditions.

MIQE compliant qPCR format is the standard for clinical PCR based tests. This format is very different from nested PCR or array format, due to its limitations on primer and probes sequence designs and continuous range of detection, traditionally measured though Cq cycle numbers.

The following steps were undertaken as part of discovery and validation of these biomarkers (table 9 shows patient data):
Stage 1. From previous markers identified by arrays, the top 24 markers were identified that satisfied initial theoretical restrictions and requirements for sequence designs on qPCR primers and probes, i.e. unique sequences for detection, correct annealing temperature of annealing for primers and probes, overlapping 3C juncture, for the detection of the chromosome conformations. At the experimental stage, the designs for 20 markers passed quality control and satisfactory optimization on temperature gradient.

Stage 2. qPCR formatted marker leads were used to identify if there is a minimal number of biomarkers which as a signature will have strong stratification power for predicting response and non-response across an extensive cohort for patients treated with immune checkpoint inhibitors - Pembrolizumab, Avelumab, Atezolizumab, Durvalumab against each of three targets (PD-1, PD-L1) from a broad selection of oncological conditions: melanoma, non-small cell lung cancer, urethral, HCC, Bladder, Prostate, NPC, Parotid Gland, Alveolar Soft Part Sarcoma, Nasopharyngeal carcinoma, vulval carcinoma, colon cancer, Breast Cancer, Bone Cancer, Brain Cancer, sagittal sinus Carcinoma, lymphoma, larynx cancer, Cervix Cancer, Oral Cavity Carcinoma.

Stage 3: In screen 1 all 20 markers were first evaluated on pooled DNA templates from 3 patients clinical outcome categories: responders, non-responders and stable diseases. The sample cohorts represented patients with various type of cancers (see annotations attached) who received various immuno-checkpoints inhibitors as monotherapies: Avelumab, Pembrolizumab, Atezolizumab and Durvalumab.

Stage 4: In screen 2, the top 13 markers, shortlisted from screen 1 were evaluated on individual samples from the same patients used in screen 1: 24 patients/samples in total (see Table 9, patients shown with an asterisk).

The selection of best markers at stage 2 and 3 was carried out using a linear model. The linear model is fitted to the Cq values for each marker, comparing PR v PD, PR v SD and PD v SD. The coefficients of the fitted models describe the differences between the CCSs in each of the comparisons. The linear models are then used to compute moderated t-statistics log-odds of differential CCSs by empirical Bayes moderation of the standard errors towards a global value (0 log or 1 linear). The markers are then ranked by the adjusted p value and their CCSs abundance difference between the groups. Markers between PR v PD are given more weight.

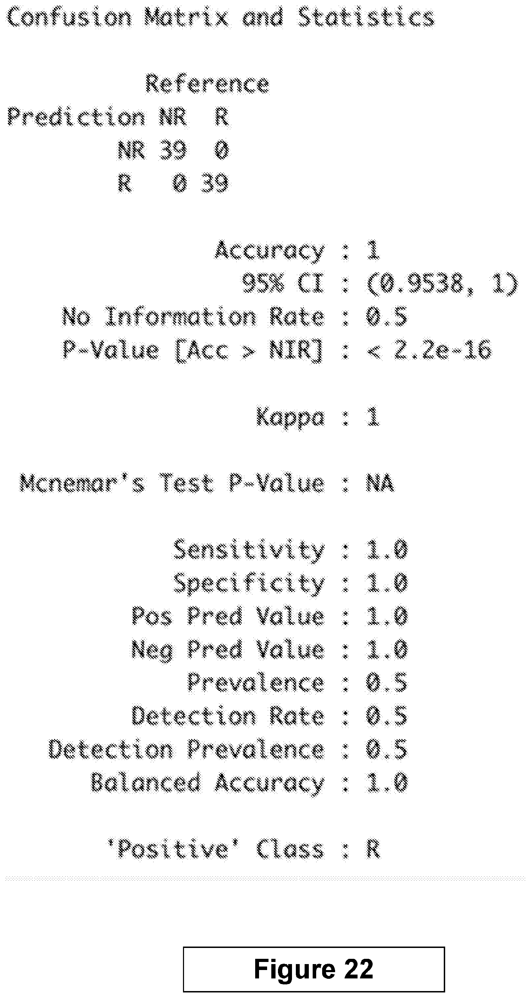

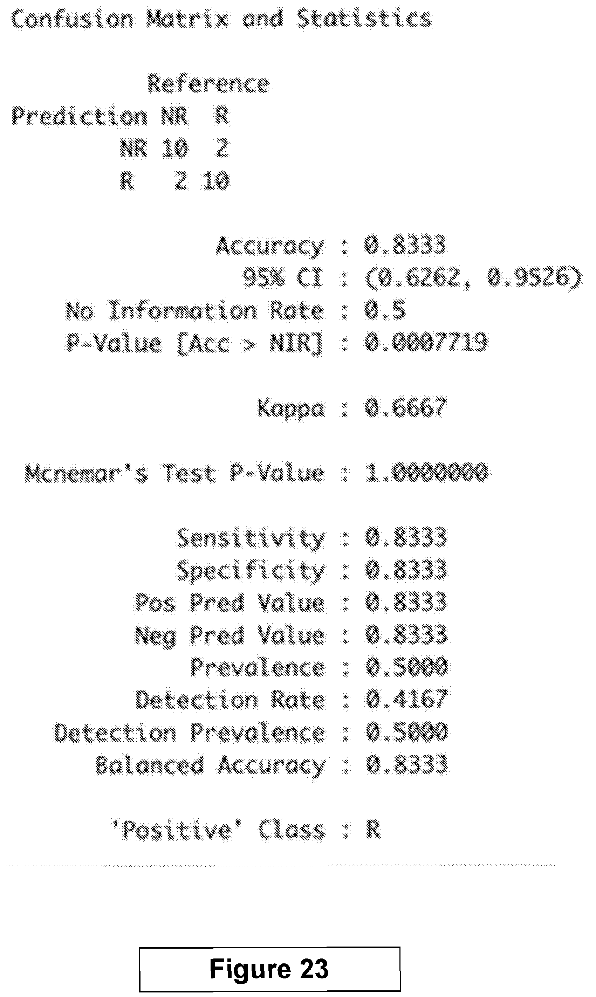

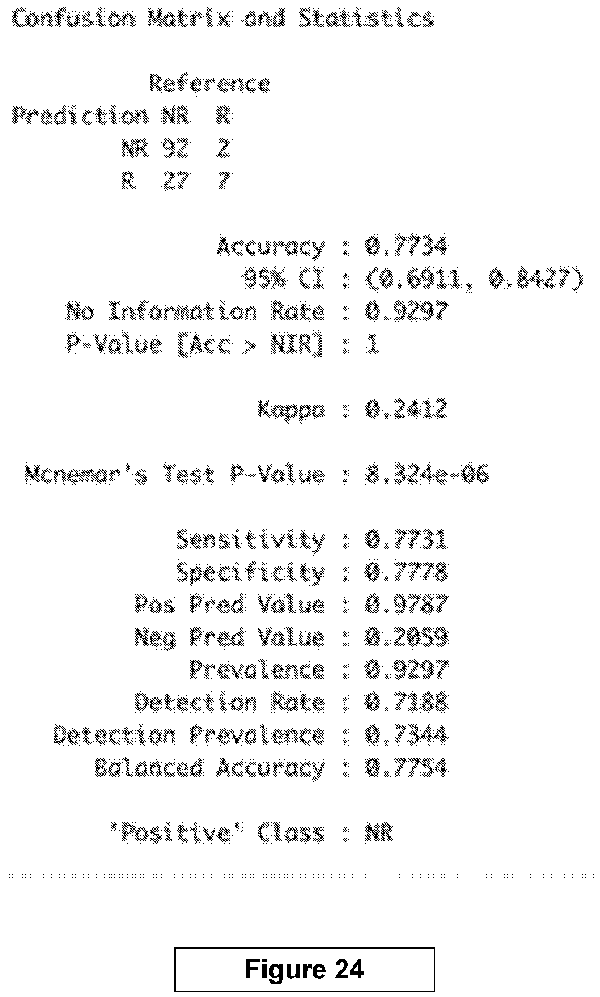

Stage 5: In screen 3 the top 8 markers (shortlisted from screen 2) were validated on IO samples consisting of patients with the following clinical outcomes: 55% NR (non-responders), 24% SD (stable disease, according to the regulatory ruling and clinical practice, SD should be subject to IO therapy just as the group of potential responders), 20% R (responders, also referred as PR for patrial responders) and 1% CR (complete responders). The efficacy of stratification by the model based on 8 markers is shown in Figures 22 to 24. Predictive Value (PPV) = 100xTP/(TP+FP). Negative Predictive Value (NPV) = 100xTN/(FN+TN).

The immunotherapy checkpoint classifier is built using CatBoost. Catboost is a member of the Gradient Boosted Decision Trees (GBDT's) machine learning ensemble techniques (see Hancock and Khoshgoftaar; J. Big Data (2020) 7:94).

### Types of Format for the Test

Both nested and qPCR formats impose stringent limitations on which of the array-based marker leads could be successfully translated into one or the other format. This is particularly true in case of qPCR format where we have to determine if we can use two primers and a fluorescent probe over the 3C juncture (this one is similar to the array probe), which 1) have unique sequence across the whole genome for specific detection, 2) have very similar annealing temperatures, 3) show how efficacy in amplification in single PCR procedure, and not in two sequential reactions as required by nested PCR (first with one pair of primers, second one with another pair of primers). Requirements for qPCR are much more stringent and selective.

### Conclusions

Based on qPCR evaluation of 8 conditional chromatin-conformations as blood-based regulatory biomarkers, individuals could be evaluated for likelihood of response to Immune Checkpoint Inhibitor (ICI) monotherapies. In the cross talk between patient tumour microenvironment and patient immune system, the PD-1 pathway, comprising receptor Programmed Death 1 (Pd-1) and its ligand PD-L1, mediates local immunosuppression in the tumour microenvironment. The present work directly relates to ICI that were antagonists targeting PD-1 (Pembrolizumab) or its ligand, PD-L1 (Atezolizumab, Avelumab & Durvalumab). Stratification based on 8 marker classifier places patients into groups of likely responders or non-responders to ICI monotherapy, prior to the application of the therapy. The classification is applied across all ICI monotherapies against PD-L1 and its ligand PD-L1, in the context of all oncological indications used in ICI monotherapy treatments.

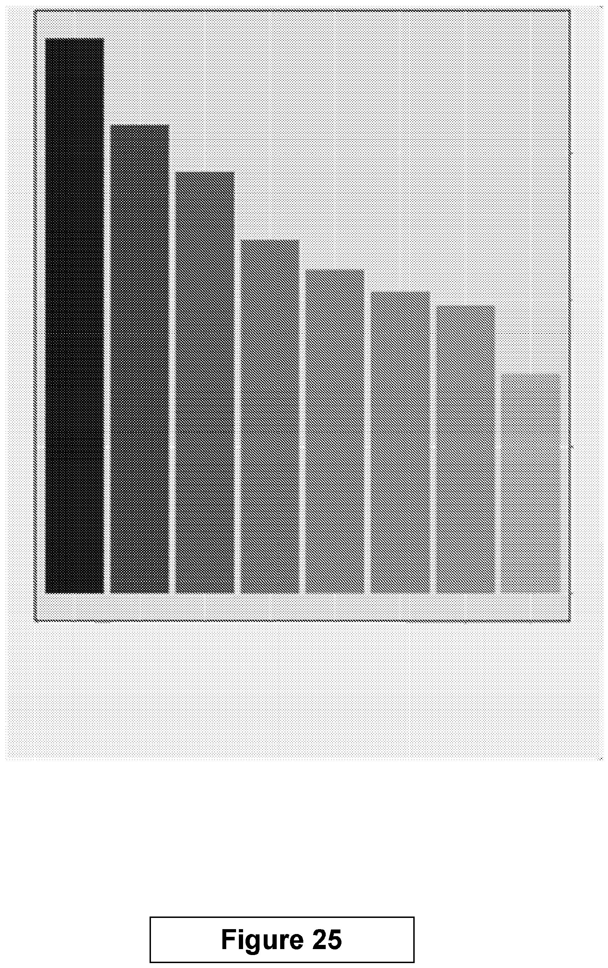

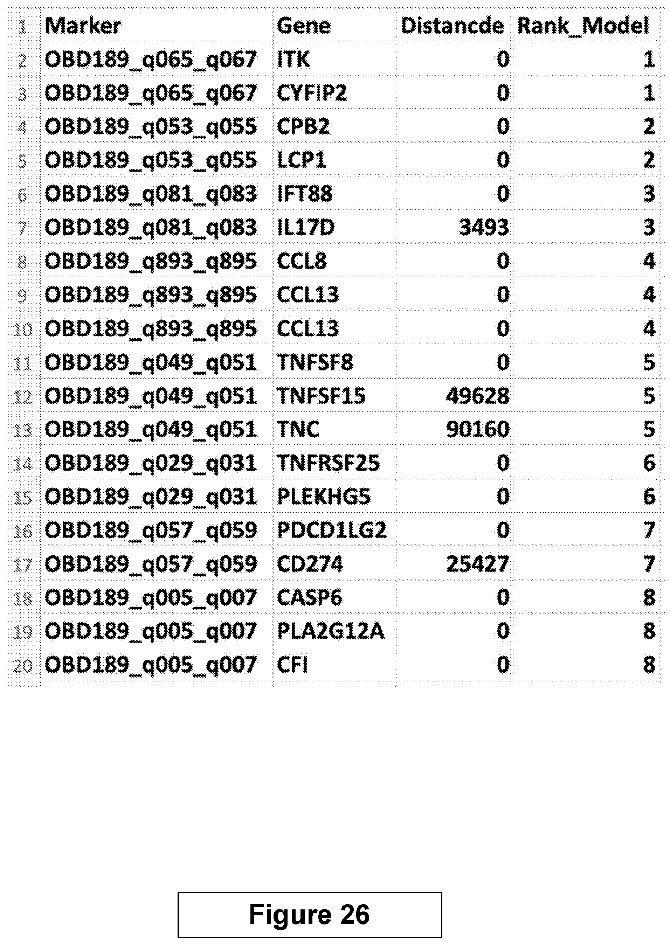

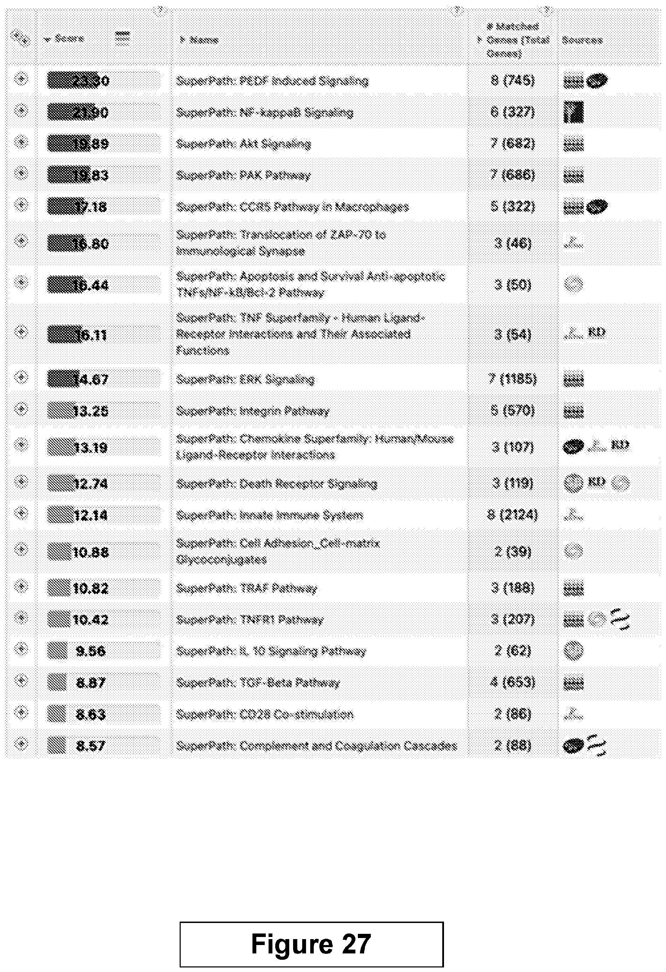

Figures 25 to 27 describe characteristics of the identified chromosome interactions markers. Figure 25 shows the importance of markers in terms of their power in the model. Figure 26 shows the genetic locations. Figure 27 shows the pathways the genes are associated with. These are pathways implicated in checkpoint response. This indicates the model predictive, and it is noteworthy the markers within the model have biological relevance. In fact, one of the markers is located between PD-L1 and PD-L2 (q057_q059).

**Table 1.a1**

| | Probe | RP/Rsum | FC |
|---|---|---|---|
| 1 | PDCD1LG2_9_5495992_5498009_5563479_5572986_RR | 6499 | -1.13 |
| 2 | MYC_8_127691489_127694045_127738939_127740424_FR | 6220 | 1.15 |
| 3 | IKBKB_8_42264241_42271203_42331044_42332799_FR | 1585 | 1.3 |
| 4 | ORF712_9_120888366_120893320_120913546_120919710_RR | 8471 | 1.11 |
| 5 | ITK_5_157178319_157181048_157266725_157271762_RR | 4605 | 1.15 |
| 6 | IL17D_13_20664875_20671757_20688261_20691044_FF | 6848 | -1.1 |
| 7 | IKBKB_8_42264241_42271203_42290979_42292124_FR | 2465 | 1.25 |
| 8 | IGF1R_15_98731539_98737034_98785670_98790114_FF | 5450 | 1.15 |
| 9 | CASP6_4_109703339_109705583_109735036_109741090_RF | 1727 | 1.29 |
| 10 | TRAF2_9_136904007_136906211_136939587_136941363_RF | 5090 | 1.16 |
| 11 | ORF313_13_20664875_20671757_20695143_20698635_FF | 4910 | -1.16 |

**Table 1.a2**

| | PFP | P.value | FDR |
|---|---|---|---|
| 1 | 0.3857 | 0.01934 | 0.188603463 |
| 2 | 0.3231 | 0.01577 | 0.169755579 |
| 3 | 0.001699 | 0.00000779 | 0.00118933 |
| 4 | 0.6186 | 0.06146 | 0.308076103 |
| 5 | 0.1332 | 0.003607 | 0.076416861 |
| 6 | 0.4249 | 0.02454 | 0.210154857 |
| 7 | 0.01154 | 0.0001149 | 0.00763801 |
| 8 | 0.2259 | 0.008388 | 0.12237213 |
| 9 | 0.002472 | 0.0000133 | 0.001705674 |
| 10 | 0.1832 | 0.005986 | 0.101918497 |
| 11 | 0.2061 | 0.004999 | 0.091747389 |

**Table 1.a3**

| | **Probe sequence** |
|---|---|
| | |

| | **60 mer** |
|---|---|
| 1 | ACAGTTATTAGAAAAATAAAACATTTGGTCGAACAGCAAAGAGAAGATATTCAACTGCGA |
| 2 | AGGGAGAACAAAAGAAGTTCCATCCATCTCGACGGAGTCCTCCCCGCAGGGCAGCCCCGA |
| 3 | CCACCCCCGCCCCGGGGGAGTCGCCCGGTCGACCCCCTGACATGGGGCTGCCTGGAGCAG |
| 4 | AGTGCTGGGTTCCACACCTCTCAGCTCTTCGACCTCCAGGTCCCCCGCCACTTCCACGGC |
| 5 | CAAAATCAAACACAAATCTAATCAAACTTCGATGTTTGGGGGCGGAGGGCTTTGATGAGA |
| 6 | TTAAAGAAGCTAATTTTAAAAATAAATGTCGAAGAGATTGTCACGTTAGAGTTATGTAAA |
| 7 | CCACCCCCGCCCCGGGGGAGTCGCCCGGTCGATTTCCAAAAGCTCACACATGGGTGCACA |
| 8 | CGTAGAACTAAGATGTATTCAAAGTCAGTCGAAATCACCTGTCCCGGCCTCTTTCCAAAC |
| 9 | GGGGCCTCCAGAGTCCCCTTTACAGGCATCGACGCCCCCTGCCTACCTGCCGGGTGCCCC |
| 10 | CCGCCTCACCTCCCGCATGGTCTTGAGGTCGAGCATGCAGCGCATCTGAGCAGTGAGGCT |
| 11 | TTAAAGAAGCTAATTTTAAAAATAAATGTCGAGGAGCATCTGGATTTAATGATAGTTCAA |

**Table 1.a4**

| | **Probe Location** | | | | |
|---|---|---|---|---|---|
| | **Chr** | **Start1** | **End1** | **Start2** | **End2** |
| 1 | 9 | 5495994 | 5496023 | 5563481 | 5563510 |
| 2 | 8 | 127694014 | 127694043 | 127738941 | 127738970 |
| 3 | 8 | 42271172 | 42271201 | 42331046 | 42331075 |
| 4 | 9 | 120888368 | 120888397 | 120913548 | 120913577 |
| 5 | 5 | 157178321 | 157178350 | 157266727 | 157266756 |
| 6 | 13 | 20671726 | 20671755 | 20691013 | 20691042 |
| 7 | 8 | 42271172 | 42271201 | 42290981 | 42291010 |
| 8 | 15 | 98737003 | 98737032 | 98790083 | 98790112 |
| 9 | 4 | 109703341 | 109703370 | 109741059 | 109741088 |
| 10 | 9 | 136904009 | 136904038 | 136941332 | 136941361 |
| 11 | 13 | 20671726 | 20671755 | 20698604 | 20698633 |

**Table 1.a5**

| | **4 kb Sequence Location** | | | | |
|---|---|---|---|---|---|
| | **Chr** | **Start1** | **End1** | **Start2** | **End2** |
| 1 | 9 | 5495994 | 5499993 | 5563481 | 5567480 |
| 2 | 8 | 127690044 | 127694043 | 127738941 | 127742940 |
| 3 | 8 | 42267202 | 42271201 | 42331046 | 42335045 |
| 4 | 9 | 120888368 | 120892367 | 120913548 | 120917547 |
| 5 | 5 | 157178321 | 157182320 | 157266727 | 157270726 |
| 6 | 13 | 20667756 | 20671755 | 20687043 | 20691042 |
| 7 | 8 | 42267202 | 42271201 | 42290981 | 42294980 |
| 8 | 15 | 98733033 | 98737032 | 98786113 | 98790112 |
| 9 | 4 | 109703341 | 109707340 | 109737089 | 109741088 |
| 10 | 9 | 136904009 | 136908008 | 136937362 | 136941361 |
| 11 | 13 | 20667756 | 20671755 | 20694634 | 20698633 |

**Table 1.a6**

| | Primer ID | Primer Sequence | Primer ID |
|---|---|---|---|
| 1 | OBD117_029 | CTCACTGCCCAACAGGCTAGAA | OBD117_031 |
| 2 | OBD148_045 | CCCTAAGCAACCACCTTGGACTG | OBD148_047 |
| 3 | OBD148_261 | CGGTGAGCACGGTCTGTCTACTT | OBD148_263 |
| 4 | OBD148_301 | CCCAGTTGTCCAGGTTGCTGCCT | OBD148_303 |
| 5 | OBD148_397 | GTCTCCTGAGGTGAAGCAAGAGG | OBD148_399 |
| 6 | OBD148_645 | TCTCTACTTCAGGCAGGCAGTGTAAG | OBD148_647 |
| 7 | OBD148_753 | GGTGTAACGGGGGTCATTTC | OBD148_755 |
| 8 | OBD148_777 | AATTCACCACACCCCAACAT | OBD148_779 |
| 9 | OBD148_821 | CAGACTAAGGGGCCTCCAGA | OBD148_823 |
| 10 | OBD148_869 | TGAAGAAGCACTCGTCGTTG | OBD148_871 |
| 11 | OBD148_929 | AGTTTTCCACCCCTTCTTCC | OBD148_931 |

**Table 1.a7**

| | Primer Sequence | Marker | Type |
|---|---|---|---|
| 1 | TCTTGACTCAGAGCCCACAACAA | OBD117_029.031 | NR |
| 2 | GCTTCGCTTACCAGAGTCGCTGC | OBD148_045.047 | NR |
| 3 | GTCCTGGGTCCTGGGTGAAAGTC | OBD148_261.263 | R |
| 4 | TGGAGCAGAACCTGTCAGACCTG | OBD148_301.303 | R |
| 5 | AGTCAGCCCACTCATCCCCTTCC | OBD148_397.399 | NR |
| 6 | GGGAGACCATTTCTGTTCACTCTGAG | OBD148_645.647 | NR |
| 7 | TGTGGGAACCATACCTGTGC | OBD148_753.755 | NR |
| 8 | CTCCGGAGGATTTCTGTGAA | OBD148_777.779 | NR |
| 9 | CGCAATCAGAACCAACTGGC | OBD148_821.823 | NR |
| 10 | AGCGGCACACCTCTACTCTC | OBD148_869.871 | NR |
| 11 | GGGCTGTGTCCTGATAAACC | OBD148_929.931 | R |

**Table 2.a1**

| | | | |
|---|---|---|---|
| | | | |

| | probe | RP/Rsum | FC |
|---|---|---|---|
| 1 | ORF479_8_81007411_81018107_81095100_81099880_FR | 5655 | 1.14 |
| 2 | ORF482_5_168579937_168582137_168614429_168620163_RR | 8291 | -1.11 |
| 3 | PDCD1LG2_9_5495992_5498009_5563479_5572986_RR | 6499 | -1.13 |
| 4 | IL17D_13_20664875_20671757_20688261_20691044_FF | 6848 | -1.1 |
| 5 | CASP6_4_109703339_109705583_109735036_109741090_RF | 1727 | 1.29 |
| 6 | ORF102_17_34316073_34325822_34367538_34373948_RF | 1958 | -1.26 |
| 7 | TNFSF8_9_114957908_114962933_114975258_114977746_RF | 8214 | -1.11 |
| 8 | ORF712_9_120888366_120893320_120913546_120919710_RR | 8471 | 1.11 |
| 9 | ORF698_18 62296384_62304812_62385139_62386748_FF | 7473 | 1.12 |
| 10 | ORF197_8_26561792_26565691_26638318_26644530_FR | 3611 | -1.2 |
| 11 | IKBKB8_42264241_42271203_42331044_42332799_FR | 1585 | 1.3 |

**Table 2.a2**

| | | | |
|---|---|---|---|
| | | | |

| | PFP | P.value | FDR |
|---|---|---|---|
| 1 | 0.252 | 0.01003 | 0.13564257 |
| 2 | 0.5777 | 0.05624 | 0.298153549 |
| 3 | 0.3857 | 0.01934 | 0.188603463 |
| 4 | 0.4249 | 0.02454 | 0.210154857 |
| 5 | 0.002472 | 0.0000133 | 0.001705674 |
| 6 | 0.009056 | 0.0000289 | 0.002854389 |
| 7 | 0.5681 | 0.0541 | 0.293528769 |
| 8 | 0.6186 | 0.06146 | 0.308076103 |
| 9 | 0.4918 | 0.03615 | 0.248717729 |
| 10 | 0.09085 | 0.0009981 | 0.034849498 |
| 11 | 0.001699 | 0.00000779 | 0.00118933 |

**Table 2.a3**

| | **Probe sequence** |
|---|---|
| | **60 mer** |
| 1 | TCAGATAAGTAACTTCCTGATAATTAACTCGATGCCAATCCACGTCATTAGATGAGGACC |
| 2 | GAATGGCCGAACAGCCATGACAGTCCTCTCGAGGCTACTGGAGTCATTGAAAAGAGGAAT |
| 3 | ACAGTTATTAGAAAAATAAAACATTTGGTCGAACAGCAAAGAGAAGATATTCAACTGCGA |
| 4 | TTAAAGAAGCTAATTTTAAAAATAAATGTCGAAGAGATTGTCACGTTAGAGTTATGTAAA |
| 5 | GGGGCCTCCAGAGTCCCCTTTACAGGCATCGACGCCCCCTGCCTACCTGCCGGGTGCCCC |
| 6 | ATATAAATCTACTTTATAAATAAGGAAATCGAAGTATAATTCAATATACTGTCCAGTAAA |
| 7 | AGTAGTGCAATCATAGCTCACTGAAACCTCGAAAGCTAATGAGGTATGAGGGGAGAATAC |
| 8 | AGTGCTGGGTTCCACACCTCTCAGCTCTTCGACCTCCAGGTCCCCCGCCACTTCCACGGC |
| 9 | GTTGGTGAAAAAGAAAGAAGAAATGGACTCGACCGCTACCACCCCAGCATTTCCAGCAGG |
| 10 | ATAAATAGACTCCACTATGTATAATGACTCGAAATTTTGCTATAAATGTGAGCTTTGAAA |
| 11 | CCACCCCCGCCCCGGGGGAGTCGCCCGGTCGACCCCCTGACATGGGGCTGCCTGGAGCAG |

**Table 2.a4**

| | **Probe Location** | | | | |
|---|---|---|---|---|---|
| | **Chr** | **Start1** | **End1** | **Start2** | **End2** |
| 1 | 8 | 81018076 | 81018105 | 81095102 | 81095131 |
| 2 | 5 | 168579939 | 168579968 | 168614431 | 168614460 |
| 3 | 9 | 5495994 | 5496023 | 5563481 | 5563510 |
| 4 | 13 | 20671726 | 20671755 | 20691013 | 20691042 |
| 5 | 4 | 109703341 | 109703370 | 109741059 | 109741088 |
| 6 | 17 | 34316075 | 34316104 | 34373917 | 34373946 |
| 7 | 9 | 114957910 | 114957939 | 114977715 | 114977744 |
| 8 | 9 | 120888368 | 120888397 | 120913548 | 120913577 |
| 9 | 18 | 62304781 | 62304810 | 62386717 | 62386746 |
| 10 | 8 | 26565660 | 26565689 | 26638320 | 26638349 |
| 11 | 8 | 42271172 | 42271201 | 42331046 | 42331075 |

**Table 2.a5**

| | **4 kb Sequence Location** | | | | |
|---|---|---|---|---|---|
| | **Chr** | **Start1** | **End1** | **Start2** | **End2** |
| 1 | 8 | 81014106 | 81018105 | 81095102 | 81099101 |
| 2 | 5 | 168579939 | 168583938 | 168614431 | 168618430 |
| 3 | 9 | 5495994 | 5499993 | 5563481 | 5567480 |
| 4 | 13 | 20667756 | 20671755 | 20687043 | 20691042 |
| 5 | 4 | 109703341 | 109707340 | 109737089 | 109741088 |
| 6 | 17 | 34316075 | 34320074 | 34369947 | 34373946 |
| 7 | 9 | 114957910 | 114961909 | 114973745 | 114977744 |
| 8 | 9 | 120888368 | 120892367 | 120913548 | 120917547 |
| 9 | 18 | 62300811 | 62304810 | 62382747 | 62386746 |
| 10 | 8 | 26561690 | 26565689 | 26638320 | 26642319 |
| 11 | 8 | 42267202 | 42271201 | 42331046 | 42335045 |

**Table 2.a6**

| | Primer ID | Primer Sequence | Primer ID | Primer Sequence |
|---|---|---|---|---|
| 1 | OBD148_105 | GGACAGCCACTACTCAACCTTTTCCT | OBD148_107 | AAATGCTGGGCTCCTCTTTTGTCCTC |
| 2 | OBD148_669 | CCGACCCTAACATTCAAGGTGTCTCT | OBD148_671 | CCACTTCATTTCATCCCTACTGCCAC |
| 3 | OBD117_029 | CTCACTGCCCAACAGGCTAGAA | OBD117_031 | TCTTGACTCAGAGCCCACAACAA |
| 4 | OBD148_645 | TCTCTACTTCAGGCAGGCAGTGTAAG | OBD148_647 | GGGAGACCATTTCTGTTCACTCTGAG |
| 5 | OBD148_821 | CAGACTAAGGGGCCTCCAGA | OBD148_823 | CGCAATCAGAACCAACTGGC |
| 6 | OBD148_893 | ACTTGTGGCTTCCTTAGCCC | OBD148_895 | TCCTTTGCAGGTATGGACATC |
| 7 | OBD148_917 | TTGCTTGTGAGTTTGATGCAG | OBD148_919 | AAGCCAAATGGGCCTAGCCA |
| 8 | OBD148_301 | CCCAGTTGTCCAGGTTGCTGCCT | OBD148_303 | TGGAGCAGAACCTGTCAGACCTG |
| 9 | OBD148_505 | TGTGTTTATTCCCTACAGAGCAGGTT | OBD148_507 | TGAGCACTGGTTCCCCGCAAATACTG |
| 10 | OBD148_661 | GATGCTGCTGGTGAGAGTAGTCC | OBD148_663 | CATTACTACTCCTCCCAGGGCAGG |
| 11 | OBD148_261 | CGGTGAGCACGGTCTGTCTACTT | OBD148_263 | GTCCTGGGTCCTGGGTGAAAGTC |

**Table 2.a7**

| | Probe | Marker | Type |
|---|---|---|---|
| 1 | ORF479_8_81007411_81018107_81095100_81099880_FR | OBD148_105.107 | S |
| 2 | ORF482_5_168579937_168582137_168614429_168620163_RR | OBD148_669.671 | H |
| 3 | PDCD1LG2_9_5495992_5498009_5563479_5572986_RR | OBD117_029.031 | H |
| 4 | IL17D_13_20664875_20671757_20688261_20691044_FF | OBD148_645.647 | H |
| 5 | CASP6_4_109703339_109705583_109735036_109741090_RF | OBD148_821.823 | S |
| 6 | ORF102_17_34316073_34325822_34367538_34373948_RF | OBD148_893.895 | S |
| 7 | TNFSF8_9_114957908_114962933_114975258_114977746_RF | OBD148_917.919 | S |
| 8 | ORF712_9_120888366_120893320_120913546_120919710_RR | OBD148_301.303 | S |
| 9 | ORF698_18 62296384_62304812_62385139_62386748_FF | OBD148_505.507 | S |
| 10 | ORF197_8_26561792_26565691_26638318_26644530_FR | OBD148_661.663 | S |
| 11 | IKBKB_8_42264241_42271203_42331044_42332799_FR | OBD148_261.263 | S |

**Table 3**

| Stimulatory checkpoint molecules | Inhibitory checkpoint molecules |
|---|---|
| CD27 | A2AR |
| CD28 | B7-H3 |
| CD40 | B7-H4 |
| CD122 | CTLA-4 |
| CD137 | IDO |
| OX40 | KIR |
| GITR | LAG3 |
| ICOS | PD-1 |
| | TIM-3 |
| | VISTA |

**Table 4. Combinations in cancer immunotherapy (biologics, immunocytokines (L19-IL2 and L19-TNF), cytotoxics (Paxlitaxel)**

| **Drug** | **targets** | **Preferred Disease** |
|---|---|---|
| Ipilimumab &Nivolumab | PD-1 and CTLA-4 | metastatic melanoma |
| Paclitaxel, ipilimumab & carboplatin | CTLA4 | non-small-cell lung cancer |
| Ipilimumab & GVAX | CTLA-4 | pancreatic cancer |
| Pidilizumab & rituximab | PD-1 | hematologic malignancies |
| L19-IL2 & L19-TNF | STAT | Melanoma |
| MEDI0680 & Durvalumab | PD1/PDL1 | Advanced solid malignancies |

**Table 5. Other single molecules, immunocytokines and biologics for cancer therapy**

| Drug | targets | Preferred Cancer |
|---|---|---|
| CA-170 (small molecule) | PD1-PDL1 and VISTA | Advanced solid tumour and lymphoma |
| Ruxolitinib (small molecule) | JAK | myelofibrosis and multiple myeloma |
| Tofacitinib (small molecule) | JAK | autoimmune disease |
| Galiellelactone (small molecule) | STAT3 | prostate cancer |
| Ipilimumab (monoclonal antibody) | CTLA4 | melanoma, prostate |
| L19-IL2 (immunocytokine) | STAT | melanoma, pancreatic cancer, RCC |
| L19-TNF (immunocytokine) | STAT | Melanoma |
| Tremelimumab (monoclonal antibody) | CTLA4 | Mesothelioma |
| Nivolumab (ditto) | PD1 | melanoma, non-small-cell lung cancer, renal cell carcinoma, and other solid tumors |
| Pembrolizumab | PD1 | melanoma, non-small-cell lung cancer, renal cell carcinoma, and other solid tumors |
| Pidilizumab | PD1 | hematologic malignancies |
| BMS935559 | PD-L1 | variety of solid tumors |
| GVAXMPDL3280A | PD-L1 | bladder cancer, head and neck cancer, and GI malignancies |
| MEDI4736 | PD-L1 | bladder cancer, head and neck cancer, and GI malignancies |
| MSB0010718C | PD-L1 | bladder cancer, head and neck cancer, and GI malignancies |
| MDX-1105/BMS-936559 | PD-L1 | Cancer |
| AMP-224 | PD1 | colorectal cancer |
| MEDI0680 | PD1 | advanced solid tumors |
| Durvalumab | PDL1 | non-small -cell lung cancer |
| Atezolizumab | PDL1 | advanced or metastatic urothelial carcinoma |
| Avelumab | PDL1 | metastatic Merkel cell carcinoma |

**Table 6**

| Drug | Targets |
|---|---|
| Alemtuzumab (monoclonal antibody). | CD52 |
| Ofatumumab (Second generation human IgG1 antibody). | CD20 |
| Pegylated liposomal doxorubicin (PLD) plus motolimod (VTX2337). | |
| Sipuleucel-T (Approved Cancer Vaccine). | |
| Rituximab (monoclonal antibody). | CD20 |
| Interferon gamma | |
| Combinatorial ablation and immunotherapy. | |
| Polysaccharide-K | |
| Adoptive cell therapy | |
| Anti-CD47 antibodies. | CD47 |
| Polypurine reverse Hoogsteen oligonucleotides (PPRHs). | |
| Anti-GD2 antibodies. | GD2 |
| BGB-A317 (monoclonal antibody). | PD-1 inhibitor |
| Affimer biotherapeutic. | PD-L1 inhibitor |
| Polysaccharides | |
| Neoantigens | |

**Table 7a**

| | Probe | RP/Rsum | FC |
|---|---|---|---|
| 1 | PDCD1LG2_S_5495992_5498009_5563479_5572586_RR | 6499 | -1.13 |
| 2 | MYC_8_127691489 127694045_127738939_127740424_FR | 6220 | 1.15 |
| 3 | IKBKB_8_42264241_42271203_42331044_42332799_FR | 1585 | 1.3 |
| 4 | ORF712_9_120888366_120893320_120913546_120919710_RR | 8471 | 1.11 |
| 5 | ITK_5_157178319_157181048_157266725_157271762_RR | 4605 | 1.15 |
| 6 | IL17D_13_20664875_20671757_20688261_20691044_FF | 6848 | -1.1 |
| 7 | IKBKB_8_42264241_42271203_42290979_42292124_FR | 2465 | 1.25 |
| 8 | IGF1R_15_98731539_98737034_98785670_98790114_FF | 5450 | 1.15 |
| 9 | CASP6_4_109703339_109705583_109735036_109741090_RF | 1727 | 1.29 |
| 10 | TRAF2_9_136904007_136906211_136939587_136941363_RF | 5090 | 1.16 |
| 11 | ORF313_13_20664875_20671757_20695143_20698635_FF | 4910 | -1.16 |
| 12 | ORF479_8_81007411_81018107_81095100_81099880_FR | 5655 | 1.14 |
| 13 | ORF482_5_168579937_168582137_168614429_168620163_RR | 8291 | -1.11 |
| 14 | ORF102_17_34316073_34325822_34367538_34373948_RF | 1958 | -1.26 |
| 15 | TNFSF8_9_114957908_114962933_114975258_114977746_RF | 8214 | -1.11 |
| 16 | ORF698_18_62296384_62304812_62385139_62386748_FF | 7473 | 1.12 |
| 17 | ORF197_8_26561792_26565691_26638318_26644530_FR | 3611 | -1.2 |
| 18 | ORF243_1_161633494_161637462_161657362_161661864_RF | 1345 | 1.16 |
| 19 | ORF313_13_20664875_20671757_20737979_20744490_FR | 2891 | 1.2 |
| 20 | ORF369_13_46087370_46090583_46186579_46193039_RF | 2719 | 1.11 |
| 21 | ORF480_11_77430379_77437843_77514783_77519103_RF | 3101 | -1.1 |
| 22 | ORF698_18_62330039_62332469_62356961_62362521_FR | 6988 | -1.28 |
| 23 | ORF703_1_6461604_6466207_6514024_6515315_FR | 1109 | -1.25 |
| 24 | ORF705_9_114855753_114859111_114920994_114929419_FR | 898 | -1.16 |

**Table 7b**

| | PFP | P.value | FDR |
|---|---|---|---|
| 1 | 0.3857 | 0.01934 | 0.188603463 |
| 2 | 0.3231 | 0.01577 | 0.169755579 |
| 3 | 0.001699 | 0.00000779 | 0.00118933 |
| 4 | 0.6186 | 0.06146 | 0.308076103 |
| 5 | 0.1332 | 0.003607 | 0.076416861 |
| 6 | 0.4249 | 0.02454 | 0.210154857 |
| 7 | 0.01154 | 0.0001149 | 0.00763801 |
| 8 | 0.2259 | 0.008388 | 0.12237213 |
| 9 | 0.002472 | 0.0000133 | 0.001705674 |
| 10 | 0.1832 | 0.005986 | 0.101918497 |
| 11 | 0.2061 | 0.004999 | 0.091747389 |
| 12 | 0.252 | 0.01003 | 0.13564257 |
| 13 | 0.5777 | 0.05624 | 0.298153549 |
| 14 | 0.009056 | 0.0000289 | 0.002854389 |
| 15 | 0.5681 | 0.0541 | 0.293528769 |
| 16 | 0.4918 | 0.03615 | 0.248717729 |
| 17 | 0.09085 | 0.0009981 | 0.034849498 |
| 18 | 0.001212 | 6.11E-06 | 0.000216321 |
| 19 | 0.0315 | 0.000344332 | 0.002847073 |
| 20 | 0.031 | 0.000300988 | 0.002596993 |
| 21 | 0.04015 | 0.000446091 | 0.003407902 |
| 22 | 0.4301 | 0.026579755 | 0.067536623 |
| 23 | 0.000988 | 5.35E-07 | 6.03E-05 |
| 24 | 0.000547 | 2.59E-07 | 4.28E-05 |

**Table 7c**

| | **Probe sequence** |
|---|---|
| | **60 mer** |
| 1 | ACAGTTATTAGAAAAATAAAACATTTGGTCGAACAGCAAAGAGAAGATATTCAACTGCGA |
| 2 | AGGGAGAACAAAAGAAGTTCCATCCATCTCGACGGAGTCCTCCCCGCAGGGCAGCCCCGA |
| 3 | CCACCCCCGCCCCGGGGGAGTCGCCCGGTCGACCCCCTGACATGGGGCTGCCTGGAGCAG |
| 4 | AGTGCTGGGTTCCACACCTCTCAGCTCTTCGACCTCCAGGTCCCCCGCCACTTCCACGGC |
| 5 | CAAAATCAAACACAAATCTAATCAAACTTCGATGTTTGGGGGCGGAGGGCTTTGATGAGA |
| 6 | TTAAAGAAGCTAATTTTAAAAATAAATGTCGAAGAGATTGTCACGTTAGAGTTATGTAAA |
| 7 | CCACCCCCGCCCCGGGGGAGTCGCCCGGTCGATTTCCAAAAGCTCACACATGGGTGCACA |
| 8 | CGTAGAACTAAGATGTATTCAAAGTCAGTCGAAATCACCTGTCCCGGCCTCTTTCCAAAC |
| 9 | GGGGCCTCCAGAGTCCCCTTTACAGGCATCGACGCCCCCTGCCTACCTGCCGGGTGCCCC |
| 10 | CCGCCTCACCTCCCGCATGGTCTTGAGGTCGAGCATGCAGCGCATCTGAGCAGTGAGGCT |
| 11 | TTAAAGAAGCTAATTTTAAAAATAAATGTCGAGGAGCATCTGGATTTAATGATAGTTCAA |
| 12 | TCAGATAAGTAACTTCCTGATAATTAACTCGATGCCAATCCACGTCATTAGATGAGGACC |
| 13 | GAATGGCCGAACAGCCATGACAGTCCTCTCGAGGCTACTGGAGTCATTGAAAAGAGGAAT |
| 14 | ATATAAATCTACTTTATAAATAAGGAAATCGAAGTATAATTCAATATACTGTCCAGTAAA |
| 15 | AGTAGTGCAATCATAGCTCACTGAAACCTCGAAAGCTAATGAGGTATGAGGGGAGAATAC |
| 16 | GTTGGTGAAAAAGAAAGAAGAAATGGACTCGACCGCTACCACCCCAGCATTTCCAGCAGG |
| 17 | ATAAATAGACTCCACTATGTATAATGACTCGAAATTTTGCTATAAATGTGAGCTTTGAAA |
| 18 | AAAGCACGCGTCAGAGTGGGTGGGGCTGTCGATTGTCATCCTCTAGGACTTACAGTTTCT |
| 19 | TTAAAGAAGCTAATTTTAAAAATAAATGTCGAAATTACTTTAAATTAATACAAGCCCCTA |
| 20 | AGGAGGGAGAAAAGTGATGAAGGCCATTTCGAGATGGGTGCCTGGGTGAGAATTTTAATA |
| 21 | TAACAAAAGTAACACCTCTTTGGTATCATCGAAGAGTCCTTGTTCCCATTTTGGCCCAGT |
| 22 | GAGAATCAATTCCATTTTTAAAGCTTAGTCGATTTTGAGGGCTTCTCACAACTCTAGATT |
| 23 | CCGCGCCCGCAGGGCCCGCCCCGCGCCGTCGAGAAGCATAAAGCAGGGACAGGTATGGAG |
| 24 | TTCACTGTTGCCTTTTGTTGTCATTATATCGAGTAATACTGACACTCCTGGCCCACAGAA |

**Table 7d**

| | **Probe Location** | | | | |
|---|---|---|---|---|---|
| | **Chr** | **Start1** | **End1** | **Start2** | **End2** |
| 1 | 9 | 5495994 | 5496023 | 5563481 | 5563510 |
| 2 | 8 | 127694014 | 127694043 | 127738941 | 127738970 |
| 3 | 8 | 42271172 | 42271201 | 42331046 | 42331075 |
| 4 | 9 | 120888368 | 120888397 | 120913548 | 120913577 |
| 5 | 5 | 157178321 | 157178350 | 157266727 | 157266756 |
| 6 | 13 | 20671726 | 20671755 | 20691013 | 20691042 |
| 7 | 8 | 42271172 | 42271201 | 42290981 | 42291010 |
| 8 | 15 | 98737003 | 98737032 | 98790083 | 98790112 |
| 9 | 4 | 109703341 | 109703370 | 109741059 | 109741088 |
| 10 | 9 | 136904009 | 136904038 | 136941332 | 136941361 |
| 11 | 13 | 20671726 | 20671755 | 20698604 | 20698633 |
| 12 | 8 | 81018076 | 81018105 | 81095102 | 81095131 |
| 13 | 5 | 168579939 | 168579968 | 168614431 | 168614460 |
| 14 | 17 | 34316075 | 34316104 | 34373917 | 34373946 |
| 15 | 9 | 114957910 | 114957939 | 114977715 | 114977744 |
| 16 | 18 | 62304781 | 62304810 | 62386717 | 62386746 |
| 17 | 8 | 26565660 | 26565689 | 26638320 | 26638349 |
| 18 | 1 | 161633496 | 161633525 | 161661833 | 161661862 |
| 19 | 13 | 20671726 | 20671755 | 20737981 | 20738010 |
| 20 | 13 | 46087372 | 46087401 | 46193008 | 46193037 |
| 21 | 11 | 77430381 | 77430410 | 77519072 | 77519101 |
| 22 | 18 | 62332438 | 62332467 | 62356963 | 62356992 |
| 23 | 1 | 6466176 | 6466205 | 6514026 | 6514055 |
| 24 | 9 | 114859080 | 114859109 | 114920996 | 114921025 |

**Table 7e**

| | **4 kb Sequence Location** | | | | |
|---|---|---|---|---|---|
| | **Chr** | **Start1** | **End1** | **Start2** | **End2** |
| 1 | 9 | 5495994 | 5499993 | 5563481 | 5567480 |
| 2 | 8 | 127690044 | 127694043 | 127738941 | 127742940 |
| 3 | 8 | 42267202 | 42271201 | 42331046 | 42335045 |
| 4 | 9 | 120888368 | 120892367 | 120913548 | 120917547 |
| 5 | 5 | 157178321 | 157182320 | 157266727 | 157270726 |
| 6 | 13 | 20667756 | 20671755 | 20687043 | 20691042 |
| 7 | 8 | 42267202 | 42271201 | 42290981 | 42294980 |
| 8 | 15 | 98733033 | 98737032 | 98786113 | 98790112 |
| 9 | 4 | 109703341 | 109707340 | 109737089 | 109741088 |
| 10 | 9 | 136904009 | 136908008 | 136937362 | 136941361 |
| 11 | 13 | 20667756 | 20671755 | 20694634 | 20698633 |
| 12 | 8 | 81014106 | 81018105 | 81095102 | 81099101 |
| 13 | 5 | 168579939 | 168583938 | 168614431 | 168618430 |
| 14 | 17 | 34316075 | 34320074 | 34369947 | 34373946 |
| 15 | 9 | 114957910 | 114961909 | 114973745 | 114977744 |
| 16 | 18 | 62300811 | 62304810 | 62382747 | 62386746 |
| 17 | 8 | 26561690 | 26565689 | 26638320 | 26642319 |
| 18 | 1 | 161633496 | 161637495 | 161657863 | 161661862 |
| 19 | 13 | 20667756 | 20671755 | 20737981 | 20741980 |
| 20 | 13 | 46087372 | 46091371 | 46189038 | 46193037 |
| 21 | 11 | 77430381 | 77434380 | 77515102 | 77519101 |
| 22 | 18 | 62328468 | 62332467 | 62356963 | 62360962 |
| 23 | 1 | 6462206 | 6466205 | 6514026 | 6518025 |
| 24 | 9 | 114855110 | 114859109 | 114920996 | 114924995 |

**Table 7f**

| | Probe | Primer ID |
|---|---|---|
| 1 | PDCD1LG2_S_5495992_5498009_5563479_5572586_RR | OBD189-q057 |
| 2 | MYC_8_127691489_127694045_127738939_127740424_FR | OBD189-q013 |
| 3 | IKBKB_8_42264241_42271203_42331044_42332799_FR | OBD148-q261 |
| 4 | ORF712_9_120888366_120893320_120913546_120919710_RR | OBD189-q017 |
| 5 | ITK_5_157178319_157181048_157266725_157271762_RR | OBD189-q065 |
| 6 | IL17D_13_20664875_20671757_20688261_20691044_FF | OBD189-q077 |
| 7 | IKBKB_8_42264241_42271203_42290979_42292124_FR | OBD189-q025 |
| 8 | IGF1R_15_98731539_98737034_98785670_98790114_FF | OBD189-q001 |
| 9 | CASP6_4_109703339_109705583_109735036_109741090_RF | OBD189-q005 |
| 10 | TRAF2_S_136504007_1365906211_136939587_136941363_RF | OBD189-q009 |
| 11 | ORF313_13_20664875_20671757_20695143_20698635_FF | OBD189-q081 |
| 12 | ORF479_8_81007411_81018107_81095100_81099880_FR | OBD189-q061 |
| 13 | ORF482_5_168579937_168582137_168614429_168620163_RR | OBD189-q021 |
| 14 | ORF102_17_34316073_34325822_34367538_34373948_RF | OBD148-q893 |
| 15 | TNFSF8_9_114957908_114962933_114975258_114977746_RF | OBD148-q917 |
| 16 | ORF698_18_62296384_62304812_62385139_62386748_FF | OBD189-q045 |
| 17 | ORF197_8_26561792_26565691_26638318_26644530_FR | OBD189-q069 |
| 18 | ORF243_1_161633494_161637462_161657362_161661864_RF | OBD189-q041 |
| 19 | ORF313_13_20664875_20671757_20737979_20744490_FR | OBD189-q073 |
| 20 | ORF369_13_46087370_46090583_46186579_46193039_RF | OBD189-q053 |
| 21 | ORF480_11_77430379_77437843_77514783_77519103_RF | OBD189-q033 |
| 22 | ORF698_18_62330039_62332469_62356961_62362521_FR | OBD189-q037 |
| 23 | ORF703_1_6461604_6466207_6514024_6515315_FR | OBD189-q029 |
| 24 | ORF705_9_114855753_114859111_114920994_114929419_FR | OBD189-q049 |

**Table 7g**

| | Primer Sequence | Primer ID |
|---|---|---|
| 1 | GAGGGTCACTCACTGCCCAACAGGC | OBD189-q059 |
| 2 | GTCACCTTCATCTCCTTCTCACAGCAG | OBD189-q015 |
| 3 | CGGTGAGCACGGTCTGTCTACTT | OBD148-q263 |
| 4 | CCCAGTTGTCCAGGTTGCTGCCT | OBD189-q019 |
| 5 | TGTATGTCTCCTGAGGTGAAGCAAGAGG | OBD189-q067 |
| 6 | GGAAGTGCCACGAGAAGGAGGATGGTCC | OBD189-q079 |
| 7 | GGTGAGCACGGTCTGTCTACTTTCCC | OBD189-q027 |
| 8 | GGCTGGTGGGAGTATTTTCAAAGAGAAC | OBD189-q003 |
| 9 | CCCCAACTCACAACACCCCAGAC | OBD189-q007 |
| 10 | AGCACTCGTCGTTGGGCGTGTAG | OBD189-q011 |
| 11 | GAAGTGCCACGAGAAGGAGGATGGTCC | OBD189-q083 |
| 12 | TGGACAGCCACTACTCAACCTTTTCCTA | OBD189-q063 |
| 13 | CCGACCCTAACATTCAAGGTGTCTCTAT | OBD189-q023 |
| 14 | ACTTGTGGCTTCCTTAGCCC | OBD148-q895 |
| 15 | TTGCTTGTGAGTTTGATGCAG | OBD148-q919 |
| 16 | CATAGACCCAGGTGTGCTCCGTGGCAGC | OBD189-q047 |
| 17 | CAGTATGAGTGTTCTGTGGCTGCTCCCA | OBD189-q071 |
| 18 | TTGCCACCTGTCTCAGATACCCTTGGTT | OBD189-q043 |
| 19 | GGAAGTGCCACGAGAAGGAGGATGGTCC | OBD189-q075 |
| 20 | TAGAAGCAGGGAGTAGTTGAGCAATGGG | OBD189-q055 |
| 21 | CATAACCACACTGCTACCAACACACCTA | OBD189-q035 |
| 22 | CCTACTGGCACCACTGTGTTGGCTGG | OBD189-q039 |
| 23 | TGCCCGTCGTGGTTCCGCCTTCA | OBD189-q031 |
| 24 | CCATTGTTGCTCAGGCTGCCCTCTTGC | OBD189-q051 |

**Table 7h**

| | Primer Sequence | Probe ID |
|---|---|---|
| 1 | GACTGTAAGGTAGAAATCCTGCCTGGGT | OBD189-p057 |
| 2 | GCTTCGCTTACCAGAGTCGCTGC | OBD189-p013 |
| 3 | GTCCTGGGTCCTGGGTGAAAGTC | OBD148-p261 |
| 4 | CCTGGAGCAGAACCTGTCAGACC | OBD189-p017 |
| 5 | CTTCCACCGTGCCCGCAGCCAGC | OBD189-p065 |
| 6 | CCACCCAGTTCCTCCAGGCATAGCAGG | OBD189-p077 |
| 7 | GGACCCAGGCTCTGCTGCTACAG | OBD189-p025 |
| 8 | GCTCTGTTCAAGTGGCTCTGTTCCA | OBD189-p001 |
| 9 | AGAGGAGGGCAAGGTGTCTGGCT | OBD189-p005 |
| 10 | CGGCACACCTCTACTCTCAGCCT | OBD189-p009 |
| 11 | GGGCTGTGTCCTGATAAACCCATTGTTA | OBD189-p081 |
| 12 | CAAACCCAGATTGGACCTCACAGCCCC | OBD189-p061 |
| 13 | GAGTCAGCGTGTAGTGCTCCCAC | OBD189-p021 |
| 14 | TCCTTTGCAGGTATGGACATC | OBD148-p893 |
| 15 | AAGCCAAATGGGCCTAGCCA | OBD148-p917 |
| 16 | GAGCACTGGTTCCCCGCAAATACTGGG | OBD189-p045 |
| 17 | GCGTGTCTCTCAGGGAAGGCAGGATGC | OBD189-p069 |
| 18 | GCTGCTCCTCTTGCCTGGAATGCCTATT | OBD189-p041 |
| 19 | GGTAAGATGAGGCTGTGGGCAAGGAGC | OBD189-p073 |
| 20 | TCTTCACTTGTGCTATTGGCTTTCCAGC | OBD189-p053 |
| 21 | CTGGTTATTCGGACACTCATAGGACTGG | OBD189-p033 |
| 22 | TATCATAATCAGGCAACTGGCTGGTGC | OBD189-p037 |
| 23 | AGAGACCCACCCCAGCCTCCTGA | OBD189-p029 |
| 24 | GCATTCAAGTGACAGAGAGAAAAGAGGC | OBD189-p049 |

**Table 7i**

| | Probe Sequence | Probe ID |
|---|---|---|
| 1 | ACATTTGGTCGAACAGCAAAGAGAAGATATTCAAC | OBD189-p059 |
| 2 | AGAAGTTCCATCCATCTCGACGGAGTCCTCCC | OBD189-p015 |
| 3 | TCGCCCGGTCGACCCCCTGACATGG | OBD148-p263 |
| 4 | TTCCACACCTCTCAGCTCTTCGACCTCCAGGT | OBD189-p019 |
| 5 | AACACAAATCTAATCAAACTTCGATGTTTGGG | OBD189-p067 |
| 6 | TAAATGTCGAAGAGATTGTCACGTTAGAGTTATG | OBD189-p079 |
| 7 | TCGCCCGGTCGATTTCCAAAAGCTCACACATGG | OBD189-p027 |
| 8 | TCAAAGTCAGTCGAAATCACCTGTCCCGGCCTC | OBD189-p003 |
| 9 | TCCAGAGTCCCCTTTACAGGCATCGACGCCC | OBD189-p007 |
| 10 | ATGGTCTTGAGGTCGAGCATGCAGCGCATCTG | OBD189-p011 |
| 11 | ATAAATGTCGAGGAGCATCTGGATTTAATGATAG | OBD189-p083 |
| 12 | ACTTCCTGATAATTAACTCGATGCCAATCCACGTC | OBD189-p063 |
| 13 | AACAGCCATGACAGTCCTCTCGAGGCTACTGG | OBD189-p023 |
| 14 | TAAGGAAATCGAAGTATAATTCAATATACTGTCCA | OBD148-p895 |
| 15 | TGAAACCTCGAAAGCTAATGAGGTATGA | OBD148-p919 |
| 16 | AGAAGAAATGGACTCGACCGCTACCACCCCAG | OBD189-p047 |
| 17 | AGACTCCACTATGTATAATGACTCGAAATTTTGC | OBD189-p071 |
| 18 | TCAGAGTGGGTGGGGCTGTCGATTGTCATCCT | OBD189-p043 |
| 19 | TAAATGTCGAAATTACTTTAAATTAATACAAGCCC | OBD189-p075 |
| 20 | AGTGATGAAGGCCATTTCGAGATGGGTGCCTGG | OBD189-p055 |
| 21 | ACCTCTTTGGTATCATCGAAGAGTCCTTGTTCCC | OBD189-p035 |
| 22 | AAGCTTAGTCGATTTTGAGGGCTTCTCACAACTC | OBD189-p039 |
| 23 | CGCGCCGTCGAGAAGCATAAAGCAGGGACA | OBD189-p031 |
| 24 | TCATTATATCGAGTAATACTGACACTCCTGGCCC | OBD189-p051 |

**Table 7j**

| | Probe Sequence | Probe |
|---|---|---|
| 1 | TGAATATCTTCTCTTTGCTGTTCGACCAAATGTT | PDCD1LG2_9_5495992_5498009_5563479_5572986_RR |
| 2 | TCCGTCGAGATGGATGGAACTTCTTTTGTTCTCCC | MYC_8_127691489_127694045_127738939_127740424_FR |
| 3 | CCATGTCAGGGGGTCGACCGGGCGA | IKBKB_8_42264241_42271203_42331044_42332799_FR |
| 4 | ACCTGGAGGTCGAAGAGCTGAGAGGTGTGGAA | ORF712_9_120888366_120893320_120913546_120919710_RR |
| 5 | AACATCGAAGTTTGATTAGATTTGTGTTTGATT | ITK_5_157178319_157181048_157266725_157271762_RR |
| 6 | ACTCTAACGTGACAATCTCTTCGACATTTATTTT | IL17D_13_20664875_20671757_20688261_20691044_FF |
| 7 | TGTGAGCTTTTGGAAATCGACCGGGCGACTCC | IKBKB_8_42264241_42271203_42290979_42292124_FR |
| 8 | AGAGGCCGGGACAGGTGATTTCGACTGACTTTG | IGF1R_15_98731539_98737034_98785670_98790114_FF |
| 9 | GGGCGTCGATGCCTGTAAAGGGGACTCTGGA | CASP6_4_109703339_109705583_109735036_109741090_RF |
| 10 | AGATGCGCTGCATGCTCGACCTCAAGACCATG | TRAF2_9_136904007_136906211_136939587_136941363_RF |
| 11 | ACTATCATTAAATCCAGATGCTCCTCGACATTTA | ORF313_13_20664875_20671757_20695143_20698635_FF |
| 12 | TGACGTGGATTGGCATCGAGTTAATTATCAGGAAG | ORF479_8_81007411_81018107_81095100_81099880_FR |
| 13 | AGTAGCCTCGAGAGGACTGTCATGGCTGTTCG | ORF482_5_168579937_168582137_168614429_168620163_RR |
| 14 | TGGACAGTATATTGAATTATACTTCGATTTCCTTA | ORF102_17_34316073_34325822_34367538_34373948_RF |
| 15 | TCATACCTCATTAGCTTTCGAGGTTTCA | TNFSF8_9_114957908_114962933_114975258_114977746_RF |
| 16 | TGGTAGCGGTCGAGTCCATTTCTTCTTTCTTT | ORF698_18_62296384_62304812_62385139_62386748_FF |
| 17 | AGCAAAATTTCGAGTCATTATACATAGTGGAGTC | ORF197_8_26561792_26565691_26638318_26644530_FR |
| 18 | AGGATGACAATCGACAGCCCCACCCACTCTGA | ORF243_1_161633494_161637462_161657362_161661864_RF |
| 19 | GGGCTTGTATTAATTTAAAGTAATTTCGACATTTA | ORF313_13_20664875_20671757_20737979_20744490_FR |
| 20 | ACCCAGGCACCCATCTCGAAATGGCCTTCATCA | ORF369_13_46087370_46090583_46186579_46193039_RF |
| 21 | TGGGAACAAGGACTCTTCGATGATACCAAAGAGG | ORF480_11_77430379_77437843_77514783_77519103_RF |
| 22 | AGAGTTGTGAGAAGCCCTCAAAATCGACTAAGC | ORF698_18_62330039_62332469_62356961_62362521_FR |
| 23 | TGTCCCTGCTTTATGCTTCTCGACGGCGCG | ORF703_1_6461604_6466207_6514024_6515315_FR |
| 24 | TGGGCCAGGAGTGTCAGTATTACTCGATATAAT | ORF705_9_114855753_114859111_114920994_114929419_FR |

**Table 7k**

| | Marker | Type |
|---|---|---|
| 1 | OBD189-q057.q059.p057 | Checkpoint Inhibitor Non-responder |
| 2 | OBD189-q013.q015.p013 | Checkpoint Inhibitor Responder |
| 3 | OBD148-q261.q263.p261 | Checkpoint Inhibitor Responder |
| 4 | OBD189-q017.q019.p017 | Checkpoint Inhibitor Responder |
| 5 | OBD189-q065.q067.p065 | Checkpoint Inhibitor Responder |
| 6 | OBD189-q077.q079.p077 | Checkpoint Inhibitor Non-responder |
| 7 | OBD189-q025.q027.p025 | Checkpoint Inhibitor Responder |
| 8 | OBD189-q001.q003.p003 | Checkpoint Inhibitor Responder |
| 9 | OBD189-q005.q007.p005 | Checkpoint Inhibitor Responder |
| 10 | OBD189-q009.q011.p009 | Checkpoint Inhibitor Responder |
| 11 | OBD189-q081.q083.p081 | Checkpoint Inhibitor Non-responder |
| 12 | OBD189-q061.q063.p061 | Checkpoint Inhibitor Responder |
| 13 | OBD189-q021.q023.p021 | Checkpoint Inhibitor Non-responder |
| 14 | OBD148-q0893.q0895.p0893 | Checkpoint Inhibitor Non-responder |
| 15 | OBD148-q917.q919.p917 | Checkpoint Inhibitor Non-responder |
| 16 | OBD189-q045.q047.p045 | Checkpoint Inhibitor Responder |
| 17 | OBD189-q069.q071.p069 | Checkpoint Inhibitor Non-responder |
| 18 | OBD189-q041.q043.p043 | Checkpoint Inhibitor Non-responder |
| 19 | OBD189-q073.q075.p073 | Checkpoint Inhibitor Non-responder |
| 20 | OBD189-q053.q055.p053 | Checkpoint Inhibitor Non-responder |
| 21 | OBD189-q033.q035.p033 | Checkpoint Inhibitor Responder |
| 22 | OBD189-q037.q039.p037 | Checkpoint Inhibitor Responder |
| 23 | OBD189-q029.q031.p031 | Checkpoint Inhibitor Responder |
| 24 | OBD189-q049.q051.p049 | Checkpoint Inhibitor Responder |

**Table 8a**

| | probe | Marker |
|---|---|---|
| 1 | PDCD1LG2_9_5495992_5498009_5563479_5572986_RR | OBD189-q057.q059.p057 |
| 2 | ITK_5_157178319_157181048_157266725_157271762_RR | OBD189-q065.q067.p065 |
| 3 | CASP6_4_109703339_109705583_109735036_109741090_RF | OBD189-q005.q007.p005 |
| 4 | ORF313_13_20664875_20671757_20695143_20698635_FF | OBD189-q081.q083.p081 |
| 5 | ORF102_17_34316073_34325822_34367538_34373948_RF | OBD148-q0893.q0895.p0893 |
| 6 | ORF369_13_46087370_46090583_46186579_46193039_RF | OBD189-q053.q055.p053 |
| 7 | ORF703_1_6461604_6466207_6514024_6515315_FR | OBD189-q029.q031.p031 |
| 8 | ORF705_9_114855753_114859111_114920994_114929419_FR | OBD189-q049.q051.p049 |

**Table 8b**

| | Primer ID | Primer Sequence | Primer ID |
|---|---|---|---|
| 1 | OBD189-q057 | GAGGGTCACTCACTGCCCAACAGGC | OBD189-q059 |
| 2 | OBD189-q065 | TGTATGTCTCCTGAGGTGAAGCAAGAGG | OBD189-q067 |
| 3 | OBD189-q005 | CCCCAACTCACAACACCCCAGAC | OBD189-q007 |
| 4 | OBD189-q081 | GAAGTGCCACGAGAAGGAGGATGGTCC | OBD189-q083 |
| 5 | OBD148-q893 | ACTTGTGGCTTCCTTAGCCC | OBD148-q895 |
| 6 | OBD189-q053 | TAGAAGCAGGGAGTAGTTGAGCAATGGG | OBD189-q055 |
| 7 | OBD189-q029 | TGCCCGTCGTGGTTCCGCCTTCA | OBD189-q031 |
| 8 | OBD189-q049 | CCATTGTTGCTCAGGCTGCCCTCTTGC | OBD189-q051 |

**Table 8c**

| | Primer Sequence | Probe ID | Probe Sequence |
|---|---|---|---|
| 1 | GACTGTAAGGTAGAAATCCTGCCTGGGT | OBD189-p057 | ACATTTGGTCGAACAGCAAAGAGAAGATATTCAAC |
| 2 | CTTCCACCGTGCCCGCAGCCAGC | OBD189-p065 | AACACAAATCTAATCAAACTTCGATGTTTGGG |
| 3 | AGAGGAGGGCAAGGTGTCTGGCT | OBD189-p005 | TCCAGAGTCCCCTTTACAGGCATCGACGCCC |
| 4 | GGGCTGTGTCCTGATAAACCCATTGTTA | OBD189-p081 | ATAAATGTCGAGGAGCATCTGGATTTAATGATAG |
| 5 | TCCTTTGCAGGTATGGACATC | OBD148-p893 | TAAGGAAATCGAAGTATAATTCAATATACTGTCCA |
| 6 | TCTTCACTTGTGCTATTGGCTTTCCAGC | OBD189-p053 | AGTGATGAAGGCCATTTCGAGATGGGTGCCTGG |
| 7 | AGAGACCCACCCCAGCCTCCTGA | OBD189-p031 | TGTCCCTGCTTTATGCTTCTCGACGGCGCG |
| 8 | GCATTCAAGTGACAGAGAGAAAAGAGGC | OBD189-p049 | TCATTATATCGAGTAATACTGACACTCCTGGCCC |

**Table 9.1**

| | | **Baseline** |
|---|---|---|
| | **Patient Sample ID** | **Clinical Diagnosis** |
| 1 | IOMA1002 * | Hepatocellular carcinoma (liver) (HCC) |
| 2 | IOMA1004 | CA Parotid Gland |
| 3 | IOMA1007 | Alveolar soft part sarcoma of gluteal region |
| 4 | IOMA1008-B | Ca Liver - Hepatocellular carcinoma |
| 5 | IOMA1009-B | Ca NPC |
| 6 | IOMP1002 | NPC, Gastric Cancer |
| 7 | IOMP1005 | T3N3M0 Nasopharyngeal carcinoma |
| 8 | IOMP1006 | Nasopharyngeal carcinoma - metastatic |
| 9 | IOMP1007 | CA Bladder |
| 10 | IOMP1009 | |
| 11 | IOMP1010 | Metastatic carcinoma of prostate |
| 12 | IOMP1011 | NPC |
| 13 | NIOA1001-B | Ca larynx |
| 14 | NIOA1003 (OBDM-0770) * | Ca lung |
| 15 | NIOA1004 | NPC |
| 16 | NIOA1005 | Small cell neuroendocrine tumour of stomach |
| 17 | NIOA1006 (OBDM-094) | Small cell lung carcinoma |
| 18 | NIOA1007 | Lymphoepithelial carcinoma of lacrimal gland |
| 19 | NIOA1008 | HCC of liver |
| 20 | NIOA1009 (OBDM-082) | Metastatic lung carcinoma |
| 21 | NIOA1010 | HCC |
| 22 | NIOA1011 | NPC |
| 23 | NIOA1012 | Lung carcinoma |
| 24 | NIOA1013 | High-grade metastatic, invasive breast carcinoma |
| 25 | NIOA1014 | Metastatic high-grade neuroendocrine tumour |
| 26 | NIOA1015 | Small cell lung cancer with TTF-1/CD56+ |
| 27 | NIOA1016 | Non-small cell lung carcinoma - metastatic |
| 28 | NIOA1017 | Hepatocellular carcinoma |
| 29 | NIOA1018 | Multi-focal hepatoma |
| 30 | NIOA1019 | Metastatic mucoepidermoid carcinoma of salivary gland (parotid gland) |
| 31 | NIOP1001-B | Nasopharyngeal carcinoma |
| 32 | NIOP1003 | Ca cervix |
| 33 | NIOP1004 (OBDM-079) | Lung carcinoma - metastatic (bone) |
| 34 | NIOP1005 | Metastatic NPC |
| 35 | NIOP1006 | NPC - metastatic |
| 36 | NIOP1007 (OBDM-096) | Metastatic lung carcinoma |
| 37 | NIOP1008 | Oral cavity - (1) Right lateral tongue; (2) Right gingival sulcus |
| 38 | NIOP1009 | Carcinoma of transverse colon |
| 39 | NIOP1010 | Metastatic malignant melanoma |
| 40 | NIOP1011 | Metastatic malignant melanoma |
| 41 | NIOP1012 | NPC |
| 42 | NIOP1013 | Metastatic NPC |
| 43 | NIOP1014 | NPC |
| 44 | NIOP1015 | Right temporal brain tumour |
| 45 | NIOP1016 * | Ca kidney - metastatic |
| 46 | NIOP1017 | Poorly differentiated adenocarcinoma of lung |
| 47 | NIOP1018 * | Adenocarcinoma - head of pancreas |
| 48 | NIOP1019 | Stage 4 adenocarcinoma of descending colon |
| 49 | NIOP1020 | Metastatic NPC |
| 50 | NIOP1021 | Recurrent vulval carcinoma |
| 51 | NIOP1022 | Ca kidney (RCC) |
| 52 | NIOP1023 | Metastatic NPC |
| 53 | NIOP1024 | Metastatic lung carcinoma (NSCLC) |
| 54 | NIOP1025 | Left lung squamous cell carcinoma - metastatic stage 4 |
| 55 | NIOP1026 | Metastatic sagittal sinus recurrence with right parotid node recurrence |
| 56 | NIOP1002 | Metastatic nasopharyngeal carcinoma |
| 57 | OBDM-001 (IOMP1003) | Lung |
| 58 | OBDM-002 (IOMP1008) | Lung |
| 59 | OBDM-008 (IOMD1003) | Lung |
| 60 | OBDM-014 (IOMP1004) | Lung |
| 61 | OBDM-019 (IOMA1003) | Lung |
| 62 | OBDM-033 (IOMA1001) | Lung |
| 63 | OBDM-034 (IOMA1005) | Lung |
| 64 | OBDM-035 (IOMA1006) | Lung |
| 65 | OBDM-042 (IOMP1001) | Lung |
| 66 | OBDM-044 (IOMD1001) | Lung |
| 67 | OBDM-046 (IOMD1004) | Lung |
| 68 | OBDM-015 (IOMD1002) | Lung |
| 69 | OBDM-074 (NIOA1002) | Metastatic, anaplastic small cell carcinoma of lung |
| 70 | NIOP1027 | Metastatic, poorly differentiated squamous cell carcinoma of lung |
| 71 | NIOP1028 | Metastatic carcinoma of lung |
| 72 | NIOP1029 | Peri-hilar cholangis carcinoma (Klatskin's Tumour) |
| 73 | NIOP1030 | Hepatocellular carcinoma |
| 74 | NIOP1031 | Metastatic carcinoma of stomach |
| 75 | NIOP1032 | Bile duct (intrahepatic) carcinoma |
| 76 | NIOP1033 | Squamous cell carcinoma of left maxilla |
| 77 | NIOP1034 | Lung carcinoma |
| 78 | NIOP1035 | NPC |
| 79 | NIOP1036 | Metastatic lung carcinoma |
| 80 | NIOP1037 | NPC |
| 81 | NIOP1038 | NPC |
| 82 | NIOP1039 | Pancreatic carcinoma - metastatic |
| 83 | NIOA1020 | Metastatic lung carcinoma |
| 84 | NIOA1021 | Hepatocellular carcinoma - liver |
| 85 | NIOA1022 | Small cell carcinoma of lung |
| 85 | | |

**Table 9.2**

| | **Therapy** | **Response to therapy** | **SEX** | Follow Up 1 | |
|---|---|---|---|---|---|
| 1 | Atezolizumab | PD (progressive disease) | M | IOMA2002 | PD (progressive disease) |
| 2 | Atezolizumab | N/A | F | IOMA2004 | N/A |
| 3 | Atezolizumab | N/A | M | | |
| 4 | Atezolizumab | PD (progressive disease) | M | | |
| 5 | Atezolizumab | PD (progressive disease) | M | IOMA2009* | PD (progressive disease) |
| 6 | Pembrolizumab | PD (progressive disease) | M | | |
| 7 | Pembrolizumab | SD (stable disease) | M | IOMP2005 | SD (stable disease) |
| 8 | Pembrolizumab | N/A | M | IOMP2006 | N/A |
| 9 | Pembrolizumab | N/A | M | IOMP2007 | N/A |
| 10 | | N/A | M | | |
| 11 | Pembrolizumab | N/A | M | | |
| 12 | Pembrolizumab | N/A | F | IOMP2011 | N/A |
| 13 | Atezolizumab | PD (progressive disease) | M | NIOA2001* | PD (progressive disease) |
| 14 | Atezolizumab | PD (progressive disease) | M | NIOA2003* | PD (progressive disease) |
| 15 | Atezolizumab | SD (progressive disease) | M | NIOA2004* | N/A |
| 16 | Atezolizumab | N/A | M | NIOA2005 | N/A |
| 17 | Atezolizumab | N/A | M | NIOA2006 | N/A |
| 18 | Atezolizumab | N/A | M | | |
| 19 | Atezolizumab | N/A | M | | |
| 20 | Atezolizumab | PD (progressive disease) | M | | |
| 21 | Atezolizumab | PD (progressive disease) | F | | |
| 22 | Atezolizumab | SD | M | NIOA2011 | SD (stable disease) |
| 23 | Atezolizumab | PD (progressive disease) | M | NIOA2012 | PD (progressive disease) |
| 24 | Atezolizumab | PD (progressive disease) | F | | |
| 25 | Atezolizumab | PR (partial response) | M | NIOA2014* | PR (partial response) |
| 26 | Atezolizumab | N/A | M | NIOA2015 | N/A |
| 27 | Atezolizumab | PD (progressive disease) | F | NIOA2016 | PD (progressive disease) |
| 28 | Atezolizumab | PD (progressive disease) | M | NIOA2017 | PD (progressive disease) |
| 29 | Atezolizumab | PD (progressive disease) | M | NIOA2018 | PD (progressive disease) |
| 30 | Atezolizumab | PD (progressive disease) | F | | |
| 31 | Pembrolizumab | PD (progressive disease) | M | NIOP2001* | PD (progressive disease) |
| 32 | Pembrolizumab | PD (progressive disease) | F | | |
| 33 | Pembrolizumab | SD | M | NIOP2004 | SD (stable disease) |
| 34 | Pembrolizumab | PD (progressive disease) | M | | |
| 35 | Pembrolizumab | PD (progressive disease) | F | | |
| 36 | Pembrolizumab | PD (progressive disease) | M | NIOP2007 | PD (progressive disease) |
| 37 | Pembrolizumab | N/A | F | | |
| 38 | Pembrolizumab | N/A | M | NIOP2009 | N/A |
| 39 | Pembrolizumab | N/A | M | | |
| 40 | Pembrolizumab | N/A | F | | |
| 41 | Pembrolizumab | PD (progressive disease) | M | NIOP2012 | PD (progressive disease) |
| 42 | Pembrolizumab | SD (stable disease) | F | NIOP2013 | SD (stable disease) |
| 43 | Pembrolizumab | PD (progressive disease) | M | NIOP2014 | PD (progressive disease) |
| 44 | Pembrolizumab | N/A | M | | |
| 45 | Pembrolizumab | PR (partial response) | M | NIOP2016 | PR (partial response) |
| 46 | Pembrolizumab | PD (progressive disease) | F | NIOP2017 | PD (progressive disease) |
| 47 | Pembrolizumab | PD (progressive disease) | M | NIOP2018* | PD (progressive disease) |
| 48 | Pembrolizumab | PD (progressive disease) | M | NIOP2019 | PD (progressive disease) |
| 49 | Pembrolizumab | PD (progressive disease) | F | NIOP2020 | PD (progressive disease) |
| 50 | Pembrolizumab | PR (partial response) | F | NIOP2021 | PR (partial response) |
| 51 | Pembrolizumab | SD (stable disease) | M | NIOP2022* | SD (stable disease) |
| 52 | Pembrolizumab | SD (stable disease) | F | NIOP2023 | SD (stable disease) |
| 53 | Pembrolizumab | PD (progressive disease) | M | | |
| 54 | Pembrolizumab | PD (progressive disease) | F | | |
| 55 | Pembrolizumab | CR (complete response) | M | NIOP2026 | CR (complete response) |
| 56 | Pembrolizumab | PD (progressive disease) | M | NIOP2002 | PD (progressive disease) |
| 57 | Pembrolizumab | SD (stable disease) | M | IOMP2003 | SD (stable disease) |
| 58 | Pembrolizumab | PD (progressive disease) | M | | |
| 59 | durvalumab | | F | IOMD2003 | PR (partial response) |
| 60 | Pembrolizumab | PR (partial response) | M | IOMP2004 | PR (partial response) |
| 61 | Atezolizumab | PD (progressive disease) | M | | |
| 62 | Atezolizumab | PD (progressive disease) | M | IOMA2001* | PD (progressive disease) |
| 63 | Atezolizumab | SD | M | IOMA2005 | SD (stable disease) |
| 64 | Atezolizumab | N/A | M | IOMA2006 | N/A |
| 65 | Pembrolizumab | N/A | F | | |
| 66 | Durvalumab | SD | M | IOMD2001 | SD (stable disease) |
| 67 | Durvalumab | N/A | M | IOMD2004 | N/A |
| 68 | Durvalumab | PR (partial response) | M | IOMD2002 | PR (partial response) |
| 69 | Atezolizumab | PD (progressive disease) | F | | |
| 70 | Pembrolizumab | PD (progressive disease) | F | NIOP2027 | PD (progressive disease) |
| 71 | Pembrolizumab | PD (progressive disease) | F | | |
| 72 | Pembrolizumab | PD (progressive disease) | F | | |
| 73 | Pembrolizumab | PD (progressive disease) | F | NIOP2030 | PD (progressive disease) |
| 74 | Pembrolizumab | N/A | F | | |
| 75 | Pembrolizumab | PD (progressive disease) | M | | |
| 76 | Pembrolizumab | N/A | M | | |
| 77 | Pembrolizumab | PR (partial response) | M | | |
| 78 | Pembrolizumab | N/A | M | | |
| 79 | Pembrolizumab | N/A | F | | |
| 80 | Pembrolizumab | N/A | F | | |
| 81 | Pembrolizumab | N/A | M | | |
| 82 | Pembrolizumab | N/A | M | | |
| 83 | Atezolizumab | N/A | M | NIOA2020 | N/A |
| 84 | Atezolizumab | N/A | M | | |
| 85 | Atezolizumab | N/A | M | | |
| | | | 48 | | |

**Table 9.3**

| | Follow Up 2 | | Follow Up 3 | |
|---|---|---|---|---|
| 1 | IOMA3002 | PD (progressive disease) | IOMA4002 | PD (progressive disease) |
| 2 | IOMA3004 | N/A | | |
| 3 | | | | |
| 4 | | | | |
| 5 | | | | |
| 6 | | | | |
| 7 | IOMP3005 | SD (stable disease) | IOMP4005 | SD (stable disease) |
| 8 | IOMP3006 | N/A | IOMP4006 | N/A |
| 9 | IOMP3007 | N/A | | |
| 10 | | | | |
| 11 | | | | |
| 12 | | | | |
| 13 | NIOA3001 | PD (progressive disease) | | |
| 14 | NIOA3003* | PD (progressive disease) | | |
| 15 | NIOA3004 | SD (stable disease) | | |
| 16 | | | | |
| 17 | | | | |
| 18 | | | | |
| 19 | | | | |
| 20 | | | | |
| 21 | | | | |
| 22 | NIOA3011* | SD (stable disease) | NIOA4011 | SD (stable disease) |
| 23 | | | | |
| 24 | | | | |
| 25 | NIOA3014* | PR (partial response) | NIOA4014 | PR (partial response) |
| 26 | | | | |
| 27 | | | | |
| 28 | | | | |
| 29 | | | | |
| 30 | | | | |
| 31 | | | | |
| 32 | | | | |
| 33 | NIOP3004 | N/A | NIOP4004 | SD (stable disease) |
| 34 | | | | |
| 35 | | | | |
| 36 | | | | |
| 37 | | | | |
| 38 | NIOP3009 | N/A | NIOP4009 | N/A |
| 39 | | | | |
| 40 | | | | |
| 41 | | | | |
| 42 | | | | |
| 43 | | | | |
| 44 | | | | |
| 45 | NIOP3016 | PR (partial response) | NIOP4016 | PR (partial response) |
| 46 | | | | |
| 47 | | | | |
| 48 | NIOP3019 | PD (progressive disease) | | |
| 49 | NIOP3020 | PD (progressive disease) | NIOP4020 | PD (progressive disease) |
| 50 | NIOP3021 | PR (partial response) | NIOP4021 | PR (partial response) |
| 51 | NIOP3022 | SD (stable disease) | | |
| 52 | NIOP3023 | SD (stable disease) | | |
| 53 | | | | |
| 54 | | | | |
| 55 | NIOP3026 | CR (complete response) | | |
| 56 | | | | |
| 57 | IOMP3003 | SD (stable disease) | IOMP4003 | SD (stable disease) |
| 58 | | | | |
| 59 | IOMD3003 | PR (partial response) | IOMD4003* | PR (partial response) |
| 60 | IOMP3004 | PR (partial response) | | |
| 61 | | | | |
| 62 | IOMA3001 | PD (progressive disease) | | |
| 63 | | | | |
| 64 | | | | |
| 65 | | | | |
| 66 | IOMD3001 | SD (stable disease) | IOMD4001* | SD (stable disease) |
| 67 | | | | |
| 68 | IOMD3002* | PR (partial response) | IOMD4002 | PR (partial response) |
| 69 | | | | |
| 70 | | | | |
| 71 | | | | |
| 72 | | | | |
| 73 | | | | |
| 74 | | | | |
| 75 | | | | |
| 76 | | | | |
| 77 | | | | |
| 78 | | | | |
| 79 | | | | |
| 80 | | | | |
| 81 | | | | |
| 82 | | | | |
| 83 | | | | |
| 84 | | | | |
| 85 | | | | |
| 25 | | | 14 | |

**Table 9.4**

| | Follow Up 4 | | Follow Up 5 | |
|---|---|---|---|---|
| 1 | IOMA5002 | PD (progressive disease) | | |
| 2 | | | | |
| 3 | | | | |
| 4 | | | | |
| 5 | | | | |
| 6 | | | | |
| 7 | | | | |
| 8 | | | | |
| 9 | | | | |
| 10 | | | | |
| 11 | | | | |
| 12 | | | | |
| 13 | | | | |
| 14 | | | | |
| 15 | | | | |
| 16 | | | | |
| 17 | | | | |
| 18 | | | | |
| 19 | | | | |
| 20 | | | | |
| 21 | | | | |
| 22 | | | | |
| 23 | | | | |
| 24 | | | | |
| 25 | NIOA5014 | PR (partial response) | | |
| 26 | | | | |
| 27 | | | | |
| 28 | | | | |
| 29 | | | | |
| 30 | | | | |
| 31 | | | | |
| 32 | | | | |
| 33 | | | | |
| 34 | | | | |
| 35 | | | | |
| 36 | | | | |
| 37 | | | | |
| 38 | | | | |
| 39 | | | | |
| 40 | | | | |
| 41 | | | | |
| 42 | | | | |
| 43 | | | | |
| 44 | | | | |
| 45 | | | | |
| 46 | | | | |
| 47 | | | | |
| 48 | | | | |
| 49 | | | | |
| 50 | NIOP5021 | PR (partial response) | | |
| 51 | | | | |
| 52 | | | | |
| 53 | | | | |
| 54 | | | | |
| 55 | | | | |
| 56 | | | | |
| 57 | IOMP5003 | SD (stable disease) | | |
| 58 | | | | |
| 59 | | | | |
| 60 | | | | |
| 61 | | | | |
| 62 | | | | |
| 63 | | | | |
| 64 | | | | |
| 65 | | | | |
| 66 | IOMD5001 | SD (stable disease) | IOMD6001 | SD (stable disease) |
| 67 | | | | |
| 68 | IOMD5002* | PR (partial response) | IOMD6002 | PR (partial response) |
| 69 | | | | |
| 70 | | | | |
| 71 | | | | |
| 72 | | | | |
| 73 | | | | |
| 74 | | | | |
| 75 | | | | |
| 76 | | | | |
| 77 | | | | |
| 78 | | | | |
| 79 | | | | |
| 80 | | | | |
| 81 | | | | |
| 82 | | | | |
| 83 | | | | |
| 84 | | | | |
| 85 | | | | |
| 6 | | | 2 | |

**Table 9.5**

| | Follow Up 6 | |
|---|---|---|
| 1 | | |
| 2 | | |
| 3 | | |
| 4 | | |
| 5 | | |
| 6 | | |
| 7 | | |
| 8 | | |
| 9 | | |
| 10 | | |
| 11 | | |
| 12 | | |
| 13 | | |
| 14 | | |
| 15 | | |
| 16 | | |
| 17 | | |
| 18 | | |
| 19 | | |
| 20 | | |
| 21 | | |
| 22 | | |
| 23 | | |
| 24 | | |
| 25 | | |
| 26 | | |
| 27 | | |
| 28 | | |
| 29 | | |
| 30 | | |
| 31 | | |
| 32 | | |
| 33 | | |
| 34 | | |
| 35 | | |
| 36 | | |
| 37 | | |
| 38 | | |
| 39 | | |
| 40 | | |
| 41 | | |
| 42 | | |
| 43 | | |
| 44 | | |
| 45 | | |
| 46 | | |
| 47 | | |
| 48 | | |
| 49 | | |
| 50 | | |
| 51 | | |
| 52 | | |
| 53 | | |
| 54 | | |
| 55 | | |
| 56 | | |
| 57 | | |
| 58 | | |
| 59 | | |
| 60 | | |
| 61 | | |
| 62 | | |
| 63 | | |
| 64 | | |
| 65 | | |
| 66 | IOMD7001* | SD (stable disease) |
| 67 | | |
| 68 | IOMD7002 | PR (partial response) |
| 69 | | |
| 70 | | |
| 71 | | |
| 72 | | |
| 73 | | |
| 74 | | |
| 75 | | |
| 76 | | |
| 77 | | |
| 78 | | |
| 79 | | |
| 80 | | |
| 81 | | |
| 82 | | |
| 83 | | |
| 84 | | |
| 85 | | |
| | 2 | 182 |
| | | 158 |

## Claims

1. A method of determining how an individual responds to immunotherapy for cancer comprising detecting the presence or absence in the individual of:
- all of the chromosome interactions represented by the probe sequences shown in Table 8c to thereby determine whether the individual will be responsive to immunotherapy; and/or
- all of the chromosome interactions represented by the probe sequences shown in Table 2.a3 to thereby determine whether the individual is a hyper-progressor in whom immunotherapy will accelerate disease.

2. A method according to claim 1, further comprising detecting the presence or absence in the individual of all of the chromosome interactions represented by the probe sequences shown in Table 1.a3 to thereby determine whether the individual will be responsive to immunotherapy.

3. A method according to claim 1 or 2, wherein the presence or absence of the chromosome interactions is determined:
- in a sample from the individual, and/or
- in DNA from the individual, and/or
- by detecting the presence or absence of a DNA loop at the site of the chromosome interactions, and/or
- detecting the presence or absence of distal regions of a chromosome being brought together in a chromosome conformation, and/or
- by detecting the presence of a ligated nucleic acid which is generated during said typing and whose sequence comprises two regions each corresponding to the regions of the chromosome which come together in the chromosome interaction, and/or
- by a process which detects the proximity of the chromosome regions which have come together in the chromosome interaction.

4. A method according to any one of the preceding claims, wherein said detecting of the presence or absence of the chromosome interactions is by a process comprising:
(i) in vitro crosslinking of epigenetic chromosomal interactions which are present;
(ii) optionally isolating the cross-linked DNA;
(iii) subjecting said cross-linked DNA to cleaving;
(iv) ligating said cross-linked cleaved DNA ends to form ligated DNA; and
(v) identifying the presence or absence in said ligated DNA of a DNA sequence that corresponds to each chromosome interaction;
to thereby determine the presence or absence of each chromosome interaction.

5. A method according to claim 3 or 4, wherein said ligated DNA is detected by PCR or by use of a probe.

6. A method according to claim 5 wherein:
(i) detection is by use of a probe, wherein said probe preferably has at least 70% identity to any of the probes shown in Table 1.a3, 2.a3, or 8c; or
(ii) detection is by use of PCR, wherein the PCR preferably uses a primer pair that has at least 70% identity to any of the primer pairs shown in:
- Table 1a6 and 1a7,
- Table 2.a6, or
- Table 8b and 8c.

7. A method according to any one of the preceding claims, wherein:
(i) the method is carried out prior to the individual receiving immunotherapy and/or is carried out to select which therapy the individual should receive for cancer, and/or
(ii) the method is carried out on an individual that has cancer or is suspected of having cancer, and/or
(iii) the method is carried out on individual that has been preselected based on a physical characteristic, risk factor or the presence of a symptom for cancer.

8. A method according to any one of the preceding claims in which the individual:
- is at an early stage of cancer; and/or
- is undergoing, or is about to undergo, cancer therapy, for example cancer immunotherapy.

9. A method according to any one of the preceding claims, wherein the cancer is:
(i) one in which immune-checkpoint inhibitors PD-1/PD-L1 are used for therapy; and/or
(ii) melanoma, lung cancer, hepatocellular carcinoma (liver cancer), bladder, prostate, nasal cancer, parotid gland cancer (salivary gland cancer), alveolar soft part sarcoma (soft tissue cancer); and/or
(iii) breast cancer, cervical cancer, colon cancer, head and neck cancer, Hodgkin lymphoma, kidney cancer, stomach cancer, rectal cancer or a solid tumour.

10. A method according to any one of the preceding claims in which the immunotherapy:
(i) comprises an antibody or immune cell, preferably a T cell or dendritic cell; and/or
(ii) comprises a vaccine, preferably against the cancer; and/or
(iii) modulates, blocks or stimulates an immune checkpoint, and preferably targets or modulates PD-L1, PD-L2 or CTLA4 or any other immune checkpoint molecule disclosed in Table 3; and/or
(iv) comprises a therapy shown in any one of tables 4 to 6; and/or
(v) increases the killing of cancer cells by the immune system, preferably wherein such killing is by a T cell.

11. A method according to any one of the preceding claims wherein the immunotherapy is:
(i) a PD-1 inhibitor or PD-L1 inhibitor, and is preferably an antibody specific for PD-1 or PD-L1; and/or
(ii) a PD-2 inhibitor or PD-L2 inhibitor, and is preferably an antibody specific for PD-2 or PD-L2.

12. A method according to claim 4, wherein detecting the presence or absence of the chromosome interactions comprises specific detection of the ligated DNA by quantitative PCR (qPCR) which uses primers capable of amplifying the ligated DNA and a probe which binds the ligation site during the PCR reaction, wherein said probe comprises sequence which is complementary to sequence from each of the chromosome regions that have come together in the chromosome interaction

13. A method according to claim 13, wherein the probe comprises:
- a fluorophore covalently attached to the 5' end of the probe, and/or
- a quencher covalently attached to the 3' end of the probe

14. A method according to claim 13, wherein:
- said fluorophore is selected from HEX, Texas Red and FAM; and/or
- said probe comprises a nucleic acid sequence of length 10 to 40 nucleotide bases, preferably a length of 20 to 30 nucleotide bases.

15. An immunotherapy for cancer which is an antibody specific for PD-1 or PD-L1 for use in a method of treating a cancer in an individual, wherein said method of treating comprises:
- identifying whether the individual is responsive to the antibody specific for PD-1 or PD-L1 by the method of any one of the preceding claims, and
- administering to an individual that has been identified responsive to an antibody specific for PD-1 or PD-L1 said antibody specific for PD-1 or PD-L1.

## Patentansprüche

1. Verfahren zum Bestimmen, wie ein Individuum auf eine Krebsimmuntherapie anspricht, die das Erfassen des Vorhandenseins oder Fehlens von Folgendem bei einem Individuum umfasst:
- Alle Chromosomen-Interaktionen, die durch die in Tabelle 8c gezeigten Sondensequenzen repräsentiert werden, um dadurch zu bestimmen, ob das Individuum auf eine Immuntherapie reagieren wird; und/oder
- alle Chromosomen-Interaktionen, die durch die in Tabelle 2.a3 gezeigten Sondensequenzen repräsentiert werden, um dadurch zu bestimmen, ob das Individuum ein Hyperprogressor ist, bei dem eine Immuntherapie die Krankheit beschleunigt.

2. Verfahren nach Anspruch 1, ferner umfassend das Erfassen des Vorhandenseins oder Fehlens aller Chromosomen-Interaktionen bei dem Individuum, die durch die in Tabelle 1.a3 gezeigten Sondensequenzen repräsentiert werden, um dadurch zu bestimmen, ob das Individuum auf eine Immuntherapie reagieren wird.

3. Verfahren nach Anspruch 1 oder 2, bei dem das Vorhandensein oder Fehlen der Chromosomen-Interaktionen bestimmt wird:
- In einer Probe von dem Individuum und/oder
- in DNA von dem Individuum, und/oder
- durch Erfassen des Vorhandenseins oder Fehlens einer DNA-Schleife an der Stelle der Chromosomen-Interaktionen und/oder
- Erfassen des Vorhandenseins oder Fehlens von distalen Regionen eines Chromosoms, die in einer Chromosome Conformation zusammengebracht werden und/oder
- durch Erfassen des Vorhandenseins einer ligierten Nukleinsäure, die während der Typisierung erzeugt wird und deren Sequenz zwei Regionen umfasst, die jeweils den Regionen des Chromosoms entsprechen, die bei der Chromosomen-Interaktion zusammenkommen, und/oder
- durch einen Vorgang, der die Umgebung der Chromosomen-Regionen erfasst, die in der Chromosomen-Interaktion zusammengekommen sind.

4. Verfahren nach einem der vorstehenden Ansprüche, wobei das Erfassen des Vorhandenseins oder Fehlens der Chromosomen-Interaktionen durch einen Vorgang erfolgt, umfassend:
(i) In Vitro-Vernetzung epigenetischer Chromosomen-Interaktionen, die vorhanden sind;
(ii) optional Isolieren der vernetzten DNA;
(iii) Unterwerfen der vernetzten DNA einer Spaltung;
(iv) Ligieren der vernetzten, gespaltenen DNA-Enden, um ligierte DNA zu formen; und
(v) Identifizieren des Vorhandenseins oder Fehlens in der ligierten DNA einer DNA-Sequenz, die jeder Chromosomen-Interaktion entspricht;
um dadurch das Vorhandensein oder das Fehlen jeder Chromosomen-Interaktion zu bestimmen.

5. Verfahren nach Anspruch 3 oder 4, wobei die ligierte DNA durch PCR oder das Verwenden einer Sonde erfasst wird.

6. Verfahren nach Anspruch 5, wobei:
(i) Das Erfassen durch den Einsatz einer Sonde erfolgt, wobei die Sonde vorzugsweise mindestens 70% Identität mit allen in Tabelle 1.a3, 2.a3 oder 8c gezeigten Sonden hat; oder
(ii) Erfassen durch den Einsatz von PCR erfolgt, wobei das PCR vorzugsweise ein Primer-Paar verwendet, das mindestens 70% Identität mit jedem der Primer-Paare hat, die in Folgendem dargestellt sind:
- Tabelle 1a6 and 1a7,
- Tabelle 2.a6 oder
- Tabelle 8b and 8c.

7. Verfahren nach einem der vorstehenden Ansprüche, wobei:
(i) Das Verfahren ausgeführt wird, bevor das Individuum die Immuntherapie erhält, und/oder ausgeführt wird, um auszuwählen, welche Therapie das Individuum gegen den Krebs erhalten sollte, und/oder
(ii) das Verfahren an einem Individuum ausgeführt wird, das Krebs hat oder bei dem vermutet wird, dass es Krebs hat, und/oder
(iii) das Verfahren an einem Individuum ausgeführt wird, das basierend auf einer physikalischen Eigenschaft, eines Risikofaktors oder des Vorhandenseins eines Krebssymptoms vorausgewählt wurde.

8. Verfahren nach einem der vorstehenden Ansprüche, bei dem das Individuum:
- in einem frühen Krebsstadium ist; und/oder
- eine Krebstherapie, beispielsweise eine Krebsimmuntherapie durchläuft oder dabei ist zu durchlaufen.

9. Verfahren nach einem der vorstehenden Ansprüche, wobei der Krebs
(i) zu denen gehört, bei denen Immuncheckpoint-Inhibitoren PD-1/PD-L1 für die Therapie verwendet werden; und/oder
(ii) Melanom, Lungenkrebs, hepatozelluläres Karzinom (Leberkrebs), Blasen-, Prostata-, Nasen-, Ohrspeicheldrüsenkrebs (Speicheldrüsenkrebs), alveoläres Weichteilsarkom (Weichteilkrebs); und/oder
(iii) Brustkrebs, Gebärmutterhalskrebs, Dickdarmkrebs, Kopf- und Halskrebs, Hodgkin-Lymphom, Nierenkrebs, Magenkrebs, Enddarmkrebs oder ein solider Tumor ist.

10. Verfahren nach einem der vorstehenden Ansprüche, bei dem die Immuntherapie:
(i) Einen Antikörper oder eine Immunzelle umfasst, vorzugsweise eine T-Zelle oder dendritische Zelle; und/oder
(ii) einen Impfstoff umfasst, vorzugsweise gegen den Krebs; und/oder
(iii) einen Immuncheckpoint moduliert, blockiert oder stimuliert und vorzugsweise auf PD-L1, PD-L2 oder CTLA4 oder jedes andere Immuncheckpoint-Molekül, das in Tabelle 3 offenbart wird, abzielt oder diese moduliert; und/oder
(iv) eine Therapie umfasst, die in einer der Tabellen 4 bis 6 dargestellt ist, und/oder
(v) das Abtöten von Krebszellen durch das Immunsystem steigert, vorzugsweise wobei das Abtöten durch T-Zellen erfolgt.

11. Verfahren nach einem der vorstehenden Ansprüche wobei die Immuntherapie:
(i) Ein PD-1-Inhibitor oder PD-L1-Inhibitor ist und vorzugsweise ein Antikörper, der spezifisch für PD-1 oder PD-L1 ist; und/oder
(ii) ein PD-2-Inhibitor oder PD-L2-Inhibitor ist und vorzugsweise ein Antikörper, der spezifisch für PD-2 oder PD-L2 ist.

12. Verfahren nach Anspruch 4, wobei das Erfassen des Vorhandensein oder Fehlens der Chromosomen-Interaktionen eine spezifische Erfassung der ligierten DNA durch quantitative PCR (qPCR) umfasst, die Primer verwendet, die in der Lage sind, die ligierte DNA zu verstärken, und eine Sonde, die die Ligationsstelle während der PCR-Reaktion bindet, wobei die Sonde eine Sequenz umfasst, die komplementär zu einer Sequenz von jeder der Chromosomen-Regionen ist, die in der Chromosomen-Interaktion zusammengekommen sind.

13. Verfahren nach Anspruch 13, wobei die Sonde Folgendes umfasst:
- Ein Fluorophor, das kovalent mit dem 5'-Ende der Sonde verbunden ist, und/oder
- ein Quencher, der kovalent mit dem 3'-Ende der Sonde verbunden ist

14. Verfahren nach Anspruch 13, wobei:
- Das Fluorophor ausgewählt ist aus HEX, Texas Red und FAM; und/oder
- die Sonde eine Nukleinsäuresequenz mit einer Länge von 10 bis 40 Nukleotidbasen, vorzugsweise mit einer Länge von 20 bis 30 Nukleotidbasen umfasst.

15. Krebsimmuntherapie, bei der es sich um einen Antikörper handelt, der spezifisch für PD-1 oder PD-L1 ist, zur Verwendung in einem Verfahren zur Behandlung von Krebs bei einem Individuum, wobei das Verfahren zur Behandlung umfasst:
- Identifizieren, ob das Individuum auf den für PD-1 oder PD-L1 spezifischen Antikörper durch das Verfahren nach einem der vorstehenden Ansprüche anspricht, und
- Verabreichen an ein Individuum, bei dem festgestellt wurde, dass es auf einen für PD-1 oder PD-L1 spezifischen Antikörper anspricht, wobei der Antikörper für PD-1 oder PD-L1 spezifisch ist.

## Revendications

1. Procédé de détermination de la façon dont un individu répond à une immunothérapie contre le cancer, comprenant la détection de la présence ou de l'absence chez l'individu de :
- toutes les interactions chromosomiques représentées par les séquences de sondes indiquées dans le tableau 8c afin de déterminer si l'individu sera sensible à l'immunothérapie ; et/ou
- toutes les interactions chromosomiques représentées par les séquences de sondes indiquées dans le tableau 2.a3 pour déterminer ainsi si l'individu est un hyperprogresseur chez qui l'immunothérapie accélérera la maladie.

2. Procédé selon la revendication 1, comprenant en outre la détection de la présence ou de l'absence chez l'individu de toutes les interactions chromosomiques représentées par les séquences de sondes indiquées dans le tableau 1.a3 pour déterminer ainsi si l'individu sera sensible à l'immunothérapie.

3. Procédé selon la revendication 1 ou 2, dans lequel la présence ou l'absence des interactions chromosomiques est déterminée :
- dans un échantillon en provenance d'un individu, et/ou
- dans l'ADN en provenance d'un individu, et/ou
- par la détection de la présence ou de l'absence d'une boucle d'ADN au niveau du site des interactions chromosomiques, et/ou
- la détection de la présence ou de l'absence de régions distales d'un chromosome réunies dans une conformation chromosomique, et/ou
- par la détection de la présence d'un acide nucléique ligaturé qui est généré pendant ledit typage et dont la séquence comprend deux régions correspondant chacune aux régions du chromosome qui se rejoignent dans l'interaction chromosomique, et/ou
- par un processus qui détecte la proximité des régions chromosomiques qui se sont réunies dans l'interaction chromosomique.

4. Procédé selon l'une quelconque des revendications précédentes, dans lequel ladite détection de la présence ou de l'absence des interactions chromosomiques s'effectue par un processus comprenant :
(i) la réticulation in vitro des interactions chromosomiques épigénétiques présentes ;
(ii) éventuellement, l'isolation de l'ADN réticulé ;
(iii) la soumission dudit ADN réticulé à un clivage ;
(iv) la ligature desdites extrémités d'ADN clivées réticulées pour former de l'ADN ligaturé ; et
(v) l'identification de la présence ou de l'absence dans ledit ADN ligaturé d'une séquence d'ADN qui correspond à chaque interaction chromosomique ;
la détermination de la présence ou de l'absence de chaque interaction chromosomique.

5. Procédé selon la revendication 3 ou 4, dans lequel ledit ADN ligaturé est détecté par PCR ou à l'aide d'une sonde.

6. Procédé selon la revendication 5 dans lequel :
(i) la détection s'effectue au moyen d'une sonde, dans lequel ladite sonde présente de préférence au moins 70 % d'identité avec l'une quelconque des sondes indiquées dans le tableau 1.a3, 2.a3, ou 8c ; ou
(ii) la détection s'effectue à l'aide de la PCR, dans lequel la PCR utilise de préférence une paire d'amorces qui présentent au moins 70 % d'identité avec l'une des paires d'amorces indiquées dans :
- les tableaux 1a6 et 1a7,
- le tableau 2.a6, ou
- les tableaux 8b et 8c.

7. Procédé selon l'une quelconque des revendications précédentes, dans lequel
(i) le procédé est mis en œuvre avant que l'individu reçoive une immunothérapie et/ou est mis en œuvre pour sélectionner quelle thérapie l'individu doit recevoir contre le cancer, et/ou
(ii) le procédé est mis en œuvre sur un individu atteint d'un cancer ou suspecté d'être atteint d'un cancer, et/ou
(iii) le procédé est mis en œuvre sur un individu présélectionné sur la base d'une caractéristique physique, d'un facteur de risque ou de la présence d'un symptôme du cancer.

8. Procédé selon l'une quelconque des revendications précédentes, dans lequel l'individu :
- est à un stade précoce du cancer ; et/ou
- subit, ou est sur le point de subir, une thérapie anticancéreuse, par exemple, une immunothérapie anticancéreuse.

9. Procédé selon l'une quelconque des revendications précédentes, dans lequel le cancer est :
(i) un traitement dans lequel des inhibiteurs de point de contrôle immunitaire PD-1/PD-L1 sont utilisés pour la thérapie ; et/ou
(ii) le mélanome, le cancer du poumon, le carcinome hépatocellulaire (cancer du foie), le cancer de la vessie, le cancer de la prostate, le cancer du nez, le cancer de la glande parotide (cancer des glandes salivaires), le sarcome alvéolaire des parties molles (cancer des tissus mous) ; et/ou
(iii) le cancer du sein, le cancer du col de l'utérus, le cancer du côlon, le cancer de la tête et du cou, le lymphome hodgkinien, le cancer du rein, le cancer de l'estomac, le cancer du rectum ou une tumeur solide.

10. Procédé selon l'une quelconque des revendications précédentes, dans lequel l'immunothérapie :
(i) comprend un anticorps ou une cellule immunitaire, de préférence une cellule T ou une cellule dendritique ; et/ou
(ii) comprend un vaccin, de préférence contre le cancer ; et/ou
(iii) module, bloque ou stimule un point de contrôle immunitaire, et de préférence cible ou module PD-L1, PD-L2 ou CTLA4 ou toute autre molécule de point de contrôle immunitaire divulgué dans le tableau 3 ; et/ou
(iv) comprend une thérapie indiquée dans l'un quelconque des tableaux 4 à 6 ; et
(v) augmente la destruction des cellules cancéreuses par le système immunitaire, de préférence dans lequel une telle destruction est effectuée par une cellule T.

11. Procédé selon l'une quelconque des revendications précédentes, dans lequel l'immunothérapie est :
(i) un inhibiteur de PD-1 ou un inhibiteur de PD-L1, et est de préférence un anticorps spécifique de PD-1 ou PD-L1 ; et/ou
(ii) un inhibiteur de PD-2 ou un inhibiteur de PD-L2, et est de préférence un anticorps spécifique de PD-2 ou PD-L2.

12. Procédé selon la revendication 4, dans lequel la détection de la présence ou de l'absence des interactions chromosomiques comprend la détection spécifique de l'ADN ligaturé par PCR quantitative (qPCR) qui utilise des amorces pouvant amplifier l'ADN ligaturé et une sonde qui se lie au site de ligature pendant la réaction PCR, dans lequel ladite sonde comprend une séquence qui est complémentaire à la séquence de chacune des régions chromosomiques qui se sont réunies dans l'interaction chromosomique.

13. Procédé selon la revendication 13, dans lequel la sonde comprend :
- un fluorophore fixé de manière covalente à l'extrémité 5' de la sonde, et/ou
- un extincteur fixé de manière covalente à l'extrémité 3' de la sonde

14. Procédé selon la revendication 13, dans lequel :
- ledit fluorophore est choisi parmi HEX, Texas Red et FAM ; et/ou
- ladite sonde comprend une séquence d'acides nucléiques de longueur de 10 à 40 bases nucléotidiques, de préférence une longueur de 20 à 30 bases nucléotidiques.

15. Immunothérapie contre le cancer qui est un anticorps spécifique de PD-1 ou PD-L1 destiné à être utilisé dans un procédé de traitement d'un cancer chez un individu, dans laquelle ledit procédé de traitement comprend :
- l'identification, si oui ou non l'individu est sensible à l'anticorps spécifique de PD-1 ou PD-L1 par le procédé selon l'une quelconque des revendications précédentes, et
- l'administration à un individu qui a été identifié sensible à un anticorps spécifique de PD-1 ou PD-L1 dudit anticorps spécifique de PD-1 ou PD-L1.
